# EUROPEAN PATENT SPECIFICATION

(11) **EP 2 033 662 B1**
(45) Date of publication and mention of the grant of the patent: **17.10.2012**
(21) Application number: 08167128.1
(22) Date of filing: 18.01.2005
(51) Int. Cl.: A61K 47/48, A61K 38/27, C07K 14/61, C12P 21/00, C12N 9/96, C07K 1/107, A61K 38/19, A61K 38/26, A61K 38/28, A61K 38/48

(54) **Transglutaminase mediated conjugation of peptides**
TRANSGLUTAMINASE VERMITTELTE KONJUGATION VON PEPTIDEN.
CONJUGAISON DE PEPTIDES INDUITE PAR LA TRANSGLUTAMINASE.

(30) Priority: 21.01.2004 DK 200400076
(43) Date of publication of application: 11.03.2009
(62) Divisional of application: 05700578.7
(73) Proprietor: Novo Nordisk Health Care AG, 8050 Zürich (CH)
(72) Inventor: Nils Langeland Johansen, Niels W., 2100 København Ø (DK); Zundel, Magali, 2870 Dyssegaard (DK); Dörwald, Florencio Zaragoza, 3930 Visp (CH)
(74) Representative: Winther, Kamilla

(56) References cited:
- WO-A-01/70685
- WO-A-90/03401
- WO-A-02/055532
- WO-A-2005/034988
- WO-A-2005/035553
- WO-A-2005/035565
- GORMAN J J ET AL: "TRANS GLUTAMINASE AMINE SUBSTRATES FOR PHOTOCHEMICAL LABELING AND CLEAVABLE CROSS LINKING OF PROTEINS" JOURNAL OF BIOLOGICAL CHEMISTRY, vol. 255, no. 3, 1980, pages 1175-1180, XP002325552 ISSN: 0021-9258
- SCOTT W L ET AL: "Synthesis of reagents for the one step incorporation of hydrazide functionality onto the lysine residues of proteins, and their use as linkers for carbonyl containing molecules" BIOORGANIC & MEDICINAL CHEMISTRY LETTERS, OXFORD, GB, vol. 6, no. 13, 9 July 1996 (1996-07-09), pages 1491-1496, XP004175739 ISSN: 0960-894X
- SATO HARUYA ET AL: "Transglutaminase-mediated dual and site-specific incorporation poly(ethylene glycol) derivatives into a chimeric interleukin-2" BIOCONJUGATE CHEMISTRY, vol. 11, no. 4, July 2000 (2000-07), pages 502-509, XP002325559 ISSN: 1043-1802
- THUMSHIRN G ET AL: "MULTIMERIC CYCLIC RGD PEPTIDES AS POTENTIAL TOOLS FOR TUMOR TARGETING: SOLID-PHASE PEPTIDE SYNTHESIS AND CHEMOSELECTIVE OXIME LIGATION" CHEMISTRY - A EUROPEAN JOURNAL, VCH PUBLISHERS, US, vol. 9, no. 12, 16 June 2003 (2003-06-16), pages 2717-2725, XP001156650 ISSN: 0947-6539
- DURIEUX P ET AL: "Synthesis of biotinylated glycosulfopeptides by chemoselective ligation" TETRAHEDRON LETTERS, ELSEVIER SCIENCE PUBLISHERS, AMSTERDAM, NL, vol. 42, no. 12, 18 March 2001 (2001-03-18), pages 2297-2299, XP004229906 ISSN: 0040-4039
- INGALLINELLA P ET AL: "A NEW METHOD FOR CHEMOSELECTIVE CONJUGATION OF UNPROTECTED PEPTIDESTO DAUNO- AND DOXURUBICIN" BIOORGANIC & MEDICINAL CHEMISTRY LETTERS, OXFORD, GB, vol. 11, no. 10, 21 May 2001 (2001-05-21), pages 1343-1346, XP001022261 ISSN: 0960-894X
- ZHANG L: "Preparation of functionally active cell-permeable peptides by single step ligation of two peptide modules" PROCEEDINGS OF THE NATIONAL ACADEMY OF SCIENCES OF USA, NATIONAL ACADEMY OF SCIENCE. WASHINGTON, US, vol. 95, no. 16, 4 August 1998 (1998-08-04), pages 9184-9189, XP002133028 ISSN: 0027-8424
- BREINBAUER ROLF ET AL: "Azide-alkyne coupling: a powerful reaction for bioconjugate chemistry." CHEMBIOCHEM : A EUROPEAN JOURNAL OF CHEMICAL BIOLOGY. 7 NOV 2003, vol. 4, no. 11, 7 November 2003 (2003-11-07), pages 1147-1149, XP009059050 ISSN: 1439-4227
- KING H D ET AL: "MONOCLONAL ANTIBODY CONJUGATES OF DOXORUBICIN PREPARED WITH BRANCHED LINKERS: A NOVEL METHOD FOR INCREASING THE POTENCY OF DOXORUBICIN IMMUNOCONJUGATES" BIOCONJUGATE CHEMISTRY, ACS, WASHINGTON, DC, US, vol. 10, no. 2, March 1999 (1999-03), pages 279-288, XP000804254 ISSN: 1043-1802
- CLARK R ET AL: "Long-acting growth hormones produced by conjugation with polyethylene glycol" JOURNAL OF BIOLOGICAL CHEMISTRY, AMERICAN SOCIETY OF BIOLOCHEMICAL BIOLOGISTS, BIRMINGHAM,, US, vol. 271, no. 36, 6 September 1996 (1996-09-06), pages 21969-21977, XP002216386 ISSN: 0021-9258
- SATO H ET AL: "Enzymatic procedure for site-specific pegylation of proteins" ADVANCED DRUG DELIVERY REVIEWS, vol. 54, 17 June 2002 (2002-06-17), pages 487-504, XP002330435
- SATO H ET AL: "Further studies on the site-specific protein modification by microbial transglutaminase", BIOCONJUGATE CHEMISTRY, ACS, WASHINGTON, DC, US, vol. 12, no. 5, 1 September 2001 (2001-09-01), pages 701-710, XP002453457, ISSN: 1043-1802, DOI: DOI:10.1021/BC000132H

## Description

### FIELD OF THE INVENTION

The present invention relates to a novel method for post-translational conjugation of peptides wherein transglutaminase is used to incorporate a point of attachment in the peptide whereto another group can be selectively attached. Said conjugated peptides have altered characteristics and may thus be of use in therapeutic applications or they may ease the analysis or isolation and purification of said peptides.

### BACKGROUND OF THE INVENTION

It is well-known to modify the properties and characteristics of peptides by conjugating groups to the peptide which duly changes the properties of the peptide. Such conjugation generally requires some functional group in the peptide to react with another functional group in a conjugating group. Typically, amino groups, such as the N-terminal amino group or the ε-amino group in lysines, have been used in combination with a suitable acylating reagent. It is often desired or even required to be able to control the conjugation reaction, i.e. to control where the conjugating compounds are attached and to control how many conjugating groups are attached. This is often referred to as specificity.

It is an object of the present invention to provide a method by which peptides may be conjugated with a high degree of specificity. In general terms, the method exploits an enzyme, e.g. transglutaminase, capable of incorporating a compound comprising a suitable functional group into the peptide, where said functional group is subsequently used as a point where to conjugate.

Conjugation of peptides in general has been known for a long time, and US 4,179,337 disclosed more than 20 years ago peptides conjugated to polyethylene or polypropylene glycols.

Different types of chemistries have been disclosed which are effective in forming a bond between the peptide and the moiety to be conjugated to the peptide. EP 605 963 discloses the grafting of aqueous polymers which form an oxime linkage with an aldehyde group on a protein. None of the natural amino acid comprises an aldehyde, so a hydroxyl group thus has to be oxidized as a first step in the conjugating process. WO 96/41813 discloses polymers which are functionalised with an amino-oxy oxime forming group useful in conjugation reactions. WO 98/05363 discloses a compound comprising a peptide and a water-soluble polymer, wherein the two are covalently bonded through an oxime bond at the N-terminal amino acid residue.

Derivatives of human growth hormone (hGH) of increasing size produced by reacting hGH with a N-hydroxysuccinimide ester of polyethylene glycol-5000 (PEG5000), a 5-kDa reagent that selectively conjugates to primary amines, have been described by Clarke et al.(JBC 271, 21969 (1996)). WO 02/055532 describes hGH and hGH variants and a variety of options for conjugating said polypeptide with a macromolar substance

Furthermore, the use of enzymes to enable a more specific conjugation of peptides is known. EP 243 929 discloses the use of proteolytic enzymes, such as carboxypeptidase to incorporate a compound with a functional group in the C-terminal of a peptide, where said functional group can subsequently be used as a point where to attach cytotoxic groups, other peptides or reporter groups used to facilitate analysis of the peptide, such as e.g. fluorescent groups. This technique, however, limits the point of attachment to the C-terminal amino acid residue, something which constitute a severe limitation if the C-terminal residue is essential for the activity of the peptide.

Transglutaminase has previously been used to alter the properties of peptides. In the food industry and particular in the diary industry many techniques are available to e.g. cross-bind peptides using transglutaminases. Other documents disclose the use of transglutaminase to alter the properties of physiologically active peptides including EP 950665 and EP 785276. Sato et al (Bioconjug.Chem 11, 502-509 (2000)) and Sato et al (Adv. Drug Del. Rev. 54, 487-504 (2002)) disclose the direct reaction between a peptide substrate comprising one or two TGase substrate sites (including one or two Gln residues) in a peptides sequence fused N-terminally to IL-2 and an amine-functionalised PEG or similar ligands in the presence of transglutaminase. Wada in Biotech. Lett., 23, 1367-1372, 2001 discloses the direct conjugation of β-lactoglobulin with fatty acids by means of transglutaminase.

### SUMMARY OF THE INVENTION

The present inventors have surprisingly found that enzymes, such as e.g. transglutaminase may be used to incorporate into a peptide one or more functional groups, which are not accessible in the in the peptide to form a functionalised peptide, and that this functionalised peptide may subsequently be reacted with another compound comprising a conjugating moiety and one or more functional groups capable of reacting with the functional group or groups thus incorporated in the peptide but not with other functional groups present in the peptide.

Such method provides a high degree of specificity in that transglutaminase can only catalyse the incorporation of compounds at amino acid residues which are substrates for transglutaminase, and in that the functional groups are selected so that they only react with each other, not with other functional groups accessible in the peptide. In this way, the conjugating moiety is only attached at controlled locus or loci, and by selecting the functional groups, the number of conjugated groups can be controlled.

Accordingly, the present invention provides a method for conjugating peptides, said method comprising the steps of
i) reacting in one or more steps a Gln-residue containing peptide represented by the formula 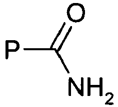 with a nitrogen containing nucleophile (first compound) represented by the formula

   H₂N-D-R-X

   comprising one or more functional groups or latent functional groups, which are not accessible in any of the amino acids constituting said peptide, in the presence of transglutaminase capable of catalysing the incorporation of said first compound into said peptide to form a transaminated peptide of the formula 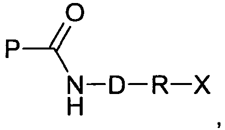 and
ii) optionally activate said latent functional group X, and
iii) reacting in one or more steps said transaminated peptide with a second compound of the formula

   Y-E-Z

   comprising one or more functional groups, wherein said functional group(s) do not react with functional groups accessible in the amino acid residues constituting said peptide, and wherein said functional group(s) in said second compound is capable of reacting with said functional group(s) in said nitrogen containing nucleophile (first compound) so that a covalent bond between said transaminated peptide and said second compound is formed resulting in a conjugated peptide of the formula 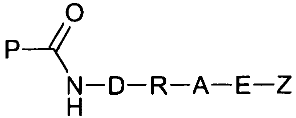
wherein D represents a bond or oxygen;
R represents a linker or a bond;
X represents a radical comprising a functional group or a latent functional group not accessible in the amino acid residues constituting the peptide P-C(O)-NH₂;
Y represents a radical comprising one or more functional groups which groups react with functional groups present in X, and which functional groups do not react with functional groups accessible in the peptide P-C(O)-NH₂;
E represents a linker or a bond;
A represents an oxime, hydrazone, phenylhydrazone, semicarbazone, triazole or isooxazolidine moiety, the moiety formed by the reaction between the functional groups comprised in X and Y; and
Z is the moiety to be conjugated to the peptide.

Peptides conjugated by the method of the present invention is also disclosed. Peptides which are modified in a way to make them better suited for the method of the present invention are also mentioned herein.

Reagents and enzymes suitable for use in the methods of the present invention is further disclosed.

Compositions, e.g. pharmaceutical compositions comprising peptides conjugated by methods of the present invention are also included in the disclosure.

Peptides conjugated according to the methods of the present invention for use in therapy are also disclosed.

Therapeutic methods for the treatment of diseases comprising the administration of conjugated peptides prepared according to the methods of the present invention is also disclosed.

It is a still further objective of the present invention to provide a method for improving the pharmacological properties of a peptide by conjugation said peptide according to the methods of the present invention.

### DEFINITIONS

In the present context "transamination" or similar is intended to indicate a reaction where nitrogen in the side chain of glutamine is exchanged with nitrogen from another compound, in particular nitrogen from another nitrogen containing nucelophile.

In the present context, the term "not accessible" is intended to indicate that something is absent or *de facto* absent in the sense that it cannot be reached. When it is stated that functional groups are not accessible in a peptide to be conjugated it is intended to indicate that said functional group is absent from the peptide or, if present, in some way prevented from taking part in reactions. By way of example, said functional group could be buried in the structure of the peptide so that it is shielded from participating in the reaction, or it could be located in an area of the peptide where restricted flexibility of the peptide chain prevents the functional group from participating in reactions. It is recognised that whether or not a functional group is accessible depends on the reaction conditions. It may be envisaged that in the presence of denaturing agents or at elevated temperatures the peptide may unfold to expose otherwise not accessible functional groups. It is to be understood that "not accessible" means "not accessible at the reaction condition chosen for the particular reaction of interest".

In the present context, the term "oxime bond" is intended to indicate a moiety of the formula -C=N-O-.

In the present context, the term "hydrazone bond" is intended to indicate a moiety of the formula -C=N-N-.

In the present context, the term "phenylhydrazone bond" is intended to indicate a moiety of the formula

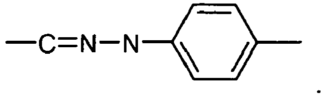

In the present context, the term "semicarbazone bond" is intended to indicate a moiety of the formula -C=N-N-C(O)-N-.

The term "alkane" is intended to indicate a saturated, linear, branched and/or cyclic hydrocarbon. Unless specified with another number of carbon atoms, the term is intended to indicate hydrocarbons with from 1 to 30 (both included) carbon atoms, such as 1 to 20 (both ncluded), such as from 1 to 10 (both included), e.g. from 1 to 5 (both included); or from 15 to 30 carbon atoms (both included).

The term "alkene" is intended to a indicate linear, branched and/or cyclic hydrocarbon comprising at least one carbon-carbon double bond. Unless specified with another number of carbon atoms, the term is intended to indicate hydrocarbons with from 2 to 30 (both included) carbon atoms, such as 2 to 20 (both included), such as from 2 to 10 (both included), e.g. from 2 to 5 (both included); or from 15 to 30 carbon atoms (both included).

The term "alkyne" is intended to indicate a linear, branched and/or cyclic hydrocarbon comprising at least one carbon-carbon triple bond, and it may optionally comprise one or more carbon-carbon double bonds. Unless specified with another number of carbon atoms, the term is intended to indicate hydrocarbons with from 2 to 30 (both included) carbon atoms, such as from 2 to 20 (both included), such as from 2 to 10 (both included), e.g. from 2 to 5 (both included); or from 15 to 30 carbon atoms (both included).

The term "homocyclic aromatic compound" is intended to indicate aromatic hydrocarbons, such as benzene and naphthalene.

The term "heterocyclic compound" is intended to indicate a cyclic compound comprising 5, 6 or 7 ring atoms from which 1, 2, 3 or 4 are hetero atoms selected from N, O and/or S. Examples of heterocyclic aromatic compounds include thiophene, furan, pyran, pyrrole, imidazole, pyrazole, isothiazole, isooxazole, pyridine, pyrazine, pyrimidine, pyridazine, as well as their partly or fully hydrogenated equivalents, such as piperidine, pirazolidine, pyrrolidine, pyroline, imidazolidine, imidazoline, piperazine and morpholine.

The terms "hetero alkane", "hetero alkene" and "hetero alkyne" is intended to indicate alkanes, alkenes and alkynes as defined above, in which one or more hetero atom or group have been inserted into the structure of said moieties. Examples of hetero groups and atoms include -O-, -S-, -S(O)-, -S(O)₂-, -C(O)- -C(S)- and -N(R*)-, wherein R* represents hydrogen or C₁-C₆-alkyl. Examples of heteroalkanes include.

| | | |
|---|---|---|
| 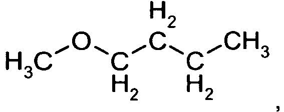 | 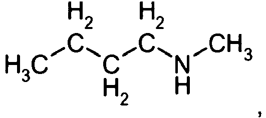 | 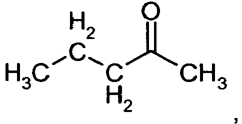 |
| 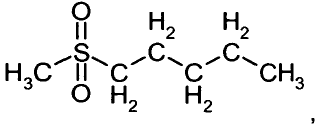 | 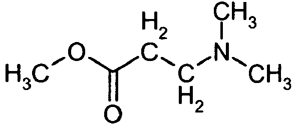 and | 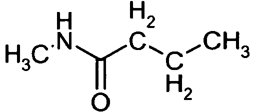 |

The term "radical" or "biradical" is intended to indicate a compound from which one or two, respectively, hydrogen atoms have been removed. When specifically stated, a radical may also indicate the moiety formed by the formal removal of a larger group of atoms, e.g. hydroxyl, from a compound.

The term "halogen" is intended to indicate members of the seventh main group of the periodic table, e.g. F, Cl, Br and I.

The term "PEG" is intended to indicate polyethylene glycol of a molecular weight between approximately 100 and approximately 1,000,000 Da, including analogues thereof, wherein for instance the terminal OH-group has been replaced by an alkoxy group, such as e.g. a methoxy group, an ethoxy group or a propoxy group. In particular, the PEG wherein the terminal -OH group has been replaced by methoxy is refered to as mPEG.

The term "mPEG" (or more properly "mPEGyI") means a polydisperse or monodisperse radical of the structure 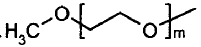 wherein m is an integer larger than 1. Thus, a mPEG wherein m is 90 has a molecular weight of 3991 Da, i.e. approx 4kDa. Likewise, a mPEG with an average molecular weight of 20 kDa has an average m of 454. Due to the process for producing mPEG these molecules often have a distribution of molecular weights. This distribution is described by the polydispersity index.

The term "polydispersity index" as used herein means the ratio between the weight average molecular weight and the number average molecular weight, as known in the art of polymer chemistry (see e.g. "Polymer Synthesis and Characterization", J.A. Nairn, University of Utah, 2003). The polydispersity index is a number which is greater than or equal to one, and it may be estimated from Gel Permeation Chromatographic data. When the polydispersity index is 1, the product is monodisperse and is thus made up of compounds with a single molecular weight. When the polydispersity index is greater than 1 it is a measure of the polydispersity of that polymer, i.e. how broad the distribution of polymers with different molecular weights is.

The use of for example "mPEG20000" in formulas, compound names or in molecular structures indicates an mPEG residue wherein mPEG is polydisperse and has a molecular weight of approximately 20 kDa.

The polydispersity index typically increases with the molecular weight of the PEG or mPEG. When reference is made to 20 kDa PEG and in particular 20 kDa mPEG it is intended to indicate a compound (or in fact a mixture of compounds) with a polydisperisty index below 1.06, such as below 1.05, such as below 1.04, such as below 1.03, such as between 1.02 and 1.03. When reference is made to 30 kDa PEG and in particular 30 kDa mPEG it is intended to indicate a compound (or in fact a mixture of compounds) with a polydisperisty index below 1.06, such as below 1.05, such as below 1.04, such as below 1.03, such as between 1.02 and 1.03. When reference is made to 40 kDa PEG and in particular 40 kDa mPEG it is intended to indicate a compound (or in fact a mixture of compounds) with a polydisperisty index below 1.06, such as below 1.05, such as below 1.04, such as below 1.03, such as between 1.02 and 1.03

In the present context, the words "peptide" and "protein" are used interchangeably and are intended to indicate the same. The term "peptide" is intended to indicate a compound with two or more amino acid residues linked by a peptide bond. The amino acids may be natural or unnatural. The term is also intended to include said compounds substituted with other peptides, saccharides, lipids, or other organic compound, as well as compounds wherein one or more amino acid residue have been chemically modified. The term is also intended to include peptides to which prosthetic groups are attached. In particular, the peptide exerts a physiological, such as e.g. a therapeutic activity.

In the present context, the term "aryl" is intended to indicate a homocyclic aromatic ring radical or a fused homocyclic ring system radical wherein at least one of the rings are aromatic. Typical aryl groups include phenyl, biphenylyl, naphthyl, tetralinyl and the like.

The term "heteroaryl", as used herein, alone or in combination, refers to an aromatic ring radical with for instance 5 to 7 ring atoms, or to a fused aromatic ring system radical with for instance from 7 to 18 ring atoms, wherein at least on ring is aromatic and contains one or more heteroatoms as ring atoms selected from nitrogen, oxygen, or sulfur heteroatoms, wherein N-oxides and sulfur monoxides and sulfur dioxides are permissible heteroaromatic substitutions. Examples include furanyl, thienyl, thiophenyl, pyrrolyl, imidazolyl, pyrazolyl, triazolyl, tetrazolyl, thiazolyl, oxazolyl, isoxazolyl, oxadiazolyl, thiadiazolyl, isothiazolyl, pyridinyl, pyridazinyl, pyrazinyl, pyrimidinyl, quinolinyl, isoquinolinyl, benzofuranyl, benzothiophenyl, indolyl, and indazolyl, and the like.

The term "conjugate" as a noun is intended to indicate a modified peptide, i.e. a peptide with a moiety bonded to it to modify the properties of said peptide. As a verb, the term is intended to indicate the process of bonding a moiety to a peptide to modify the properties of said peptide.

The term "prodrug" as used herein is intended to indicate a compound which not or which not necessarily has a therapeutic activity but which upon administration is transformed into a therapeutically active compound by a reaction taking place in the body. Typically such reactions are hydrolysis, e.g. by esterases or oxidations. Examples of prodrugs include biohydrolyzable amides and biohydrolyzable esters and also encompasses a) compounds in which the biohydrolyzable functionality in such a prodrug is encompassed in the compound according to the present invention, and b) compounds which may be oxidized or reduced biologically at a given functional group to yield drug substances according to the present invention. Examples of these functional groups include 1,4-dihydropyridine, N-alkylcarbonyl-1,4-dihydropyridine, 1,4-cyclohexadiene, tert-butyl, and the like.

As used herein, the term "biohydrolyzable ester" is an ester of a drug substance (in casu, a compound according to the invention) which either a) does not interfere with the biological activity of the parent substance but confers on that substance advantageous properties *in vivo* such as duration of action, onset of action, and the like, or b) is biologically inactive but is readily converted in vivo by the subject to the biologically active principle. The advantage is, for example increased solubility or that the biohydrolyzable ester is orally absorbed from the gut and is transformed to a compound according to the present invention in plasma. Many examples of such are known in the art and include by way of example lower alkyl esters (*e.g*., C₁-C₄), lower acyloxyalkyl esters, lower alkoxyacyloxyalkyl esters, alkoxyacyloxy esters, alkyl acylamino alkyl esters, and choline esters.

As used herein, the term "biohydrolyzable amide" is an amide of a drug substance (in casu, a compound according to the present invention) which either a) does not interfere with the biological activity of the parent substance but confers on that substance advantageous properties in vivo such as duration of action, onset of action, and the like, or b) is biologically inactive but is readily converted in vivo by the subject to the biologically active principle. The advantage is, for example increased solubility or that the biohydrolyzable amide is orally absorbed from the gut and is transformed to a compound according to the present invention in plasma. Many examples of such are known in the art and include by way of example lower alkyl amides, α-amino acid amides, alkoxyacyl amides, and alkylaminoalkylcarbonyl amides.

In the present context, the term "pharmaceutically acceptable salt" is intended to indicate salts which are not harmful to the patient. Such salts include pharmaceutically acceptable acid addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids and the like. Further examples of pharmaceutically acceptable inorganic or organic acid addition salts include the pharmaceutically acceptable salts listed in J. Pharm. Sci. 1977, 66, 2,. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like.

A "therapeutically effective amount" of a compound as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician or veterinary.

The term "treatment" and "treating" as used herein means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. The patient to be treated is preferably a mammal, in particular a human being, but it may also include animals, such as dogs, cats, cows, sheep and pigs.

### DESCRIPTION OF THE INVENTION

Transglutaminase (E.C.2.3.2.13) is also known as protein-glutamine-γ-glutamyltransferase and catalyses the general reaction

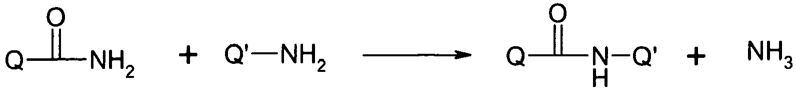

In one embodiment, Q-C(O)-NH₂ (amine acceptor) represents a glutamine containing peptide and Q'-NH₂ (amine donor) then represents a first compound, as indicated above, or Q-C(O)-NH₂ represents a first compound as indicated above and Q'-NH₂ then represents a lysine containing peptide. In a particular embodiment, however, Q-C(O)-NH₂ represents a glutamine containing peptide and Q'-NH₂ represents a first compound as indicated above.

A common amine donor *in vivo* is peptide bound lysine, and the above reaction then affords cross-bonding of peptides. The coagulation factor Factor XIII is a transglutaminase which effects clotting of blood upon injuries. Different transglutaminases differ from each other, e.g. in what amino acid residues around the Gln are required for the protein to be a substrate, i.e. different transglutaminases will have different Gln-containing peptides as substartes depending on what amino acid residues are neighbours to the Gln residue. This aspect can be exploited if a peptide to be modified contains more than one Gln residue. If it is desired to selectively conjugate the peptide only at some of the Gln residues present, this selectivity can be obtained be selection of a transglutaminase which only accepts the relevant Gln residue(s) as substrate. Alternatively, one or more amino acid residues close to a Gln may be altered, e.g. by means of genetic engineering to modify the activity of a given transglutaminase to said Gln residue.

It is recognised that whether or not a compound is substrate for a given enzyme in principle depends on the reaction conditions, e.g. the time frame. Given sufficient time, many compounds not normally regarded as substrates are, in fact, substrates. When it is stated above that for a given transglutaminase some Gln residues may be substrates while other are not it is intended to indicate that "others are not" to an extend where the desired selectivity can still be achieved. If one or more Gln residues, which it is desired to leave unconjugated, is, in fact, a substrate for transglutaminase, however, only if in contact with transglutaminase for an extended period of time, selectivity may be achieved by removing or inactivating the transglutaminase after a suitable time.

Examples of useful transglutaminases include microbial transglutaminases, such as e.g. from *Streptomyces mobaraense, Streptomyces cinnamoneum* and *Streptomyces griseocarneum* (all disclosed in US 5,156,956), and *Streptomyces lavendulae* (disclosed in US 5,252,469) and *Streptomyces ladakanum* (JP2003199569). It should be noted that members of the former genus *Streptoverticillium* are now included in the genus *Streptomyces* [Kaempfer, J.Gen.Microbiol., 137, 1831-1892, 1991]. Other useful microbial transglutaminases have been isolated from *Bacillus subtilis* (disclosed in US 5,731,183) and from various Myxomycetes. Other examples of useful microbial transglutaminases are those disclosed in WO 96/06931 (e.g. transglutaminase from *Bacilus lydicus*) and WO 96/22366. Useful non-microbial transglutaminases include guinea-pig liver transglutaminase, and transglutaminases from various marine sources like the flat fish *Pagrus major* (disclosed in EP-0555649), and the japanese oyster *Crassostrea gigas* (disclosed in US 5,736,356).

In one embodiment, Q'-NH₂, i.e. the first compound as indicated above, is a nitrogen containing nucleophile, wherein a nucleophile is understood to be a basic, electron-rich compound which tends to attack the nucleus of carbon. A nitrogen containing nucleophile can for instance be an amine or an oxy amine derivative.

In one embodiment, the invention relates to a method of conjugating peptides, wherein a Gln residue containing peptide represented by the formula 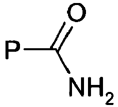 is reacted in one or more steps with a nitrogen containing nucleophile (first compound) represented by the formula

H₂N-D-R-X

in the presence of a transglutaminase to form a transaminated peptide of the formula 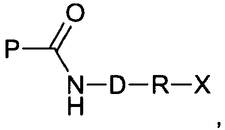 optionally said latent functional group in X is then activated,
said transaminated peptide being further reacted with a second compound of the formula

Y-E-Z

to form a conjugated peptide of the formula 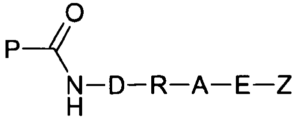 wherein D represents a bond or oxygen;
R represents a linker or a bond;
X represents a radical comprising one or more functional groups or latent functional groups not accessible in the amino acid residues constituting the peptide P-C(O)-NH₂;
Y represents a radical comprising one or more functional groups which groups react with functional groups present in X, and which functional groups do not react with functional groups accessible in the peptide P-C(O)-NH₂;
E represents a linker or a bond;
A represents an oxime, hydrazone, phenylhydrazone, semicarbazone, triazole or isooxazolidine moiety, the moiety formed by the reaction between the pair of functional groups comprised in X and Y; and
Z is the moiety to be conjugated to the peptide.

Following the conjugation, the conjugated peptide may be isolated and purified by techniques well-known in the art. The conjugated peptide may also be converted into a pharmaceutically acceptable salt or prodrug, if relevant.

In particular, said method may also comprise a step wherein the resulting conjugated peptide is formulated as a pharmaceutical composition.

The moiety A formed in the reaction between the functional groups of X and Y may in principle be of any kind depending on what properties of the final conjugated peptide is desired. In some situation it may be desirable to have a labile bond which can be cleaved at some later stage, e.g. by some enzymatic action or by photolysis. In other situations, it may be desirable to have a stable bond, so that a stable conjugated peptide is obtained. Particular mentioning is made of the type of moieties formed by reactions between amine derivatives and carbonyl groups, such as oxime, hydrazone, phenylhydrazone and semicarbazone moieties.

In one embodiment the functional groups of X and Y are selected from amongst carbonyl groups, such as keto and aldehyde groups, and amino derivatives, such as
- hydrazine derivatives: -NH-NH₂,
- hydrazine carboxylate derivatives: -O-C(O)-NH-NH₂,
- semicarbazide derivatives: -NH-C(O)-NH-NH₂,
- thiosemicarbazide derivatives: -NH-C(S)-NH-NH₂,
- carbonic acid dihydrazide derivatives: -NHC(O)-NH-NH-C(O)-NH-NH₂,
- carbazide derivatives: -NH-NH-C(O)-NH-NH₂,
- thiocarbazide derivatives: -NH-NH-C(S)-NH-NH₂,
- aryl hydrazine derivatives: -NH-C(O)-C₆H₄-NH-NH₂, and
- hydrazide derivatives: -C(O)-NH-NH₂; or
oxylamine derivatives, such as -O-NH₂, -C(O)-O-NH₂, -NH-C(O)-O-NH₂ and -NH-C(S)-O-NH₂.

It is to be understood, that if the functional group comprised in X is a carbonyl group, then the functional group comprised in Y is an amine derivative, and vice versa. Due to the presence of -NH₂ groups in most peptides, a better selectivity is believed to be obtained if X comprises a keto- or an aldehyde- functionality.

Another example of a suitable pair of functional groups present in X and Y is azide derivatives (-N₃) and alkynes which react to form a triazole moiety. Still another example of a suitable pair is alkyne and nitril-oxide which react to form a isooxazolidine moiety.

It is to be understood that the functional group comprised in X may be latent in the sense that it has to be activated prior to the reaction with Y-E-Z. By way of example, X may comprise a moiety which upon reaction with a suitable reagent is transformed to an aldehyde or a ketone. Examples of such moieties include 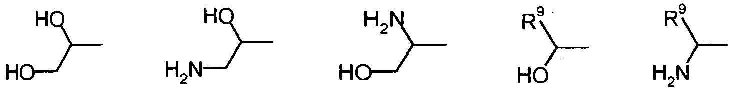 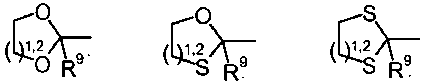 wherein R⁹ represents H, C₁₋₆alkyl, aryl or heteroaryl. Particular examples include methyl, ethyl and propyl. Said moieties may be transformed to an aldehyde or ketone by oxidation with a suitable agent, such as e.g. periodate, or by hydrolysis with an aqueous acid, optionally in the presence of a catalyst, such as copper, silver, or mercury salts.

In particular, the compound of the formula (first compound),

H₂N-D-R-X

may be selected from amongst 4-(aminomethyl)phenyl ethanone, 4-(2-aminoethyl)phenyl ethanone, N-(4-acetylphenyl) 2-aminoacetamide, 1-[4-(2-aminoethoxy)phenyl]ethanone, 1-[3-(2-aminoethoxy)phenyl]ethanone, 1,4-bis(aminoxy)butane, 3-oxapentane-1,5-dioxyamine, 1,8-diaminoxy-3,6-dioxaoctane, 1,3-bis(aminoxy)propan-2-ol, 1,11-bis(aminoxy)-3,6,9-trioxaundecane, 1,3-diamino-2-propanol, 1,2-bis(aminoxy)ethane, and 1,3-bis(aminoxy)propane.

Both the compound to transaminate (first compound) and the compound to be reacted with the transaminated peptide (second compound) comprises a linker, R and E, respectively. These linkers, which are independent of each other, may be absent or selected from amongst alkane, alkene or alkyne diradicals and hetero alkane, hetero alkene and hetero alkyne diradicals, wherein one or more optionally substituted aromatic homocyclic biradical or biradical of a heterocyclic compound, e.g. phenylene or piperidine biradical may be inserted into the aforementioned biradicals. It is to be understood that said linkers may also comprise substitutions by groups selected from amongst hydroxyl, halogen, nitro, cyano, carboxyl, aryl, alkyl and heteroaryl.

Both E and R represent bonds or linkers, and in the present context the term "linker" is intended to indicate a moiety functioning as a means to separate Y from Z and X from NH₂-D-, respectively. One function of the linkers E and R may be to provide adequate flexibility in the linkage between the peptide and the conjugated moiety Z. Typical examples of E and R include straight, branched and/or cyclic C₁₋₁₀alkylene, C₂₋₁₀alkenylene, C₂₋₁₀alkynylene, C₂₋₁₀heteroalkylene, C₂₋₁₀heteroalkenylene, C₂₋₁₀heteroalkynylene, wherein one or more homocyclic aromatic compound biradical or heterocyclic compound biradical may be inserted. Particular examples of E and R include

| | |
|---|---|
| 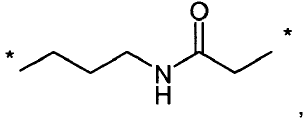 | 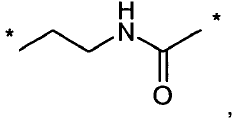 |
| 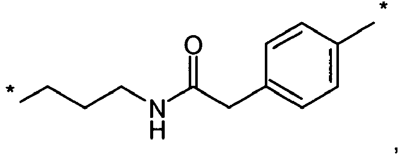 | 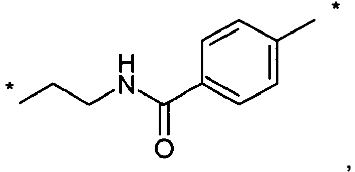 |
| 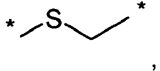 | 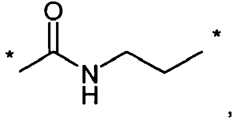 |
| 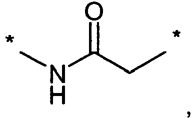 | 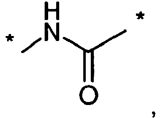 |
| 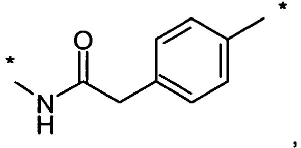 | 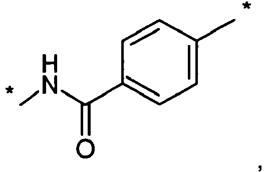 |
| 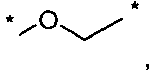 | 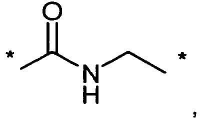 |
| 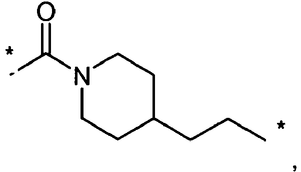 | 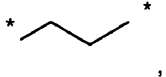 |
| 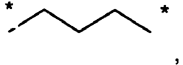 | 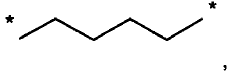 and |
| 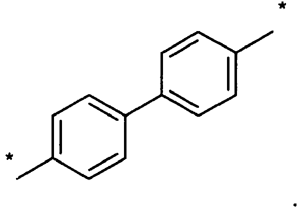 | |

| | |
|---|---|
| wherein * denotes points of attachment. | |

A need for modifying peptides may arise for any number of reasons, and this is also reflected in the kinds of compounds that may be conjugated to peptides according to the methods of the present invention. It may be desirable to conjugate peptides to alter the physico-chemical properties of the peptide, such as e.g. to increase (or to decrease) solubility to modify the bioavailability of therapeutic peptides. In another embodiment, it may be desirable to modify the clearance rate in the body by conjugating compounds to the peptide which binds to plasma proteins, such as e.g. albumin, or which increase the size of the peptide to prevent or delay discharge through the kidneys. Conjugation may also alter and in particular decrease the susceptibility of a peptide to hydrolysis, such as e.g. *in vivo* proteolysis. In another embodiment, it may be desirable to conjugate a label to facilitate analysis of the peptide. Examples of such label include radioactive isotopes, fluorescent markers and enzyme substrates. In still another embodiment, a compound is conjugated to a peptide to facilitate isolation of the peptide. For example, a compound with a specific affinity to a particular column material may be conjugated to the peptide. It may also be desirable to modify the immunogenecity of a peptide, e.g. by conjugating a peptide so as to hide, mask or eclipse one or more immunogenic epitopes at the peptide.

In one embodiment, the invention provides a method of improving pharmacological properties of peptides. The improvement is with respect to the corresponding un-conjugated peptide. Examples of such pharmacological properties include functional *in vivo* half-life, immunogencity, renal filtration, protease protection and albumin binding.

The term "functional *in vivo* half-life" is used in its normal meaning, i.e., the time at which 50% of the biological activity of the peptide or conjugated peptide are still present in the body/target organ, or the time at which the activity of the peptide or conjugated peptide is 50% of its initial value. As an alternative to determining functional *in vivo* half-life, "*in vivo* plasma half-life" may be determined, i.e., the time at which 50% of the peptide or peptide conjugate circulate in the plasma or bloodstream prior to being cleared. Determination of plasma half-life is often more simple than determining functional half-life and the magnitude of plasma half-life is usually a good indication of the magnitude of functional *in vivo* half-life. Alternative terms to plasma half-life include serum half-life, circulating half-life, circulatory half-life, serum clearance, plasma clearance, and clearance half-life.

The term "increased" as used in connection with the functional *in vivo* half-life or plasma half-life is used to indicate that the relevant half-life of the peptide conjugate is statistically significantly increased relative to that of the un-conjugated (parent) peptide, as determined under comparable conditions. For instance the relevant half-life may be increased by at least about 25%, such as by at lest about 50%, e.g., by at least about 100%, 150%, 200%, 250%, or 500%. In one embodiment, the compounds of the present invention exhibit an increase in half-life of at least about 5 h, preferably at least about 24 h, more preferably at least about 72 h, and most preferably at least about 7 days, relative to the half-life of the parent peptide.

Measurement of *in vivo* plasma half-life can be carried out in a number of ways as described in the literature. An increase in *in vivo* plasma half-life may be quantified as a decrease in clearance (CL) or as an increase in mean residence time (MRT). Conjugated peptides of the present invention for which the CL is decreased to less than 70%, such as less than 50%, such than less than 20%, such than less than 10% of the CL of the parent peptide as determined in a suitable assay is said to have an increased *in* vivo plasma half-life. Conjugated peptides of the present invention for which MRT is increased to more than 130%, such as more than 150%, such as more than 200%, such as more than 500% of the MRT of the parent peptide in a suitable assay is said to have an increased *in vivo* plasma half-life. Clearance and mean residence time can be assessed in standard pharmacokinetic studies using suitable test animals. It is within the capabilities of a person skilled in the art to choose a suitable test animal for a given protein. Tests in human, of course, represent the ultimate test. Typically, and as an example, the mice, rats, dogs, monkeys or pigs are in injected with the compiound of interest. The amount injected depends on the test animal. Subsequently, blood samples are taken over a period of one to five days as appropriate for the assessment of CL and MRT. The blood samples are conveniently analysed by ELISA techniques.

The term "Immunogenicity" of a compound refers to the ability of the compound, when administered to a human, to elicit a deleterious immune response, whether humoral, cellular, or both. In any human sub-population, there may exist individuals who exhibit sensitivity to particular administered proteins. Immunogenicity may be measured by quantifying the presence of growth hormone antibodies and/or growth hormone responsive T-cells in a sensitive individual, using conventional methods known in the art. In one embodiment, the conjugated peptide of the present invention exhibit a decrease in immunogenicity in a sensitive individual of at least about 10%, preferably at least about 25%, more preferably at least about 40% and most preferably at least about 50%, relative to the immunogenicity for that individual of the parent peptide.

The term "protease protection" or "protease protected" as used herein is intended to indicate that the conjugated peptide of the present invention is more resistant to the plasma peptidase or proteases than is the parent peptide. Protease and peptidase enzymes present in plasma are known to be involved in the degradation of circulating proteins.

Resistance of a protein to degradation by for instance dipeptidyl aminopeptidase IV (DPPIV) is determined by the following degradation assay: Aliquots of the protein (5 nmol) are incubated at 37 °C with 1 µL of purified dipeptidyl aminopeptidase IV corresponding to an enzymatic activity of 5 mU for 10-180 minutes in 100 µL of 0.1 M triethylamine-HCl buffer, pH 7.4. Enzymatic reactions are terminated by the addition of 5 µL of 10% trifluoroacetic acid, and the protein degradation products are separated and quantified using HPLC analysis. One method for performing this analysis is : The mixtures are applied onto a Vydac C18 widepore (30 nm pores, 5 µm particles) 250 x 4.6 mm column and eluted at a flow rate of 1 ml/min with linear stepwise gradients of acetonitrile in 0.1% trifluoroacetic acid (0% acetonitrile for 3 min, 0-24% acetonitrile for 17 min, 24-48% acetonitrile for 1 min) according to Siegel et al., Regul. Pept. 1999;79:93-102 and Mentlein et al. Eur. J. Biochem. 1993;214:829-35. Proteins and their degradation products may be monitored by their absorbance at 220 nm (peptide bonds) or 280 nm (aromatic amino acids), and are quantified by integration of their peak areas related to those of standards. The rate of hydrolysis of a protein by dipeptidyl aminopeptidase IV is estimated at incubation times which result in less than 10% of the peptide being hydrolysed. In one embodiment, the rate of hydrolysis of the peptide conjugate is less than 70%, such as less than 40%, such as less than 10% of that of the parent peptide.

The most abundant protein component in circulating blood of mammalian species is serum albumin, which is normally present at a concentration of approximately 3 to 4.5 grams per 100 millilitres of whole blood. Serum albumin is a blood protein of approximately 70,000 daltons which has several important functions in the circulatory system. It functions as a transporter of a variety of organic molecules found in the blood, as the main transporter of various metabolites such as fatty acids and bilirubin through the blood, and, owing to its abundance, as an osmotic regulator of the circulating blood. Serum albumin has a half-life of more than one week, and one approach to increasing the plasma half-life of proteins has been to conjugate to the protein a group that binds to serum albumin. Albumin binding property may be determined as described in J.Med.Chem, 43, 2000, 1986-1992.

Particular examples of Z include radicals comprising one or more labels, such as fluorescent markers, such as fluorescein radical, rhodamine radical, Texas Red ® radical and phycobili protein radical; enzyme substrates, such as p-nitrophenol acetate radical; and radioactive isotopes, such as Cu-64, Ga67, Ga-68, Zr-89, Ru-97, Tc-99, Rh-105, Pd-109, In-111, I-123, I-125, I-131, Re-186, Re-188, Au-198, Pb-203, At-211, Pb-212 and Bi-212; organic moieties, such as PEG or mPEG radicals and amino derivatives thereof (including straight and branched PEG and mPEG radicals); straight, branched and/or cyclic C₁₋₂₂alkyl, C₂₋₂₂alkenyl, C₂₋₂₂alkynyl, C₁₋₂₂heteroalkyl, C₂₋₂₂heteroalkenyl, C₂₋₂₂heteroalkynyl, wherein one or more homocyclic aromatic compound biradical or heterocyclic compound biradical may be inserted, and wherein said C₁-C₂₂ or C₂-C₂₂ radicals may optionally be substituted with one or more substituents selected from hydroxyl, halogen, carboxyl, heteroaryl and aryl, wherein said aryl or heteroaryl may optionally be further substituted by one or more substituents selected from hydroxyl, halogen, and carboxyl; steroid radicals; lipid radicals; polysaccharide radicals, e.g. dextrans; polyamide radicals e.g. polyamino acid radicals; PVP radicals; PVA radicals; poly(1-3-dioxalane); poly(1,3,6-trioxane); ethylene/maleic anhydride polymer; Cibacron dye stuffs, such as Cibacron Blue 3GA; polyamide chains of specified length, as disclosed in WO 00/12587 and hydroxyalkyl starch, such as e.g. hydroxyethyl starch, such as disclosed in WO 03/074087 and WO 02/80979.

Particular mentioning is made of C₁₀₋₂₀alkyl, such as C₁₅ and C₁₇, and in particular linear C₁₅ and C₁₇, and benzophenone derivatives of the formula

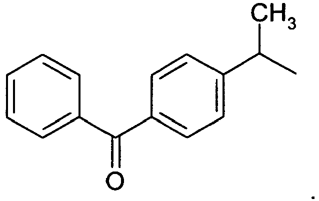

Particular mentioning is made of Z comprising a cibacronyl radical as sketched below

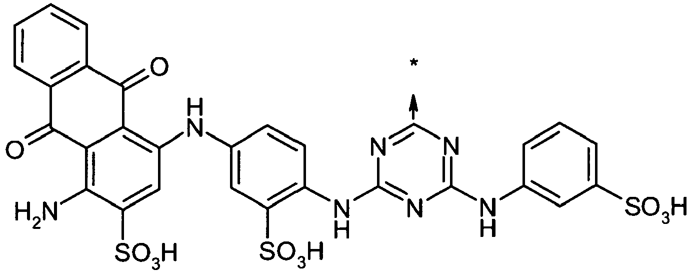

The PEG or mPEG conjugated to a peptide according to the present invention may be of any molecular weight. In particular the molecular weight may be between 500 and 1000,000 Da, such as between 500 and 500,000 Da, such as between 500 and 100,000 Da, such as between 500 and 60,000 Da, such as between 1000 and 40,000 Da, such as between 5000 and 40,000 Da. In particular, PEG with molecular weights of between 10,000 Da and 40,000 Da, such as between 20,000 Da and 40,000 Da, such as between 20,000 and 30,000 Da or between 30,000 and 40,000 Da may be used. Particular mentioning is made of PEG or mPEG with a molecular weight of 10,000, 20,000, 30,000 or 40,000 Da.

Z may be branched so that Z comprises more than one of the above mentioned labels or radicals. For instance, mPEG40K is typically achieved as a branched mPEG with two arm each comprising a mPEG20k.

In one embodiment, Z comprises one or more moieties that are known to bind to plasma proteins, such as e.g. albumin. The ability of a compound to bind to albumin may be determined as described in J.Med.Chem, 43, 2000, 1986-1992. In the present context, a compound is defined as binding to albumin if Ru/Da is above 0.05, such as above 0.10, such as above 0.12 or even above 0.15.

In another embodiment of the invention the albumin binding moiety is a peptide, such as a peptide comprising less than 40 amino acid residues. A number of small peptides which are albumin binding moieties, are disclosed in J. BiolChem. 277, 38 (2002)35035-35043

Particular examples of compounds of the formula Y-E-Z include 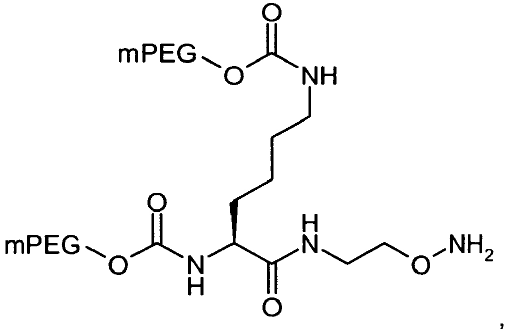 wherein mPEG has a molecular weight of 20 kDa, 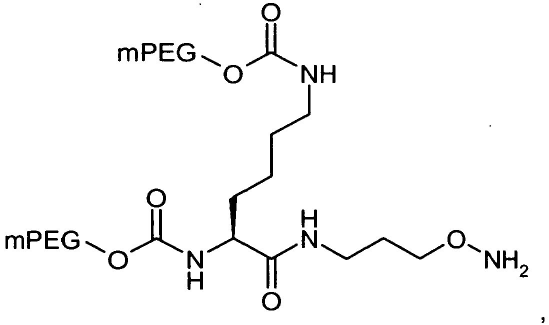 wherein mPEG has a molecular weight of 20 kDa, 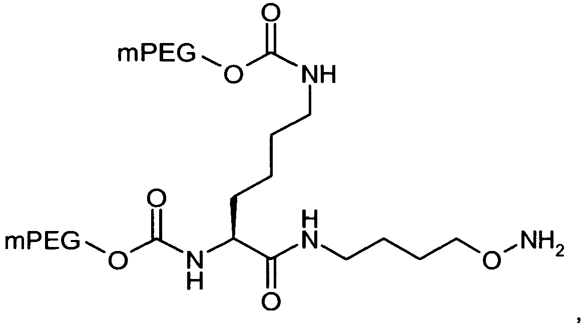 wherein mPEG has a molecular weight of 20 kDa, 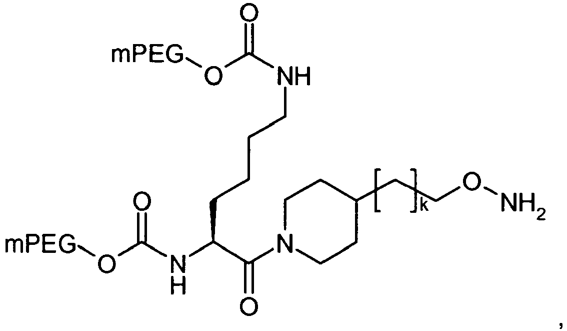 wherein mPEG has a molecular weight of 20 kDa, 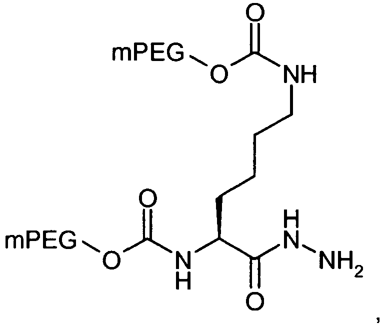 wherein mPEG has a molecular weight of 20 kDa, 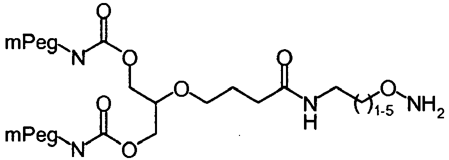 wherein mPEG has a molecular weight of 20 kDa or 30 kDa, 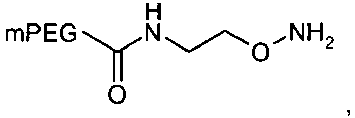 wherein mPEG has a molecular weight of 20 kDa, 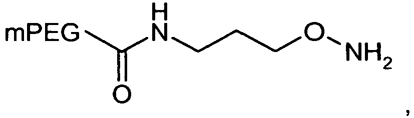 wherein mPEG has a molecular weight of 20 kDa, 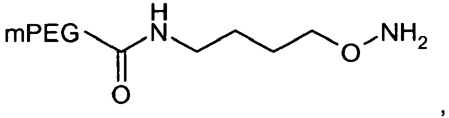 wherein mPEG has a molecular weight of 20 kDa, 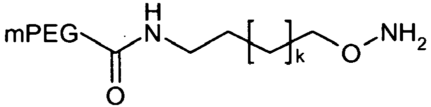 wherein mPEG has a molecular weight of 20 kDa, 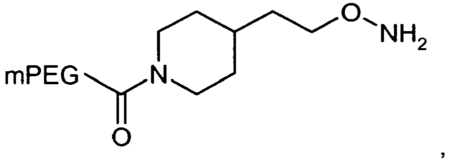 wherein mPEG has a molecular weight of 20 kDa, 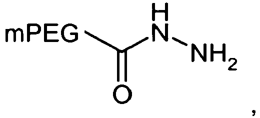 wherein mPEG has a molecular weight of 20 kDa, 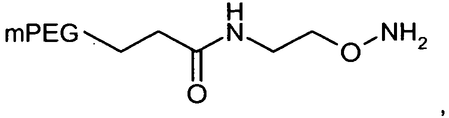 wherein mPEG has a molecular weight of 20 kDa, 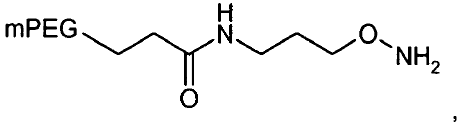 wherein mPEG has a molecular weight of 20 kDa, 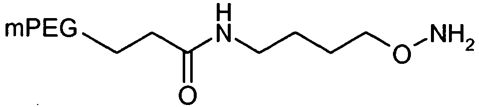 wherein mPEG has a molecular weight of 20 kDa, 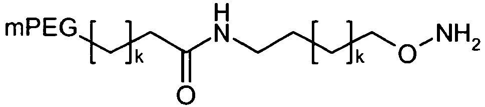 wherein mPEG has a molecular weight of 20 kDa, 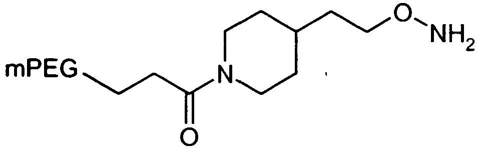 wherein mPEG has a molecular weight of 20 kDa, 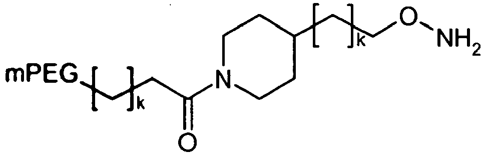 wherein mPEG has a molecular weight of 20 kDa, 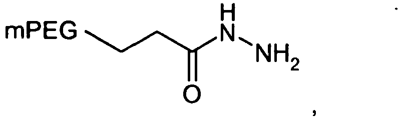 wherein mPEG has a molecular weight of 20 kDa, 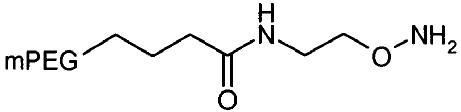 wherein mPEG has a molecular weight of 20 kDa, 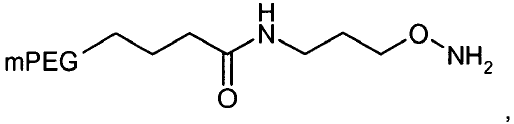 wherein mPEG has a molecular weight of 20 kDa, 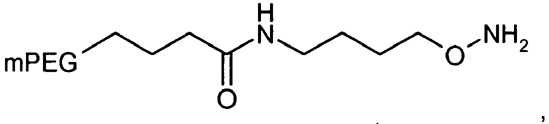 wherein mPEG has a molecular weight of 20 kDa, 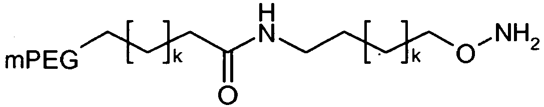 wherein mPEG has a molecular weight of 20 kDa, 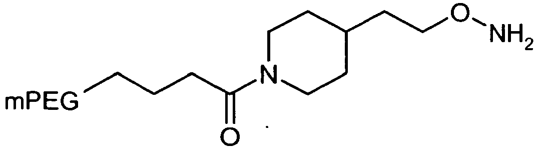 wherein mPEG has a molecular weight of 20 kDa, 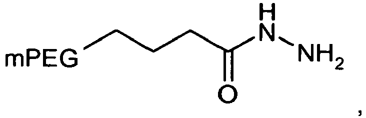 wherein mPEG has a molecular weight of 20 kDa, 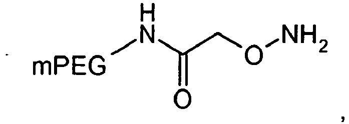 wherein mPEG has a molecular weight of 20 kDa, 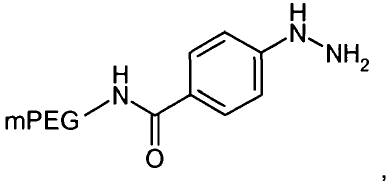 wherein mPEG has a molecular weight of 20 kDa, 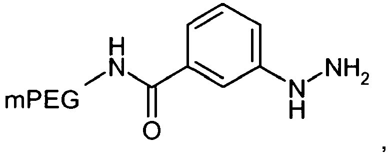 wherein mPEG has a molecular weight of 20 kDa, 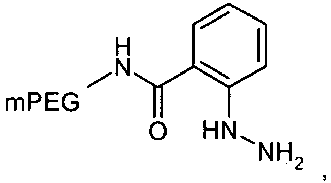 wherein mPEG has a molecular weight of 20 kDa, 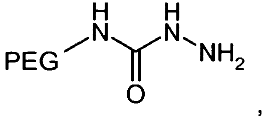 wherein mPEG has a molecular weight of 20 kDa, 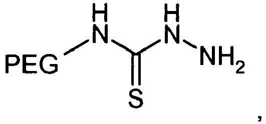 wherein mPEG has a molecular weight of 20 kDa, 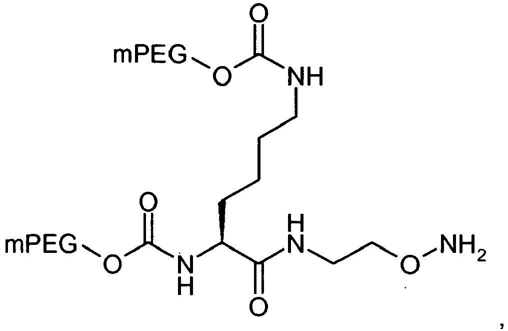 wherein mPEG has a molecular weight of 10 kDa, 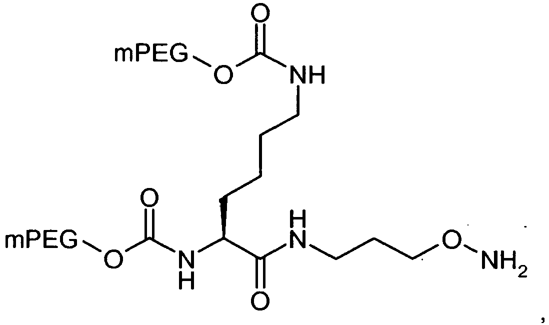 wherein mPEG has a molecular weight of 10 kDa, 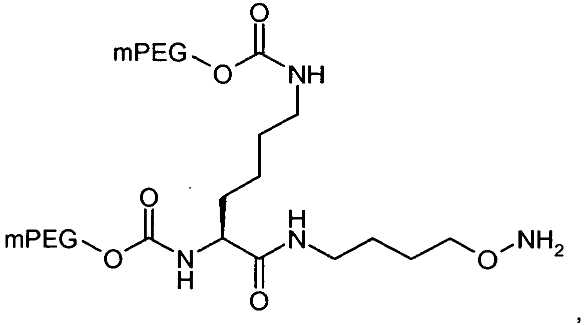 wherein mPEG has a molecular weight of 10 kDa, 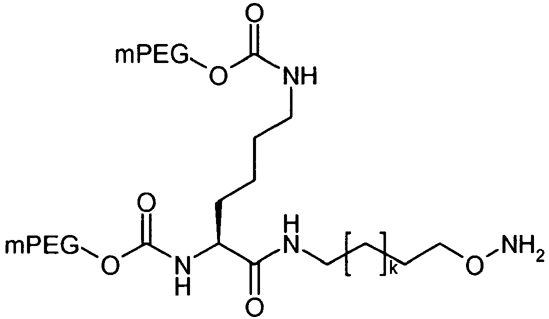 wherein mPEG has a molecular weight of 10 kDa, 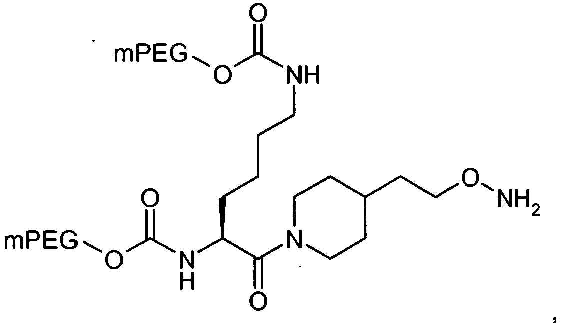 wherein mPEG has a molecular weight of 10 kDa, 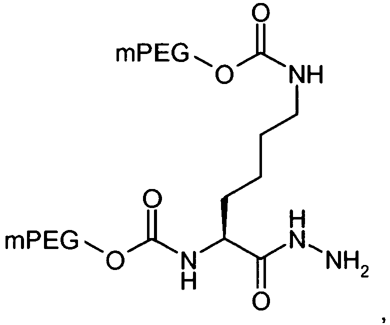 wherein mPEG has a molecular weight of 10 kDa, 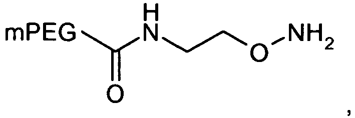 wherein mPEG has a molecular weight of 10 kDa, 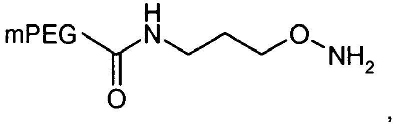 wherein mPEG has a molecular weight of 10 kDa, 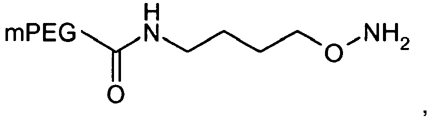 wherein mPEG has a molecular weight of 10 kDa, 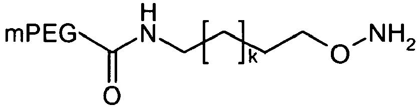 wherein mPEG has a molecular weight of 10 kDa, 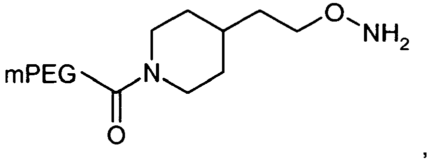 wherein mPEG has a molecular weight of 10 kDa, 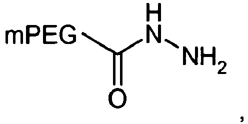 wherein mPEG has a molecular weight of 10 kDa, 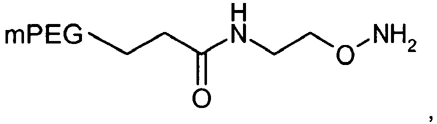 wherein mPEG has a molecular weight of 10 kDa, 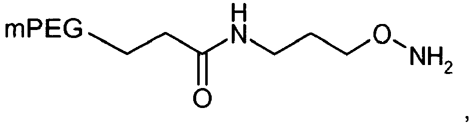 wherein mPEG has a molecular weight of 10 kDa, 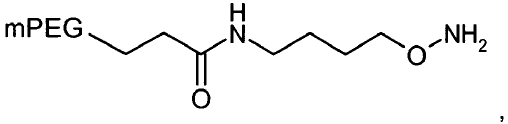 wherein mPEG has a molecular weight of 10 kDa, 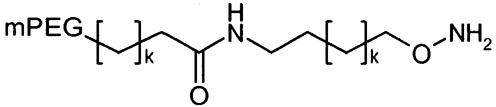 wherein mPEG has a molecular weight of 10 kDa, 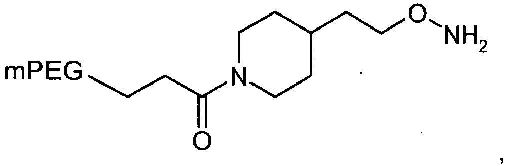 wherein mPEG has a molecular weight of 10 kDa, 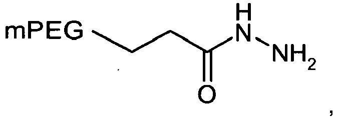 wherein mPEG has a molecular weight of 10 kDa, 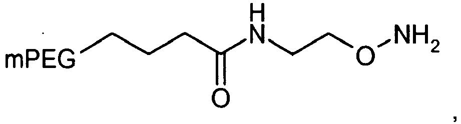 wherein mPEG has a molecular weight of 10 kDa, 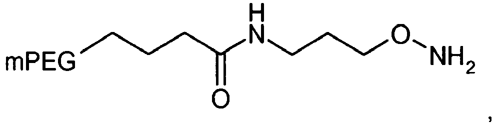 wherein mPEG has a molecular weight of 10 kDa, 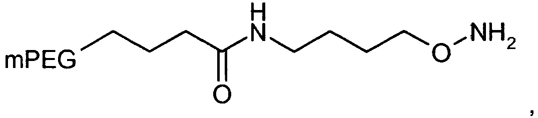 wherein mPEG has a molecular weight of 10 kDa, 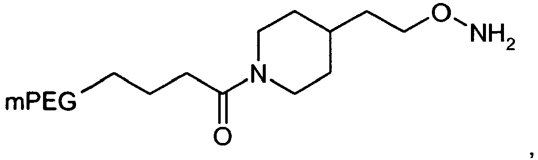 wherein mPEG has a molecular weight of 10 kDa, 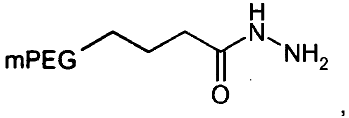 wherein mPEG has a molecular weight of 10 kDa, 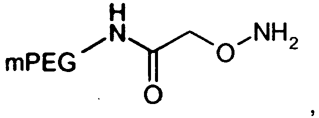 wherein mPEG has a molecular weight of 10 kDa, 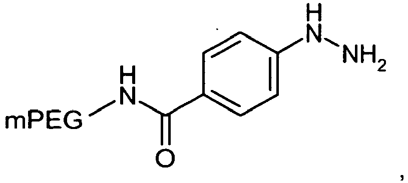 wherein mPEG has a molecular weight of 10 kDa, 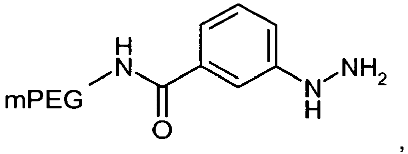 wherein mPEG has a molecular weight of 10 kDa, 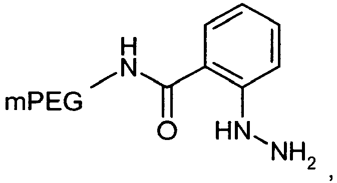 wherein mPEG has a molecular weight of 10 kDa, 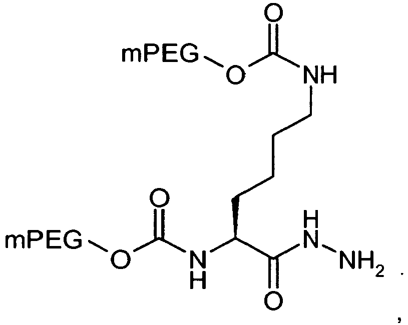 wherein mPEG has a molecular weight of 10 kDa, 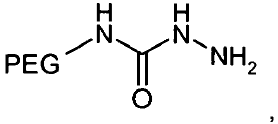 wherein mPEG has a molecular weight of 10 kDa, 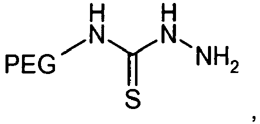 wherein mPEG has a molecular weight of 10 kDa, 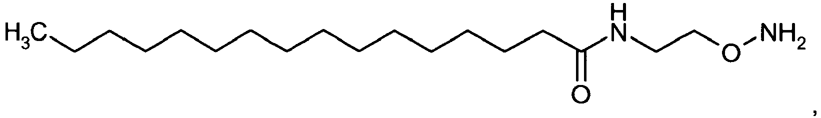 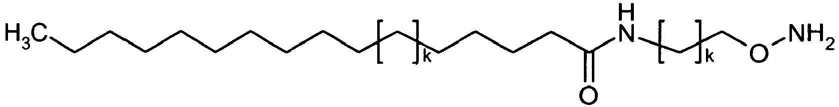 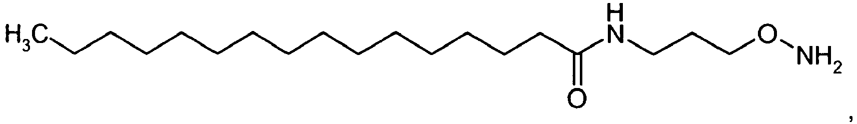 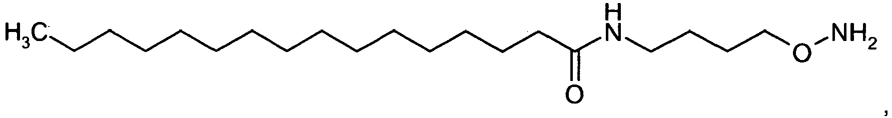 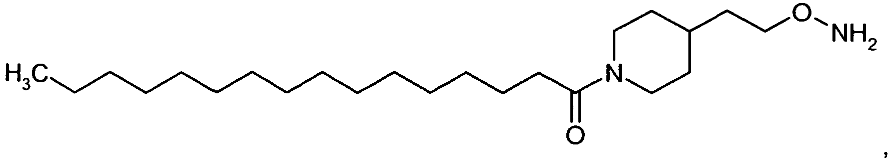 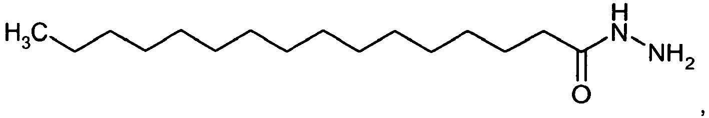 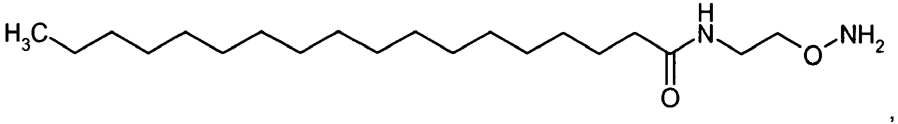 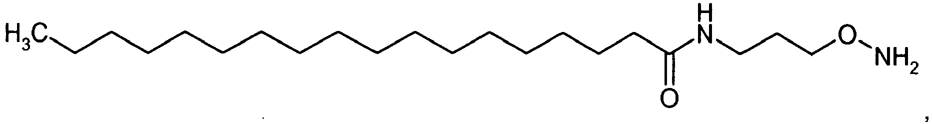 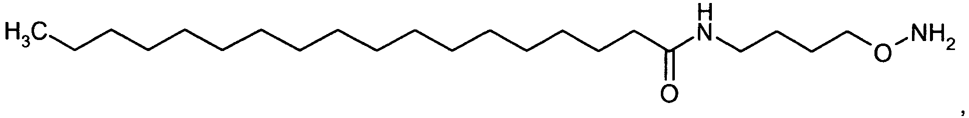 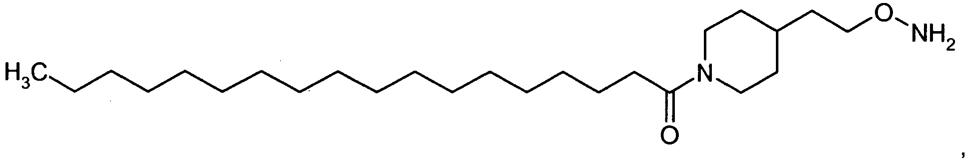 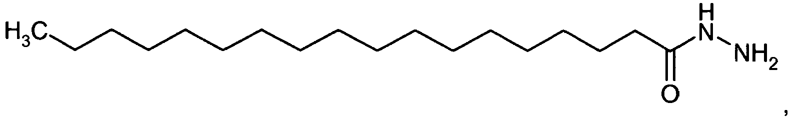 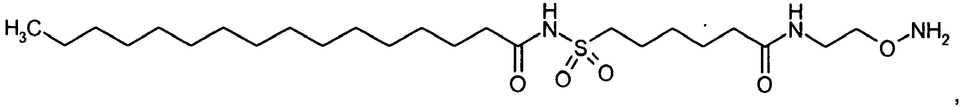 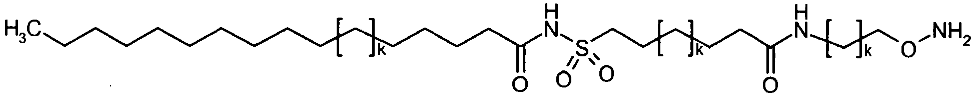 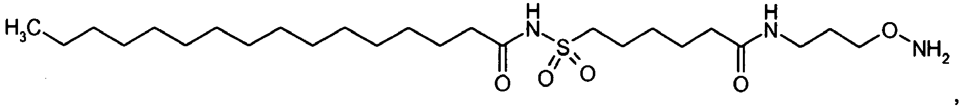 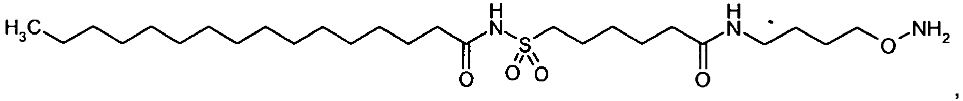 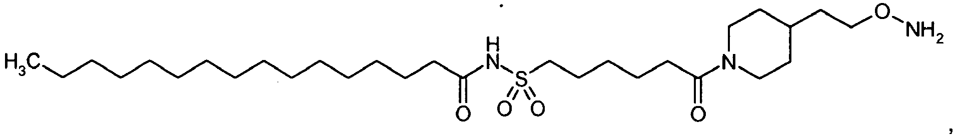 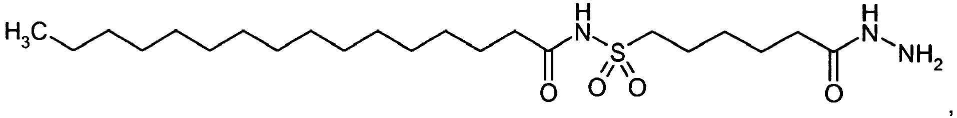 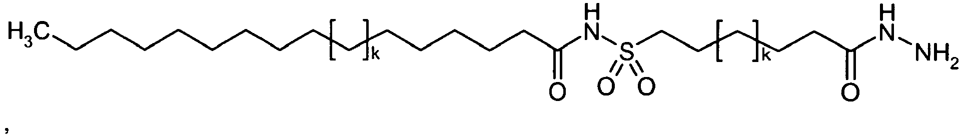 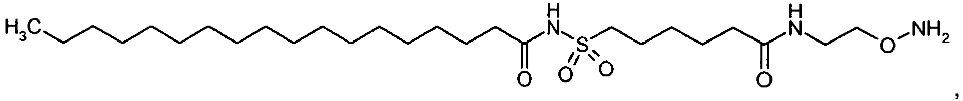 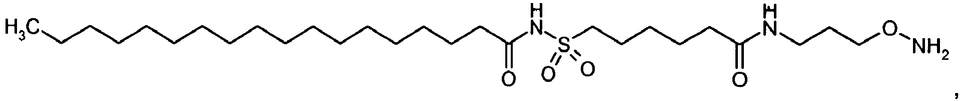 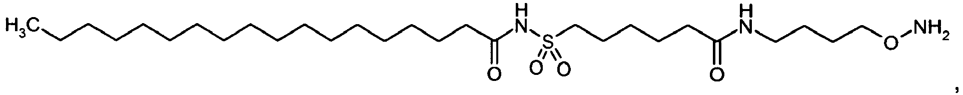 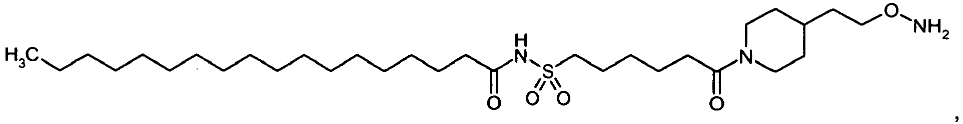 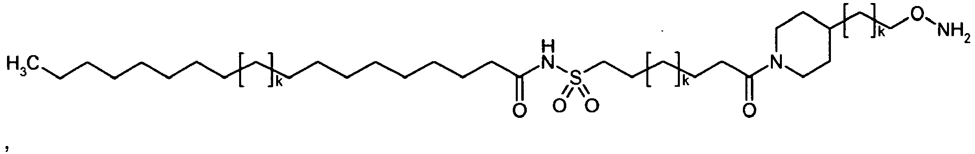 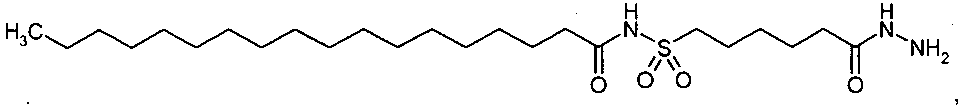 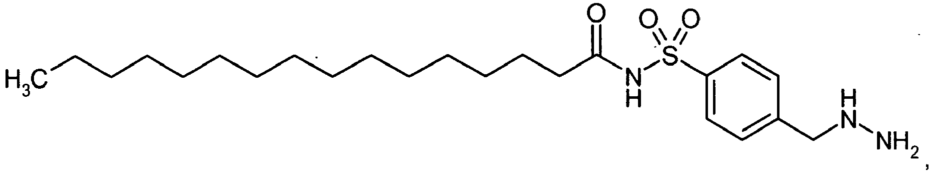 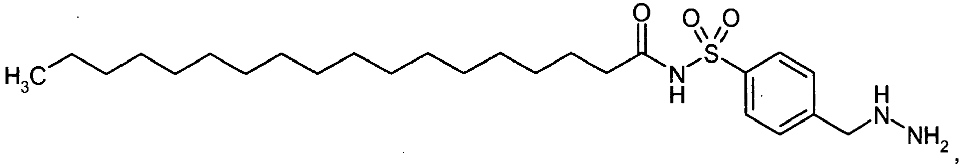 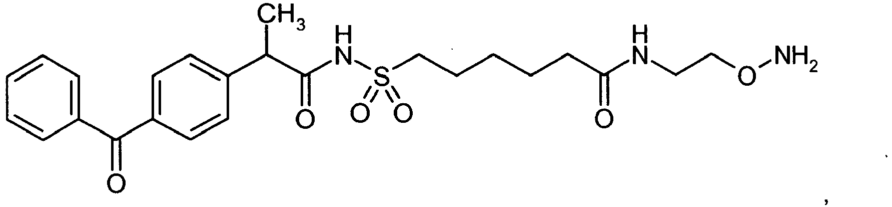 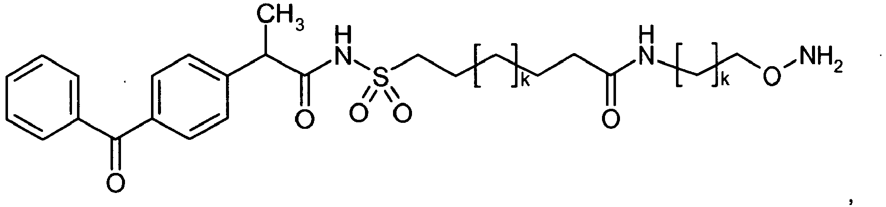 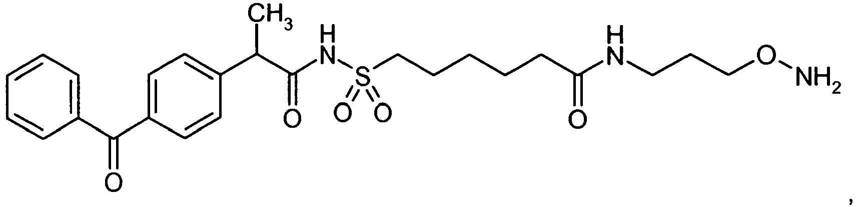 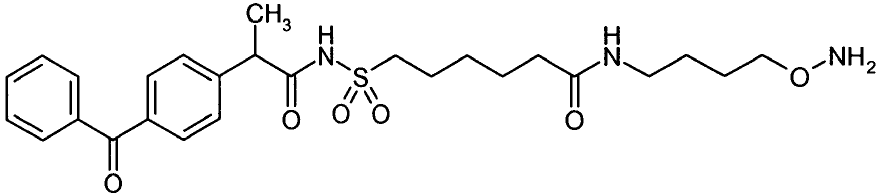 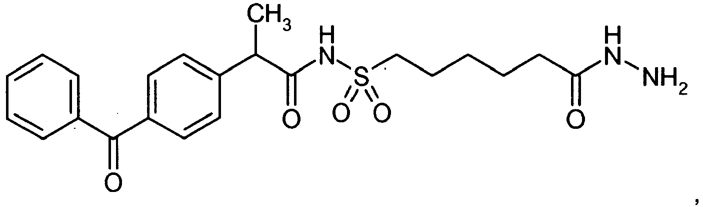 wherein each k in the above formulas independently represent an integer from 0 to 5, i.e. 0, 1, 2, 3, 4 or 5.

As discussed in the "Background of the invention" part, direct conjugation of e.g. amine functionalised PEG or fatty acids to Gln containing peptides is known. It is, however, clear from the examples disclosed in, e.g. EP 950665, EP 785276, Sato, Adv. Drug Delivery Rev., 54, 459-476, 2002 and Wada, Biotech. Lett., 23, 1367-1372, 2001 that it requires a significant excess (up to 100-1000 fold) of the compound to be conjugated to the peptide for the reaction to proceed. Such excess constitute a limitation to the utility of the reaction in technical or large scale. For instance, mPEG with a small poly dispersity index are very expensive, and a requirement for a large excess is in practise prohibitive. Moreover, for the conjugation of large moieties, such as e.g. PEG 10 kDa or PEG 20k Da, excess of the reagent in the order of 100-1000 fold is not feasible due to the molecular weight of such compounds. It is also well-known that the presence of large amounts of PEG is likely to precipitate peptides, i.e. both the peptide to be conjugated and the transglutaminase. In contrast hereto, the present two-step method offers the advantage that the reactant which in the enzymatic step is required in large excess is a small molecule which can easily be handled even in large excess. With a proper selection of the bond to be formed in the second step no large excess is required as e.g. oxime formation takes place at almost equimolar amounts of amine- and keto-functionalities.

A further advantage is the possibility to make "ready-to-conjugate" peptides. A peptide may be reacted with a suitable nucleophile (H₂N-D-R-X) in the presence of a transglutaminase to generate a functionalised peptide. Said functionalised peptide may then be stored as needed to be reacted later with one or more second compound (Y-E-Z) to generate various different conjugated peptides. This allows one functionalised peptide to be used to generate a multitude of conjugated peptides. In this way, numerous optimisations to identify appropriate reaction conditions can be avoided.

A peptide has to be a substrate for transglutaminase according to the methods of the present invention. It is thus a requirement that the peptide contains a Gln or a Lys residue, and in particular a Gln residue. If a given peptide is not a transglutaminase substrate it is possible to insert one or more Gln or Lys residues, and in particular Gln residues in the peptide sequence to make the peptide a substrate for transglutaminase. In principle , such Gln or Lys residue may be inserted at any position in the sequence, however, it is preferably inserted at a position where the physiological, such as the therapeutic activity of the peptide is not affected to a degree where the peptide is not useful anymore, e.g. in a therapeutic intervention. Insertions of amino acid residues in peptides can be brought about by standard techniques known to persons skilled in the art, such as post-translational chemical modification or transgenetic techniques.

Any peptide which are substrates to transglutaminase can be conjugated by the methods of the present invention, such as e.g. enzymes, peptide hormones, growth factors, antibodies, cytokines, receptors, lymphokines and vaccine antigenes, and particular mentioning is made of therapeutic peptides, such as insulin, glucagon like-peptide 1 (GLP-1), glucagon like-peptide 2 (GLP-2), growth hormone, cytokines, trefoil factor peptides (TFF), peptide melanocortin receptor modifiers and factor VII compounds.

Particular applicable insulin is human insulin. In the present context the term "human insulin" refers to naturally produced insulin or recombinantly produced insulin. Recombinant human insulin may be produced in any suitable host cell, for example the host cells may be bacterial, fungal (including yeast), insect, animal or plant cells. Many insulin compounds have been disclosed in the literature, and they too are particular useful in the methods of the present invention. By "insulin compound" (and related expressions) is meant human insulin in which one or more amino acids have been deleted and/or replaced by other amino acids, including non-codeable amino acids, and/or human insulin comprising additional amino acids, i.e. more than 51 amino acids, and/or human insulin in which at least one organic substituent is bound to one or more of the amino acids.

The following patent documents are mentioned as disclosures of insulin compounds particularly applicable in the methods provided by the present invention.

WO 97/31022 (Novo Nordisk) discloses insulin compounds with a protracted activity profile wherein the amino group of the N-terminal amino acid of the B-chain and/or the e-amino group of Lys^{B29} has a carboxylic acid containg lipophilic substituent. Particular mentioning is made of N^{εB29}-(CO-(CH₂)₁₄-COOH) human insulin; N^{εB29}-(CO-(CH₂)₁₆-COOH) human insulin; N^{ε129}-(CO-(CH₂)₁₈-COOH) human insulin; N^{εB29}-(CO-(CH₂)₂₀-COOH); N^{εB29}-(CO-(CH₂)₂₂-COOH) human insulin; N^{εB29}-(CO-(CH₂)₁₄-COOH) Asp^{B28} -human insulin; N^{εB29}-(CO-(CH₂)₁₆-COOH) Asp^{B28} -human insulin; N^{εB29}-(CO-(CH₂)₁₈-COOH) Asp^{B28} -human insulin; N^{εB29}-(CO-(CH₂)₂₀-COOH) Asp^{B28} -human insulin; N^{εB29}-(CO-(CH₂)₂₂-COOH) Asp^{B28} -human insulin; N^{εB30}-(CO-(CH₂)₁₄-COOH) Thr^{B29}Lys^{B30}-human insulin; N^{εB30}-(CO-(CH₂)₁₆-COOH) Thr^{B29}Lys^{B30}-human insulin; N^{εB30}-(CO-(CH₂)₁₈-COOH) Thr^{B29}Lys^{B30}-human insulin; N^{εB30}-(CO-(CH₂)₂₀-COOH) Thr^{B29}Lys^{B30}-human insulin; N^{εB30}-(CO-(CH₂)₂₂-COOH) Thr^{B29}Lys^{B30}-human insulin;

N^{εB28}-(CO-(CH₂)₁₄-COOH) Lys^{B28}Pro^{B29}-human insulin; N^{εB28}-(CO-(CH₂)₁₆-COOH) Lys^{B28}Pro^{B29}-human insulin; N^{εB28}-(CO-(CH₂)₁₈-COOH) Lys^{B28}Pro^{B29}-human insulin; N^{εB28}-(CO-(CH₂)₂₀-COOH) Lys^{B28}Pro^{B29}-human insulin; N^{εB28}-(CO-(CH₂)₂₂-COOH) Lys^{B28}Pro^{B29}-human insulin; N^{εB29}-(CO-(CH₂)₁₄-COOH) desB30 human insulin; N^{εB29}-(CO-(CH₂)₁₆-COOH) desB30 human insulin; N^{εB29}-(CO-(CH₂)₁₈-COOH) desB30 human insulin; N^{εB29}-(CO-(CH₂)₂₀-COOH) desB30 human insulin; and N^{εB29}-(CO-(CH₂)₂₂COOH) desB30 human insulin.

WO 96/29344 (Novo Nordisk), which is incoporated herein by reference, discloses insulin compounds with a protracted activity profile wherein either the amino group of the N-terminal amino acid of the B-chain has a lipophilic substituent comprising from 12 to 40 carbon atoms attached, or wherein the carboxylic acid group of the C-terminal amino acid of the B-chain has a lipophilic substituent comprising from 12 to 40 carbon atoms attached.

WO 95/07931 (Novo Nordisk) discloses insulin compounds with a protracted activity profile, wherein the ε-amino group of Lys^{B29} has a lipophilic substituent. Particular mentioning is made of N^{εB29}-tridecanoyl des(B30) human insulin, N^{εB29}-tetradecanoyl des(B30) human insulin, N^{εB29}-decanoyl des(B30) human insulin, N^{εB29}-dodecanoyl des(B30) human insulin, N^{εB29}-tridecanoyl Gly^{A21} des(B30) human insulin, N^{εB29}-tetradecanoyl Gly^{A21} des(B30) human insulin, N^{εB29}-decanoyl Gly^{A21} des(B30) human insulin, N^{εB29}-dodecanoyl Gly^{A21} des(B30) human insulin, N^{εB29}-tridecanoyl Gly^{A21} Gln^{B3} des(B30) human insulin, N^{εB29}-tetradecanoyl Gly^{A21} Gln^{B3} des(B30) human insulin, N^{εB29}-decanoyl Gly^{A21} Gln^{B3} des(B30) human insulin, N^{εB29}-dodecanoyl Gly^{A21} Gln^{B3} des(B30) human insulin, N^{εB29}-tridecanoyl Ala^{A21} des(B30) human insulin, N^{εB29} -tetradecanoyl Ala^{A21} des(B30) human insulin, N^{εB29}-decanoyl Ala^{A21} des(B30) human insulin, N^{εB29}-dodecanoyl Ala^{A21} des(B30) human insulin, N^{εB29}-tridecanoyl Ala^{A21} Gln^{B3} des(B30) human insulin, N^{εB29}-tetradecanoyl Ala^{A21} Gln^{B3} des(B30) human insulin, N^{εB29}-decanoyl Ala^{A21} Gln^{B3} des(B30) human insulin, N^{εB29}-dodecanoyl Ala^{A21} Gln^{B3} des(B30) human insulin, N^{εB29}-tridecanoyl Gln^{B3} des(B30) human insulin, N^{εB29}-tetradecanoyl Gln^{B3} des(B30) human insulin, N^{εB29}-decanoyl Gln^{B3} des(B30) human insulin, N^{εB29}-dodecanoyl Gln^{B3} des(B30) human insulin, N^{εB29}-tridecanoyl Gly^{A21} human insulin, N^{εB29}-tetradecanoyl Gly^{A21} human insulin, N^{εB29}-decanoyl Gly^{A21} human insulin, N^{εB29}-dodecanoyl Gly^{A21} human insulin, N^{εB29}-tridecanoyl Gly^{A21} Gln^{B3} human insulin, N^{εB29}-tetradecanoyl Gly^{A21} Gln^{B3} human insulin, N^{εB29}-decanoyl Gly^{A21} Gln^{B3} human insulin, N^{εB29}-dodecanoyl Gly^{A21} Gln^{B3} human insulin, N^{εB29}-tridecanoyl Ala^{A21} human insulin, N^{εB29}-tetradecanoyl Ala^{A21} human insulin, N^{εB29}-decanoyl Ala^{A21} human insulin, N^{εB29}-dodecanoyl Ala^{A21} human insulin, N^{εB29}-tridecanoyl Ala^{A21} Gln^{B3} human insulin, N^{εB29}-tetradecanoyl Ala^{A21} Gln^{B3} human insulin, N^{εB29}-decanoyl Ala^{A21} Gln^{B3} human insulin, N^{εB29}-dodecanoyl Ala^{A21} Gln^{B3} human insulin, N^{εB29}-tridecanoyl Gln^{B3} human insulin, N^{εB29}-tetradecanoyl Gln^{B3} human insulin, N^{εB29}-decanoyl Gln^{B3} human insulin, N^{εB29}-dodecanoyl Gln^{B3} human insulin, N^{εB29}-tridecanoyl Glu^{B30} human insulin, N^{εB29}-tetradecanoyl Glu^{B30} human insulin, N^{εB29}-decanoyl Glu^{B30} human insulin, N^{εB29}-dodecanoyl Glu^{B30} human insulin, N^{εB29}-tridecanoyl Gly^{A21} Glu^{B30} human insulin, N^{εB29}-tetradecanoyl Gly^{A21} Glu^{B30} human insulin, N^{εB29}-decanoyl Gly^{A21} Glu^{B30} human insulin, N^{εB29}-dodecanoyl Gly^{A21} Glu^{B30} human insulin, N^{εB29}-tridecanoyl Gly^{A21} Gln^{B3} Glu^{B30} human insulin, N^{εB29}-tetradecanoyl Gly^{A21} Gln^{B3} Glu^{B30} human insulin, N^{εB29}-decanoyl Gly^{A21} Gln^{B3} Glu^{B30} human insulin, N^{εB29}-dodecanoyl Gly^{A21} Gln^{B3} Glu^{B30} human insulin, N^{εB29}-tridecanoyl Ala^{A21} Glu^{B30} human insulin, N^{εB29}-tetradecanoyl Ala^{A21} Glu^{B30} human insulin, N^{εB29}-decanoyl Ala^{A21} Glu^{B30} human insulin, N^{εB29}-dodecanoyl Ala^{A21} Glu^{B30} human insulin, N^{εB29}-tridecanoyl Ala^{A21} Gln^{B3} Glu^{B30} human insulin, N^{εB29}-tetradecanoyl Ala^{A21} Gln^{B3} Glu^{B30} human insulin, N^{εB29}-decanoyl Ala^{A21} Gln^{B3} Glu^{B30} human insulin, N^{εB29}-dodecanoyl Ala^{A21} Gln^{B3} Glu^{B30} human insulin, N^{εB29}-tridecanoyl Gln^{B3} Glu^{B30} human insulin, N^{εB29}-tetradecanoyl Gln^{B3} Glu^{B30} human insulin, N^{εB29}-decanoyl Gln^{B3} Glu^{B30} human insulin and N^{εB29}-dodecanoyl Gln^{B3} Glu^{B30} human insulin.

WO 97/02043 (Novo Nordisk) discloses hormonally inactive insulin compounds which are useful in insulin prophylaxis, and in particular such analogues of human insulin are selected from amongst desA1 human insulin; des(A1-A2) human insulin; des(A1-A3) human insulin; desA21 human insulin; des(B1-B5) human insulin; des(B1-B6) human insulin; des(B23-B30) human insulin; des(B24-B30) human insulin; des(B25-B30) human insulin; Gly^{A2} human insulin; Ala^{A2} human insulin; Nle^{A2} human insulin; Thr^{A2} human insulin; Pro^{A2} human insulin; D-allo Ile^{A2} human insulin; Nva^{A3} human insulin; Nle^{A3} human insulin; Leu^{A3} human insulin; Val^{A2},Ile^{A3} human insulin; Abu^{A2},Abu^{A3} human insulin; Gly^{A2},Gly^{A3} human insulin; D-Cys^{A6} human insulin; D-Cys^{A6},D-Cys^{A11} human insulin; Ser^{A6},Ser^{A11},des(A8-A10) human insulin; D-Cys^{A7} human insulin; D-Cys^{A11} human insulin; Leu^{A19} human insulin; Gly^{B6} human insulin; Glu^{B12} human insulin; Asn^{B12} human insulin; Phe^{B12} human insulin; D-Ala^{B12} human insulin; and Asp^{B25} human insulin are applicable in the methods of the present invention.

WO 92/15611 (Novo nordisk) discloses analogues of human insulin with a fast association rate constants in the insulin receptor binding process and characterised by comprising a tyrosine in position A13 and/or a phenylalanin, tryptophane or tyrosine in position B17. In particular, such analogues are selected from amongst Tyr^{A13} human insulin, Phe^{B17} human insulin, Trp^{B17} human insulin, Tyr^{B17} human insulin, Tyr^{A13},Phe^{B17} human insulin, Tyr^{A13},Trp^{B17} human insulin, Tyr^{A13},Tyr^{B17} human insulin, Phe^{A13},Phe^{B17} human insulin, Phe^{A13},Trp^{B17} human insulin, Phe^{A13},Tyr^{B17} human insulin, Trp^{A13},Phe^{B17} human insulin, Trp^{A13},Trp^{B17} human insulin and Trp^{A13},Tyr^{B17} human insulin.

WO 92/00322 (Novo Nordisk), discloses analogues of human insulin which are capable of being targeted to specific tissues, and which are characterized by having in the A13 position and/or in the B17 position in the insulin molecule a naturally occurring amino acid residue different from leucine and/or by having in the B18 position in the insulin molecule a naturally occurring amino acid residue different from valine. In particular, such analogues are selected from amongst Ala^{B17} human insulin, Ala^{B18} human insulin, Asn^{A13} human insulin, Asn^{A13},Ala^{B17} human insulin, Asn^{A13},Asp^{B17} human insulin, Asn^{A13},Glu^{B17} human insulin, Asn^{B18} human insulin, Asp^{A13} human insulin, Asp^{A13},Ala^{B17} human insulin, Asp^{A13},Asp^{B17} human insulin, Asp^{A13},Glu^{B17} human insulin, Asp^{B18} human insulin, Gln^{A13} human insulin, Gln^{A13},Ala^{B17} human insulin, Gln^{A13},Asp^{B17} human insulin, Gln^{B18} human insulin, Glu^{A13} human insulin, Glu^{A13},Ala^{B17} human insulin, Glu^{A13},Asp^{B17} human insulin, Glu^{A13},Glu^{B17} human insulin, Glu^{B18} human insulin, Gly^{A13} human insulin, Gly^{A13},Ala^{B17} human insulin, Gly^{A13},Asn^{B17} human insulin, Gly^{A13},Asp^{B17} human insulin, Gly^{A13},Glu^{B17} human insulin, Gly^{B18} human insulin, Ser^{A13} human insulin, Ser^{A13},Gln^{A17},Glu^{B10},Gln^{B17}-des(Thr^{B30}) human insulin, Ser^{A13},Ala^{B17} human insulin, Ser^{A13},Asn^{B17} human insulin, Ser^{Al3},Asp^{B17} human insulin, Ser^{A13},Gln^{B17} human insulin, Ser^{A13},Glu^{B17} human insulin, Ser^{A13},Thr^{B17} human insulin, Ser^{B14},Asp^{B17} human insulin, Ser^{B18} human insulin, Thr^{A13} human insulin or Thr^{B18} human insulin.

WO 90/01038 (Novo Nordisk) discloses analogues of human insulin with high biological activity and characterized by having Phe^{B25} substituted by His or Tyr, by having substitutions in one or more of positions A4, A8, A17, A21, B9, B10, B12, B13, B21, B26, B27, B28 and B30, and by having the amino acid residue at position B30 optionally absent. In particular, such analogues are selected from amongst Tyr^{B25} human insulin, Tyr^{B25},Asp^{B28} human insulin, His^{B25} human insulin, His^{B25},Asp^{B28} human insulin, Tyr^{B25} human insulin -B30-amide and His^{B25} human insulin-B30-amide.

WO 86/05496 (Nordisk Gentofte) discloses analogues of human insulin with a protracted action and characterized by having a blocked B30 carboxylic group, and by having one to four blocked carboxylic groups in the amino acid residues at positions A4, A17, A21, B13 and B21. In particular, such analogues are selected from amongst insulin-B30-octyl ester, insulin-B30-dodecyl amide, insulin-B30-hexadecyl amide, insulin-(B21,B30)-dimethyl ester, insulin-(B17,B30)-dimethyl ester, insulin-(A4,B30) diamide, insulin-A17amide-B30-octyl ester, insulin-(A4,B13)-diamide-B30-hexylamide, insulin-(A4,A17,B21,B30)-tetraamide, insulin-(A17,B30)-diamide, A4-Ala-insulin-B30-amide and B30-Leu-insulin-(A4,B30)-diamide.

WO 86/05497(Nordisk Gentofte) discloses insulin compounds in which one or more of the four amino acid residues in positions A4, A17, B13 and B21 comprises an uncharged side chain. Particular mentioning is made of human insulin A17-Gln, human insulin A4-Gln, porcine insulin B21-Gln, human insulin B13-Gln, human insulin (A17,B21)-Gln, human insulin A4-Ala, human insulin B21-Thr, human insulin B13-Val, human insulin-Thr-A17-Gln, human insulin B21-methyl ester and human insulin A17-methyl ester.

WO 92/00321 (Novo Nordisk), discloses insulin compounds with prolonged activity wherein a positive charge in the N-terminal end of the B-chain has been introduced. Particular mentioning is made of Arg^{B5},Ser^{A21},Thr^{B30}-NH₂ human insulin, Arg^{B5},Pro^{B6},Ser^{A21},Thr^{B30} -NH₂ human insulin, Arg^{B5},Gly^{A21},Thr^{B30}-NH₂ human insulin, Arg^{B5},Pro^{B6},Gly^{A21},Thr^{B30}-NH₂ human insulin, Arg^{B2},Ser^{A21},Thr^{B30}-NH₂ human insulin, Arg^{B2},Pro^{B3},Ser^{A21},Thr^{B30}-NH₂ human insulin, Arg^{B2},Gly^{A21},Thr^{B30}-NH₂ human insulin, Arg^{B2},Pro^{B3},Gly^{A21},Thr^{B30}-NH₂ human insulin, Arg^{B2},Arg^{B3},Ser^{A21},Thr^{B30}-NH₂ human insulin, Arg^{B2},Arg^{B3},Ser^{A21} human insulin, Arg^{B4},Pro^{B5},Ser^{A21},Thr^{B30}-NH₂ human insulin, Arg^{B4},Arg^{B5},Pro^{B6},Gly^{A21} ,Thr^{B30} human insulin, Arg^{B3},Gly^{A21},Thr^{B30}-NH₂ human insulin, Arg^{B3},Ser^{A21},Thr^{B30}-NH₂ human insulin, Arg^{B4},Gly^{A21},Thr^{B30}-NH₂ human insulin, Arg^{B4},Ser^{A21},Thr^{B30}-NH₂ human insulin and Arg^{B1},Pro^{B2},Gly^{A21},Thr^{B30}-NH₂ human insulin.

WO 90/07522 (Novo Nordisk) discloses insulin compounds exhibiting a low ability to associate in solution wherein there is a positively charged amino acid residue, i.e. Lys or Arg in the position B28. Particular mentioning is made of des[Phe^{B25}]-human insulin, des[Tyr^{B26}]-human insulin, des[Thr^{B27}]-human insulin, des[Pro^{B28}]-human insulin, des[Phe^{B25}]-porcine insulin, des[Pro^{B28}]-porcine insulin, des[Pro^{B28}]-rabbit insulin, des[Phe^{B25}],des[Th^{B30}]-human insulin, des[Tyr^{B26}],des[Thr^{B30}]-human insulin, [Se^{A21}]-des[Pro^{B28}]-human insulin, [Gly^{A21}]-des[Pro^{B28}]-human insulin, [Gly^{A21}]-des[Phe^{B25}]-human insulin, [Asp^{A21}]-des[Phe^{B25}]-human insulin, [His^{B25}]-des[Tyr^{B25}],des[Th^{B30}]-human insulin, [Asn^{B25}]-des[Tyr^{B26}],des[Thr^{B30}]-human insulin, [Asp^{A21}]-des[Phe^{B25}],des[Thr^{B30}]-human insulin, [Asp^{B28}]-des[Phe^{B25}]-human insulin, [Asp^{B3}]-des[Phe^{B25}]-human insulin, [Lys^{B28}]-human insulin, [Lys^{B28},Th^{B29}]-human insulin and [Arg^{B28}]-des[Lys^{B29}]-human insulin.

WO 90/11290 (Novo Nordisk) discloses insulin compounds with a prolonged activity. Particular mentioning is made of [Arg^{A0}]-human insulin-(B30-amide), [Arg^{A0},Gln^{B13}]-human insulin-(B30-amide), [Arg^{A0},Gln^{A4},Asp^{A21}]-human insulin-(B30-amide), [Arg^{A0},Ser^{A21}]-human insulin-(B30-amide) and [Arg^{A0},Arg^{B27}]-des[Thr³⁰]-human insulin.

WO 90/10645 (Novo Nordisk) discloses glycosylated insulins. Particular mentioning is made of Phe(B1) glucose human insulin, Phe(B1) mannose human insulin, Gly(A1) mannose human insulin, Lys(B29) mannose human insulin, Phe(B1) galactose human insulin, Gly(A1) galactose human insulin, Lys(B29) galactose human insulin, Phe(B1) maltose human insulin, Phe(B1) lactose human insulin, Gly(A1) glucose human insulin, Gly(A1) maltose human insulin, Gly(A1) lactose human insulin, Lys(B29) glucose human insulin, Lys(B29) maltose human insulin, Lys(B29) lactose human insulin, Gly(A1),Phe(B1) diglucose human insulin, Gly(A1),Lys(B29) diglucose human insulin, Phe(B1),Lys(B29) diglucose human insulin, Phe(B1) isomaltose human insulin, Gly(A1) isomaltose human insulin, Lys(B29) isomaltose human insulin, Phe(B1) maltotriose human insulin, Gly(A1) maltotriose human insulin, Lys(B29) maltotriose human insulin, Gly(A1),Phe(B1) dimaltose human insulin, Gly(A1),Lys(B29) dimaltose human insulin, Phe(B1),Lys(B29) dimaltose human insulin, Gly(A1),Phe(B1) dilactose human insulin, Gly(A1),Lys(B29) dilactose human insulin, Phe(B1),Lys(B29) dilactose human insulin, Gly(A1),Phe(B1) dimaltotriose human insulin, Gly(A1),Lys(B29) dimaltotriose human insulin, Phe(B1),Lys(B29) dimaltotriose human insulin, Phe(B1),Gly(A1) dimannose human insulin, Phe(B1),Lys(B29) dimannose human insulin, Gly(A1),Lys(B29) dimannose human insulin, Phe(B1),Gly(A1) digalactose human insulin, Phe(B1),Lys(B29) digalactose human insulin,

Gly(A1),Lys(B29) digalactose human insulin, Phe(B1),Gly(A1) diisomaltose human insulin, Phe(B1),Lys(B29) diisomaltose human insulin, Gly(A1),Lys(B29) diisomaltose human insulin, Phe(B1) glucose [Asp^{B10}] human insulin and Gly(A1),Phe(B1) diglucose [Asp^{B10}] human insulin.

WO 88/065999 (Novo Nordisk) discloses stabilized insulin compounds, wherein Ans^{21A} has been substituted with other amino acid residues. Particular mentioning is made of Gly^{A21} human insulin, Ala^{A21} human insulin, Ser^{A21} human insulin, Thr^{A21} human insulin and hSer^{A21} human insulin.

EP 254516 (Novo Nordisk) discloses insulin compounds with a prolonged action, wherein basic amino acid residues have been substituted by neutral amino acid residues. Particular mentioning is made of
Gly^{A21},Lys^{B27},Thr^{B30}-NH₂ human insulin, Ser^{A21},Lys^{B27} ,Thr^{B30}-NH₂ human insulin, Thr^{A21},Lys^{B27},Thr^{B20}-NH₂ human insulin, Ala^{B21},Lys^{B27},Thr^{B30}-NH₂ human insulin, His^{A21},Lys^{B27},Thr³⁰-NH₂ human insulin, Asp^{B21},Lys^{B27},Thr^{B30}-NN₂ human Insulin, Gly^{A21},Arg^{B21},Thr^{B30}-NH₂ human insulin, Ser^{A21} ,Arg^{B27}, Thr^{B30}- NH₂ human insulin, Thr^{A21},Arg^{B27},Thr^{B30}- NH₂ human insulin, Ala^{B21},Arg^{B27},Thr^{B30}-NH₂ human insulin, His^{A21},Arg^{B27},Thr^{B30}- NH₂ human insulin, Asp^{B21},Arg^{B27},Thr^{B30}- NH₂ human insulin, Gln^{B13},Gly^{A21},Arg^{B27},Thr^{B30}-NH₂ human insulin, Gln^{B13},Ser^{A21},Thr^{B30}-NH₂ human insulin, Gln^{B13},Ser^{A21},Arg^{B27},Thr^{B30}-NH₂ human insulin, Gln^{B13},Thr^{A21},Arg^{B27},Thr^{B30}-NH₂ human insulin, Gln^{B13},Ala^{A21},Arg^{B27},Thr^{B30}-NH₂ human insulin, Gln^{B13},His^{A21},Arg^{B27},Thr^{B30}-NH₂ human insulin, Gln^{B13},Asp^{A21},Arg^{B27},Thr^{B30}-NH₂ human insulin, Gln^{B13},Gly^{A21},Lys^{B27},Thr^{B27}-NH₂ human insulin, Gln^{B13},Ser^{A21},Lys^{B27},Thr^{B30}-NH₂ human insulin, Gln^{B13},Thr^{A21},Lys^{B27},Thr^{B30}-NH₂ human insulin, Gln^{B13},Ala^{A21},Lys^{B27},Thr^{B30}-NH₂ human insulin, Gln^{B13},His^{A21},Lys^{B27},Thr^{B30}-NH₂ human insulin, Gln^{B13},Asp^{A21},Lys^{B27},Thr^{B30}-NH₂ human insulin, Asn^{A21},Lys^{B27} human insulin, Ser^{A21},Lys^{B27} human insulin, Thr^{A21}, Lys^{B27} human insulin, Ala^{A21},Lys^{B27} human insulin, His^{A21},Lys^{B27} human insulin, Asp^{A21},Lys^{B27} human insulin, Gly^{A21},Lys^{B27} human insulin, Asn^{A21},Arg^{B27} human insulin, Ser^{A21},Arg^{B27} human insulin, Thr^{A21},Arg^{B27} human insulin, Ala^{A21},Arg^{B27} human insulin, His^{A21},Arg^{B27} human insulin, Asp^{A21},Arg^{B27} human insulin, Gly^{A21},Arg^{B27} human insulin, Gln^{A17},Asn^{A21},Arg^{B27}human insulin, Gln^{A17},Ser^{A21},Arg^{B27}human insulin, Gln^{A17},Thr^{A21},Arg^{B27}human insulin, Gln^{A17},Ala^{A21},Arg^{B27}human insulin, Gln^{A17},His^{A21},Arg^{B27}human insulin, Gln^{A17} Asp^{A21},Arg^{B27}human insulin, Gln^{A17},Gly^{A21},Arg^{B27}human insulin, Gln^{A17},Asn^{A21},Gln^{B13}human insulin, Gln^{A17},Ser^{A21},Gln^{B13}human insulin, Gln^{A17},Thr^{A21},Gln^{B13}human insulin, Gln^{A17},Ala^{A21},Gln^{B13}human insulin, Gln^{A17},His^{A21},Gln^{B13}human insulin, Gln^{A17},Asp^{A2},Gln^{B13}human insulin, Gln^{A17},Gly^{A21},Gln^{B13}human insulin, Arg^{A27},Asn^{A21},Gln^{B13}human insulin, Arg^{A27},Ser^{A21},Gln^{B13}human insulin, Arg^{A27},Thr^{A21},Gln^{B13}human insulin, Arg^{A27},Ala^{A21},Gln^{B13}human insulin, Arg^{A27},HisA²¹,Gln^{B13}human insulin, Arg^{A27},Asp^{A21},Gln^{B13}human insulin, Arg^{A27},Gly^{A21},Gln^{B13}human insulin, Gln^{A17},Asn^{A21},Lys^{B27}human insulin, Gln^{A17},Ser^{A21},Lys^{B27}human insulin, Gln^{A17},Thr^{A21},Lys^{B27}human insulin, Gln^{A17},Ala^{A21},Lys^{B27}human insulin, Gln^{A17},His^{A21},Lys^{B27}human insulin, Gln^{A17},Asp^{A2},Lys^{B27}human insulin, Gln^{A17},Gly^{A21},Lys^{B27}human insulin, Gln^{B13},Asn^{A21},Lys^{B27}human insulin, Gln^{B13},Ser^{A21},Lys^{B27}human insulin, Gln^{B13},Thr^{A21},Lys^{B27}human insulin, Gln^{B13},Ala^{A21},Lys^{B27}human insulin, Gln^{B13},His^{A21},Lys^{B27}human insulin, Gln^{B13},Asp^{A21},Lys^{B27}human insulin, and Gln^{B13},Gly^{A21},Lys^{B27}human insulin.

EP 214826 (Novo Nordisk) discloses rapid onset insulin compounds.

EP 194864 (Novo Nordisk) discloses insulin compounds with a prolonged action, wherein basic amino acid residues have been substituted by neutral amino acid residues. Particular mentioning is made of Gln^{A17},Arg^{B27},Thr^{B30}-NH₂ human insulin, Gln^{A17},Gln^{B13},Thr^{B30}-NH₂ human insulin, Gln^{A17},Lys^{B27},Thr^{B30}-NH₂ human insulin, Gln^{A17},Lys^{B27}-NH₂ human insulin, Gln^{A17}, Gln^{A17},Thr^{B30}-NH₂ human insulin, Gln^{B13},Arg^{B27},Thr^{B30}-NH₂ human insulin, Gln^{B13},Lys^{B27},Thr^{B30}-NH₂ human insulin, Gln^{B13},Lys^{B30}-NH₂ human insulin, Gln^{B13},Thr^{B30}-NH₂ human insulin, Arg^{B27},Arg^{B30}- NH₂ human insulin, Arg^{B27},Lys^{B30}- NH₂ human insulin, Arg^{B27},Thr^{B30}- NH₂ human insulin, Lys^{B27},Arg^{B30}- NH₂ human insulin, Lys^{B27},Lys^{B30}- NH₂ human insulin, Lys^{B27},Thr^{B30}- NH₂ human insulin, Lys^{B29}-NH₂,des-(B30)human insulin, Thr^{B30}-NH₂ human insulin, Lys^{B30}- NH₂ human insulin, Lys^{B30}(Lau)- NH₂ human insulin, Lys^{B30},Arg^{B31}- NH₂ human insulin, Lys^{B30},Lys^{B31}- NH2 human insulin, Arg^{B30}- NH₂ human insulin, Arg^{B30},Arg^{B31}- NH₂ human insulin, and Arg^{B30},Lys^{B31}- NH₂ human insulin.

US Patent No. 3,528,960 (Eli Lilly) discloses N-carboxyaroyl insulin compounds in which one, two or three primary amino groups of the insulin molecule has a carboxyaroyl group.

GB Patent No. 1.492.997 (Nat. Res. Dev. Corp.) discloses insulin compounds with a carbamyl substitution at N^{εB29} with an improved profile of hypoglycaemic effect.

JP laid-open patent application No. 1-254699 (Kodama Co., Ltd.) discloses insulin compounds, wherein an alkanoyl group is bound to the amino group of Phe^{B1} or to the ε-amino group of Lys^{B29} or to both of these..

JP laid-open patent application No. 57-067548 (Shionogi) discloses insulin compounds, in which the B30 position have an amino acid having at least five carbon atoms which cannot necessarily be coded for by a triplet of nucleotides.

WO 03/053339 (Eli Lilly) disclose insulin compounds, wherein the A-chain in the N-terminal has been extended with two amino acid residues, A-1 and A0, wherein the B-chain has been extended at the N-terminal with two amino acid residues, B-1 and B0, wherein the amino acid residues at positions B28, B29 and B39 may be substituted, and wherein the ε-amino group of Lys at position B28 or B29 is covalently bound to the α-carboxyl group of a positively charged amino acid to form a Lys-Nε-amino acid derivative. Particular mentioning is made of said analogues, wherein A-1 and B-1 are both absent, and wherein A0 represent Arg and B0 represents Arg or is absent.

Insulin compounds selected from the group consisting of
i. An analogue wherein position B28 is Asp, Lys, Leu, Val, or Ala and position B29 is Lys or Pro; and
ii. des(B28-B30), des(B27) or des(B30) human insulin.
are also applicable for the methods of the present invention, and in particular, the insulin compound wherein position B28 is Asp or Lys, and position B29 is Lys or Pro. des(B30) human insulin is also applicable in the methods of the present invention.

Other applicable insulin compounds are selected from the group consisting of B29-N^{ε}-myristoyl-des(B30) human insulin, B29-N^{ε}-palmitoyl-des(B30) human insulin, B29-N^{ε}-myristoyl human insulin, B29-N^{ε}-palmitoyl human insulin, B28-N^{ε}-myristoyl Lys^{B28} Pro^{B29} human insulin, B28-N^{ε}-paimitoyl Lys^{B28} Pro^{B29} human insulin, B30-N^{ε}-myristoyl-Thr^{B29}Lys^{B30} human insulin, B30-N^{ε}-palmitoyl-Thr⁶²⁹Lys^{B30} human insulin, B29-N^{ε}-(N-palmitoyl-y-glutamyl)-des(B30) human insulin, B29-N^{ε}-(N-lithocholyl-y-glutamyl)-des(B30) human insulin, B29-N^{ε}-(ω-carboxyheptadecanoyl)-des(B30) human insulin, B29-N^{ε}-(ω-carboxyheptadecanoyl) human insulin and B29-N^{ε}-myristoyl-des(B30) human insulin.

Examples of GLP-1 applicable in the methods of the present invention include human GLP-1 and GLP-1 compounds. Human GLP-1 is a 37 amino acid residue peptide originating from preproglucagon which is synthesised *i.a*. in the L-cells in the distal ileum, in the pancreas and in the brain. GLP-1 is an important gut hormone with regulatory function in glucose metabolism and gastrointestinal secretion and metabolism. Processing of preproglucagon to give GLP-1 (7-36)-amide, GLP-1 (7-37) and GLP-2 occurs mainly in the L-cells. The fragments GLP-1 (7-36)-amide and GLP-1 (7-37) are both glucose-dependent insulinotropic agents. In the past decades a number of structural analogues of GLP-1 were isolated from the venom of the Gila monster lizards (*Heloderma suspectum* and *Heloderma horridum*)*.* Exendin-4 is a 39 amino acid residue peptide isolated from the venom of Heloderma horridum, and this peptide shares 52% homology with GLP-1. Exendin-4 is a potent GLP-1 receptor agonist which has been shown to stimulate insulin release and ensuring lowering of the blood glucose level when injected into dogs. The group of GLP-1 (1-37) and exendin-4(1-39) and certain fragments, analogues and derivatives thereof (designated GLP-1 compounds herein) are potent insulinotropic agents, and they are all applicable in the method of the present invention. Insulinotropic fragments of GLP-1(1-37) are insulinotropic peptides for which the entire sequence can be found in the sequence of GLP-1(1-37) and where at least one terminal amino acid has been deleted. Examples of insulinotropic fragments of GLP-1 (1-37) are GLP-1 (7-37) wherein the amino acid residues in positions 1-6 of GLP-1(1-37) have been deleted, and GLP-1 (7-36) where the amino acid residues in position 1-6 and 37 of GLP-1(1-37) have been deleted. Examples of insulinotropic fragments of exendin-4(1-39) are exendin-4(1-38) and exendin-4(1-31). The insulinotropic property of a compound may be determined by in vivo or in vitro assays well known in the art. For instance, the compound may be administered to an animal and monitoring the insulin concentration over time. Insulinotropic analogs of GLP-1(1-37) and exendin-4(1-39) refer to the respective molecules wherein one or more of the amino acids residues have been exchanged with other amino acid residues and/or from which one or more amino acid residues have been deleted and/or from which one or more amino acid residues have been added with the proviso that said analogue either is insulinotropic or is a prodrug of an insulinotropic compound. Examples of insulinotropic analogs of GLP-1 (1-37) is e.g. Met⁸ -GLP-1 (7-37) wherein the alanine in position 8 has been replaced by methionine and the amino acid residues in position 1 to 6 have been deleted, and Arg³⁴-GLP-1(7-37) wherein the valine in position 34 has been replaced with arginine and the amino acid residues in position 1 to 6 have been deleted. An example of an insulinotropic analog of exendin-4(1-39) is Ser²Asp³-exendin-4(1-39) wherein the amino acid residues in position 2 and 3 have been replaced with serine and aspartic acid, respectively (this particular analog also being known in the art as exendin-3). Insulinotropic derivatives of GLP-1 (1-37), exendin-4(1-39) and analogs thereof are what the person skilled in the art considers to be derivatives of these peptides, i.e. having at least one substituent which is not present in the parent peptide molecule with the proviso that said derivative either is insulinotropic or is a prodrug of an insulinotropic compound. Examples of substituents are amides, carbohydrates, alkyl groups and lipophilic substituents. Examples of insulinotropic derivatives of GLP-1 (1-37), exendin-4(1-39) and analogs thereof are GLP-1 (7-36)-amide, Arg³⁴, Lys²⁶(N^{ε}-(γ-Glu(N^{α}-hexadecanoyl)))-GLP-1(7-37) and Tyr³¹-exendin-4(1-31)-amide. Further examples of GLP-1 (1-37), exendin-4(1-39), insulinotropic fragments thereof, insulinotropic analogs thereof and insulinotropic derivatives thereof are described in WO 98/08871, WO 99/43706, US 5424286 and WO 00/09666.

GLP-2 and GLP-2 compounds may also be modified by the methods provided by the present invention. In the present context a GLP-2 compound binds to a GLP-2 receptor, preferably with an affinity constant (K_{D}) or a potency (EC₅₀) of below 1 µM, e.g. below 100 nM. The term "GLP-2 compound" is intended to indicate human GLP-2 in which one or more amino acid residue has been deleted and/or replaced by another amino acid residue, natural or unnatural, and/or human GLP-2 comprising additional amino acid residues, and/or human GLP-2 in which at least one organic substituent is bound to one or more of the amino acid residues. In particular, those peptides are considered, which amino acid sequence exhibit at any sequence of 33 consecutive amino acids more than 60% of the amino acid sequence of human GLP-2. Also those peptides are considered, which amino acid sequence exhibit at any sequence of 37 consecutive amino acids more than 60% of the amino acid sequence of human GLP-2, when up to four amino acids are deleted from the amino acid sequence. Also those peptides are considered, which amino acid sequence exhibit at any sequence of 31 consecutive amino acids more than 60% of the amino acid sequence of GLP-2, when up to two amino acids are added to their amino acid sequence. The term "GLP compounds" also includes natural allelic variations that may exist and occur from one individual to another. Also, degree and location of glycosylation or other post-translation modifications may vary depending on the chosen host cells and the nature of the host cellular environment.

Candidate GLP-2 compounds, which may be used according to the present invention include the GLP-2 compounds described in WO 96/32414, WO 97/39031, WO 98/03547, WO 96/29342, WO 97/31943 and WO 98/08872.

In particular, the following GLP-2 compounds are applicable in the methods of the present invention. A2G-GLP-2(1-33); K30R-GLP-2(1-33); S5K-GLP-2(1-33); S7K-GLP-2(1-33); D8K-GLP-2(1-33); E9K-GLP-2(1-33); M10K-GLP-2(1-33); N11 K-GLP-2(1-33); T12K-GLP-2(1-33); I13K-GLP-2(1-33); L14K-GLP-2(1-33); D15K-GLP-2(1-33); N16K-GLP-2(1-33); L17K-GLP-2(1-33); A18K-GLP-2(1-33); D21 K-GLP-2(1-33); N24K-GLP-2(1-33); Q28K-GLP-2(1-33); S5K/K30R-GLP-2(1-33); S7K/K30R-GLP-2(1-33); D8K/K30R-GLP-2(1-33); E9K/K30R-GLP-2(1-33); M10K/K30R-GLP-2(1-33); N11K/K30R-GLP-2(1-33); T12K/K30R-GLP-2(1-33); I13K/K30R-GLP-2(1-33); L14K/K30R-GLP-2(1-33); D15K/K30R-GLP-2(1-33); N16K/K30R-GLP-2(1-33); L17K/K30R-GLP-2(1-33); A18K/K30R-GLP-2(1-33); D21K/K30R-GLP-2(1-33); N24K/K30R-GLP-2(1-33); Q28K/K30R-GLP-2(1-33); K30R/D33K-GLP-2(1-33); D3E/K30R/D33E-GLP-2(1-33); D3E/S5K/K30R/D33E-GLP-2(1-33); D3E/S7K/K30R/D33E-GLP-2(1-33); D3E/D8K/K30R/D33E-GLP-2(1-33); D3E/E9K/K30R/D33E-GLP-2(1-33); D3E/M10K/K30R/D33E-GLP-2(1-33); D3E/N11 K/K30R/D33E-GLP-2(1-33); D3E/T12K/K30R/D33E-GLP-2(1-33); D3E/113K/K30R/D33E-GLP-2(1-33); D3E/L14K/K30R/D33E-GLP-2(1-33); D3E/D15K/K30R/D33E-GLP-2(1-33); D3E/N16K/K30R/D33E-GLP-2(1-33); D3E/L17K/K30R/D33E-GLP-2(1-33); D3E/A18K/K30R/D33E-GLP-2(1-33); D3E/D21 K/K30R/D33E-GLP-2(1-33); D3E/N24K/K30R/D33E-GLP-2(1-33); and D3E/Q28K/K30R/D33E-GLP-2(1-33).

GLP-2 derivatives with only one lipophilic substituent attached to the GLP-2 peptide are also applicable in the methods of the present invention, such as GLP-2 derivatives wherein the lipophilic substituent comprises from 4 to 40 carbon atoms, such as from 8 to 25 carbon atoms, e.g. from 12 to 20 carbon atoms.

The lipophilic substituent may be attached to an amino acid residue in such a way that a carboxyl group of the lipophilic substituent forms an amide bond with an amino group of the amino acid residue.

By way of example, the lipophilic substituent is attached to a Lys residue.

The lipophilic substituent may be attached to an amino acid residue in such a way that an amino group of the lipophilic substituent forms an amide bond with a carboxyl group of the amino acid residue.

The lipophilic substituent may also be attached to the GLP-2 peptide by means of a spacer, and said spacer may be selected from amongst β-alanine, gamma-aminobutyric acid (GABA), γ-glutamic acid, Lys, Asp, Glu, a dipeptide containing Asp, a dipeptide containing Glu, or a dipeptide containing Lys. In one embodiment of the invention the spacer is β-alanine. A carboxyl group of the parent GLP-2 peptide may also form an amide bond with an amino group of a spacer, and the carboxyl group of the amino acid or dipeptide spacer forms an amide bond with an amino group of the lipophilic substituent.

An amino group of the parent GLP-2 peptide may also form an amide bond with a carboxylic group of a spacer, and an amino group of the spacer forms an amide bond with a carboxyl group of the lipophilic substituent.

In one embodiment of the invention the lipophilic substituent is a straight-chain or branched alkyl group. In one embodiment of the invention the lipophilic substituent is the acyl group of a straight-chain or branched fatty acid.

In one embodiment of the invention the lipophilic substituent is an acyl group of a straight-chain or branched alkane α,ω-dicarboxylic acid.

In one embodiment of the invention the GLP-2 derivative has one lipophilic substituent. In one embodiment of the invention the GLP-2 derivative has two lipophilic substituents. In one embodiment of the invention the GLP-2 derivative has three lipophilic substituents. In one embodiment of the invention the GLP-2 derivative has four lipophilic substituents.

The following list contains GLP-2 derivatives which are particular applicable in the methods of the present invention.
S5K(3-(hexadecanoylamino)propionyl)-GLP-2(1-33);
S7K(3-(hexadecanoylamino)propionyl)-GLP-2(1-33);
D8K(3-(hexadecanoylamino)propionylyGLP-2(1-33);
E9K(3-(hexadecanoylamino)propionyl)-GLP-2(1-33);
M10K(3-(hexadecanoylamino)propionyl)-GLP-2(1-33);
N11K(3-(hexadecanoylamino)propionyl)-GLP-2(1-33);
T12K(3-(hexadecanoylamino)propionyl)-GLP-2(1-33);
I13K(3-(hexadecanoylamino)propionyl)-GLP-2(1-33);
L14K(3-(hexadecanoylamino)propionyl)-GLP-2(1-33);
D15K(3-(hexadecanoylamino)propionyl)-GLP-2(1-33);
N 16K(3-(hexadecanoylamino)propionyl)-GLP-2(1-33);
L17K(3-(octanoylamino)propionyl)-GLP-2(1-33);
L17K(3-(nonanoylamino)propionyl)-GLP-2(1-33);
L17K(3-(decanoylamino)propionyl)-GLP-2(1-33);
L17K(3-(undecanoylamino)propionyl)-GLP-2(1-33);
L17K(3-(dodecanoylamino)propionyl)-GLP-2(1-33);
L17K(3-(tridecanoylamino)propionyl)-GLP-2(1-33);
L17K(3-(tetradecanoylamino)propionyl)-GLP-2(1-33);
L17K(3-(pentadecanoylamino)propionyl)-GLP-2(1-33);
L17K(3-(hexadecanoylamino)propionyl)-GLP-2(1-33);
L17K(3-(heptadecanoylamino)propionyl)-GLP-2(1-33);
L17K(3-(octadecanoylamino)propionyl)-GLP-2(1-33);

L17K(3-(nonadecanoylamino)propionyl)-GLP-2(1-33);
L17K(3-(eicosanoylamino)propionyl)GLP-2(1-33);
L17K((S)-4-carboxy-4-(octanoylamino)butanoyl)-GLP-2(1-33);
L17K((S)-4-carboxy-4-(nonanoylamino)butanoyl)-GLP-2(1-33);
L17K((S)-4-carboxy-4-(decanoylamino)butanoyl)-GLP-2(1-33);
L17K((S)-4-carboxy-4-(undecanoylamino)butanoyl)-GLP-2(1-33);
L17K((S)-4-carboxy-4-(dodecanoylamino)butanoyl)-GLP-2(1-33);
L17K((S)-4-carboxy-4-(tridecanoylamino)butanoyl)-GLP-2(1-33);
L17K((S)-4-carboxy-4-(tetradecanoylamino)butanoyl)-GLP-2(1-33);
L17K((S)-4-carboxy-4-(pentadecanoylamino)butanoyl)-GLP-2(1-33);
L17K((S)-4-carboxy-4-(hexadecanoylamino)butanoyl)-GLP-2(1-33);
L17K((S)-4-carboxy-4-(heptadecanoylamino)butanoyl)-GLP-2(1-33);
L17K((S)-4-carboxy-4-(octadecanoylamino)butanoyl)-GLP-2(1-33);
L17K((S)-4-carboxy-4-(nonadecanoylamino)butanoyl)-GLP-2(1-33);
L17K((SF4-carboxy-4-(eicosanoylamino)butanoyl)-GLP-2(1-33);
L17K(4-(octanoylamino)butanoyl)-GLP-2(1-33);
L17K(4-(nonanoylamino)butanoyl)-GLP-2(1-33);
L17K(4-(decanoylamino)butanoyl)-GLP-2(1-33);
L17K(4-(undecanoylamino)butanoyl)-GLP-2(1-33);
L17K(4-(dodecanoylamino)butanoyl)-GLP-2(1-33);
L17K(4-(tridecanoylamino)butanoyl)-GLP-2(1-33);
L17K(4-(tetradecanoylamino)butanoyl)-GLP-2(1-33);
L17K(4-(pentadecanoylamino)butanoyl)-GLP-2(1-33);
L17K(4-(hexadecanoylamino)butanoyl)-GLP-2(1-33);
L17K(4-(heptadecanoylamino)butanoyl)-GLP-2(1-33);
L17K(4-(octadecanoylamino)butanoyl)-GLP-2(1-33);
L17K(4-(nonadecanoylamino)butanoyl)-GLP-2(1-33);
L17K(4-(eicosanoylamino)butanoyl)-GLP-2(1-33);
A18K(3-(hexadecanoylamino)propionyl)-GLP-2(1-33);
D21K(3-(hexadecanoylamino)propionyl)-GLP-2(1-33);
N24K(3-(hexadecanoylamino)propionyl)-GLP-2(1-33);
Q28K(3-(hexadecanoylamino)propionyl)-GLP-2(1-33);
S5K(3-(hexadecanoylamino)propionyl)/K30R-GLP-2(1-33);
S7K(3-(hexadecanoylamino)propionyl)/K30R-GLP-2(1-33);
D8K(3-(hexadecanoylamino)propionyl)/K30R-GLP-2(1-33);
E9K(3-(hexadecanoylamino)propionyl)/K30R-GLP-2(1-33);
M10K(3-(hexadecanoylamino)propionyl)/K30R-GLP-2(1-33);
N11K(3-(hexadecanoylamino)propionyl)/K30R-GLP-2(1-33);
T12K(3-(hexadecanoylamino)propionyl)/K30R-GLP-2(1-33);
I13K(3-(hexadecanoylamino)propionyl)/K30R-GLP-2(1-33);
L14K(3-(hexadecanoylamino)propionyl)/K30R-GLP-2(1-33);
D15K(3-(hexadecanoylamino)propionyl)/K30R-GLP-2(1-33);
N 16K(3-(hexadecanoylamino)propionyl)/K30R-GLP-2(1-33);
L17K(3-(octanoylamino)propionyl)/K30R-GLP-2(1-33);
L17K(3-(nonanoylamino)propionyl)/K30R-GLP-2(1-33);
L17K(3-(decanoylamino)propionyl)/K30R-GLP-2(1-33);
L17K(3-(undecanoylamino)propionyl)/K30R-GLP-2(1-33);
L17K(3-(dodecanoylamino)propionyl)/K30R-GLP-2(1-33);
L17K(3-(tridecanoylamino)propionyl)/K30R-GLP-2(1-33);
L17K(3-(tetradecanoylamino)propionyl)/K30R-GLP-2(1-33);
L17K(3-(pentadecanoylamino)propionyl)/K30R-GLP-2(1-33);
L17K(3-(hexadecanoylamino)propionyl)/K30R-GLP-2(1-33);
L17K(3-(heptadecanoylamino)propionyl)/K30R-GLP-2(1-33);
L17K(3-(octadecanoylamino)propionyl)/K30R-GLP-2(1-33);
L17K(3-(nonadecanoylamino)propionyl)/K30R-GLP-2(1-33);
L17K(3-(eicosanoylamino)propionyl)/K30R-GLP-2(1-33);
L17K((S)-4-carboxy-4-(octanoylamino)butanoyl)/K30R-GLP-2(1-33);
L17K((S)-4-carboxy-4-(nonanoylamino)butanoyl)/K30R-GLP-2(1-33);
L17K((S)-4-carboxy-4-(decanoylamino)butanoyl)/K30R-GLP-2(1-33);
L17K((S)-4-carboxy-4-(undecanoylamino)butanoyl)/K30R-GLP-2(1-33);
L17K((S)-4-carboxy-4-(dodecanoylamino)butanoyl)/K30R-GLP-2(1-33);
L17K((S)-4-carboxy-4-(tridecanoylamino)butanoyl)/K30R-GLP-2(1-33);
L17K((S)-4-carboxy-4-(tetradecanoylamino)butanoyl)/K30R-GLP-2(1-33);
L17K((S)-4-carboxy-4-(pentadecanoylamino)butanoyl)/K30R-GLP-2(1-33);
L17K((S)-4-carboxy-4-(hexadecanoylamino)butanoyl)1K30R-GLP-2(1-33);
L17K((S)-4-carboxy-4-(heptadecanoylamino)butanoyl)/K30R-GLP-2(1-33);
L17K((S)-4-carboxy-4-(octadecanoylamino)butanoyl)/K30R-GLP-2(1-33);
L17K((S)-4-carboxy-4-(nonadecanoylamino)butanoyl)/K30R-GLP-2(1-33);
L17K((S)-4-carboxy-4-(eicosanoylamino)butanoyl)/K30R-GLP-2(1-33);
L17K(4-(octanoylamino)butanoyl)/K30R-GLP-2(1-33);
L17K(4-(nonanoylamino)butanoyl)/K30R-GLP-2(1-33);
L17K(4-(decanoylamino)butanoyl)/K30R-GLP-2(1-33);
L17K(4-(undecanoylamino)butanoyl)/K30R-GLP-2(1-33);
L17K(4-(dodecanoylamino)butanoyl)/K30R-GLP-2(1-33);
L17K(4-(tridecanoylamino)butanoyl)/K30R-GLP-2(1-33);
L17K(4-(tetradecanoylamino)butanoyl)/K30R-GLP-2(1-33);
L17K(4-(pentadecanoylamino)butanoyl)/K30R-GLP-2(1-33);
L17K(4-(hexadecanoylamino)butanoyl)/K30R-GLP-2(1-33);
L17K(4-(heptadecanoylamino)butanoyl)/K30R-GLP-2(1-33);
L17K(4-(octadecanoylamino)butanoyl)/K30R-GLP-2(1-33);
L17K(4-(nonadecanoylamino)butanoyl)/K30R-GLP-2(1-33);
L17K(4-(eicosanoylamino)butanoyl)/K30R-GLP-2(1-33);
A18K(3-(hexadecanoylamino)propionyl)/K30R-GLP-2(1-33);
D21 K(3-(hexadecanoylamino)propionyl)/K30R-GLP-2(1-33);
N24K(3-(hexadecanoylamino)propionyl)/K30R-GLP-2(1-33);
Q28K(3-(hexadecanoylamino)propionyl)/K30R-GLP-2(1-33);
D3E/S5K(3-(hexadecanoylamino)propionyl)/K30R/D33E-GLP-2(1-33);
D3E/S7K(3-(hexadecanoylamino)propionyl)/K30R/D33E-GLP-2(1-33);
D3E/D8K(3-(hexadecanoylamino)propionyl)/K30R/D33E-GLP-2(1-33);
D3E/E9K(3-(hexadecanoylamino)propionyl)/K30R/D33E-GLP-2(1-33);
D3E/M10K(3-(hexadecanoylamino)propionyl)/K30R/D33E-GLP-2(1-33);
D3E/N11K(3-(hexadecanoylamino)propionyl)/K30R/D33E-GLP-2(1-33);
D3E/T12K(3-(hexadecanoylamino)propionyl)/K30R/D33E-GLP-2(1-33);
D3E/113K(3-(hexadecanoylamino)propionyl)/K30R/D33E-GLP-2(1-33);
D3E/L14K(3-(hexadecanoylamino)propionyl)/K30R/D33E-GLP-2(1-33);
D3E/D15K(3-(hexadecanoylamino)propionyl)/K30R/D33E-GLP-2(1-33);
D3E/N16K(3-(hexadecanoylamino)propionyl)/K30R/D33E-GLP-2(1-33);
D3E/L17K(3-(octanoylamino)propionyl)/K30R/D33E-GLP-2(1-33);
D3E/L17K(3-(nonanoylamino)propionyl)/K30R/D33E-GLP-2(1-33);
D3E/L17K(3-(decanoylamino)propionyl)/K30R/D33E-GLP-2(1-33);
D3E/L17K(3-(undecanoylamino)propionyl)/K30R/D33E-GLP-2(1-33);
D3E/L17K(3-(dodecanoylamino)propionyl)/K30R/D33E-GLP-2(1-33);
D3E/L17K(3-(tridecanoylamino)propionyl)/K30R/D33E-GLP-2(1-33);
D3E/L17K(3-(tetradecanoylamino)propionyl)/K30R/D33E-GLP-2(1-33);
D3E/L17K(3-(pentadecanoylamino)propionyl)/K30R/D33E-GLP-2(1-33);
D3E/L17K(3-(hexadecanoylamino)propionyl)/K30R/D33E-GLP-2(1-33);
D3E/L17K(3-(heptadecanoylamino)propionyl)/K30R/D33E-GLP-2(1-33);
D3E/L17K(3-(octadecanoylamino)propionyl)/K30R/D33E-GLP-2(1-33);
D3E/L17K(3-(nonadecanoylamino)propionyl)/K30R/D33E-GLP-2(1-33);
D3E/L17K(3-(eicosanoylamino)propionyl)/K30R/D33E-GLP-2(1-33);
D3E/L17K((S)-4-carboxy-4-(octanoylamino)butanoyl)/K30R/D33E-GLP-2(1-33);
D3E/L17K((S)-4-carboxy-4-(nonanoylamino)butanoyl)/K30R/D33E-GLP-2(1-33);
D3E/L17K((S)-4-carboxy-4-(decanoylamino)butanoyl)/K30R/D33E-GLP-2(1-33);
D3E/L17K((S)-4-carboxy-4-(undecanoylamino)butanoyl)/K30R/D33E-GLP-2(1-33);
D3E/L17K((S)-4-carboxy-4-(dodecanoylamino)butanoyl)/K30R/D33E-GLP-2(1-33);
D3E/L17K((S)-4-carboxy-4-(tridecanoylamino)butanoyl)/K30R1D33E-GLP-2(1-33);
D3E/L17K((S)-4-carboxy-4-(tetradecanoylamino)butanoyl)/K30R/D33E-GLP-2(1-33);
D3E/L17K((S)-4-carboxy-4-(pentadecanoylamino)butanoyl)/K30R/D33E-GLP-2(1-33);
D3E/L17K((S)-4-carboxy-4-(hexadecanoylamino)butanoyl)/K30R/D33E-GLP-2(1-33);
D3E/L17K((S)-4-carboxy-4-(heptadecanoylamino)butanoyl)/K30RID33E-GLP-2(1-33);
D3E/L17K((S)-4-carboxy-4-(octadecanoylamino)butanoyl)/K30R/D33E-GLP-2(1-33);
D3E/L17K((S)-4-carboxy-4-(nonadecanoylamino)butanoyl)/K30R/D33E-GLP-2(1-33);
D3E/L17K((S)-4-carboxy-4-(eicosanoylamino)butanoyl)/K30R/D33E-GLP-2(1-33);
D3E/L17K(4-(octanoylamino)butanoyl)/K30R/D33E-GLP-2(1-33);
D3E/L17K(4-(nonanoylamino)butanoyl)/K30R/D33E-GLP-2(1-33);
D3E/L17K(4-(decanoylamino)butanoyl)/K30R/D33E-GLP-2(1-33);
D3E/L17K(4-(undecanoylamino)butanoyl)/K30R/D33E-GLP-2(1-33);
D3E/L17K(4-(dodecanoylamino)butanoyl)/K30R/D33E-GLP-2(1-33);
D3E/L17K(4-(tridecanoylamino)butanoyl)/K30R/D33E-GLP-2(1-33);
D3E/L17K(4-(tetradecanoylamino)butanoyl)/K30R/D33E-GLP-2(1-33);
D3E/L17K(4-(pentadecanoylamino)butanoyl)/K30R/D33E-GLP-2(1-33);
D3E/L17K(4-(hexadecanoylamino)butanoyl)/K30R/D33E-GLP-2(1-33);
D3E/L17K(4-(heptadecanoylamino)butanoyl)/K30R/D33E-GLP-2(1-33);
D3E/L17K(4-(octadecanoylamino)butanoyl)/K30R/D33E-GLP-2(1-33);
D3E/L17K(4-(nonadecanoylamino)butanoyl)/K30R1D33E-GLP-2(1-33);
D3E/L17K(4-(eicosanoylamino)butanoyl)/K30R/D33E-GLP-2(1-33);
D3E/A18K(3-(hexadecanoylamino)propionyl)/K30R/D33E-GLP-2(1-33);
D3E/D21 K(3-(hexadecanoylamino)propionyl)/K30R/D33E-GLP-2(1-33);
D3E/N24K(3-(hexadecanoylamino)propionyl)/K30R/D33E-GLP-2(1-33); and
D3E/Q28K(3-(hexadecanoylamino)propionyl)/K30R/D33E-GLP-2(1-33).

Factor VII compounds applicable in the methods of the present invention encompasses wild-type Factor VII (i.e., a polypeptide having the amino acid sequence disclosed in U.S. Patent No. 4,784,950), as well as variants of Factor VII exhibiting substantially the same or improved biological activity relative to wild-type Factor VII, Factor VII-related polypeptides as well as Factor VII derivatives and Factor VII conjugates. The term "Factor VII compounds" is intended to encompass Factor VII polypeptides in their uncleaved (zymogen) form, as well as those that have been proteolytically processed to yield their respective bioactive forms, which may be designated Factor VIIa. Typically, Factor VII is cleaved between residues 152 and 153 to yield Factor VIIa. Such variants of Factor VII may exhibit different properties relative to human Factor VII, including stability, phospholipid binding, altered specific activity, and the like.

As used herein, "Factor VII-related polypeptides" encompasses polypeptides, including variants, in which the Factor VIIa biological activity has been substantially modified or reduced relative to the activity of wild-type Factor VIIa. These polypeptides include, without limitation, Factor VII or Factor VIIa into which specific amino acid sequence alterations have been introduced that modify or disrupt the bioactivity of the polypeptide.

The term "Factor VII derivative" as used herein, is intended to designate wild-type Factor VII, variants of Factor VII exhibiting substantially the same or improved biological activity relative to wild-type Factor VII and Factor VII-related polypeptides, in which one or more of the amino acids of the parent peptide have been chemically modified, e.g. by alkylation, PEGylation, acylation, ester formation or amide formation or the like. This includes but are not limited to PEGylated human Factor VIIa, cysteine-PEGylated human Factor VIIa and variants thereof.

The term "PEGylated human Factor VIIa" means human Factor VIIa, having a PEG mole-cule conjugated to a human Factor VIIa polypeptide. It is to be understood, that the PEG molecule may be attached to any part of the Factor VIIa polypeptide including any amino acid residue or carbohydrate moiety of the Factor VIIa polypeptide. The term "cysteine-PEGylated human Factor VIIa" means Factor VIIa having a PEG molecule conjugated to a sulfhydryl group of a cysteine introduced in human Factor VIIa.

The biological activity of Factor VIIa in blood clotting derives from its ability to (i) bind to tissue factor (TF) and (ii) catalyze the proteolytic cleavage of Factor IX or Factor X to produce activated Factor IX or X (Factor IXa or Xa, respectively). For purposes of the invention, Factor VIIa biological activity may be quantified by measuring the ability of a preparation to promote blood clotting using Factor VII-deficient plasma and thromboplastin, as described, e.g., in U.S. Patent No. 5,997,864. In this assay, biological activity is expressed as the reduction in clotting time relative to a control sample and is converted to "Factor VII units" by comparison with a pooled human serum standard containing 1 unit/ml Factor VII activity. Alternatively, Factor VIIa biological activity may be quantified by (i) measuring the ability of Factor VIIa to produce of Factor Xa in a system comprising TF embedded in a lipid membrane and Factor X. (Persson et al., J. Biol. Chem. 272:19919-19924, 1997); (ii) measuring Factor X hydrolysis in an aqueous system; (iii) measuring its physical binding to TF using an instrument based on surface plasmon resonance (Persson, FEBS Letts. 413:359-363, 1997) and (iv) measuring hydrolysis of a synthetic substrate.

Factor VII variants having substantially the same or improved biological activity relative to wild-type Factor VIIa encompass those that exhibit at least about 25%, preferably at least about 50%, more preferably at least about 75% and most preferably at least about 90% of the specific activity of Factor VIIa that has been produced in the same cell type, when tested in one or more of a clotting assay, proteolysis assay, or TF binding assay as described above. Factor VII variants having substantially reduced biological activity relative to wild-type Factor VIIa are those that exhibit less than about 25%, preferably less than about 10%, more preferably less than about 5% and most preferably less than about 1% of the specific activity of wild-type Factor VIIa that has been produced in the same cell type when tested in one or more of a clotting assay, proteolysis assay, or TF binding assay as described above. Factor VII variants having a substantially modified biological activity relative to wild-type Factor VII include, without limitation, Factor VII variants that exhibit TF-independent Factor X proteolytic activity and those that bind TF but do not cleave Factor X.

Variants of Factor VII, whether exhibiting substantially the same or better bioactivity than wild-type Factor VII, or, alternatively, exhibiting substantially modified or reduced bioactivity relative to wild-type Factor VII, include, without limitation, polypeptides having an amino acid sequence that differs from the sequence of wild-type Factor VII by insertion, deletion, or substitution of one or more amino acids.

The terms "variant" or "variants", as used herein, is intended to designate Factor VII having the sequence of wild-type factor VII, wherein one or more amino acids of the parent protein have been substituted by another amino acid and/or wherein one or more amino acids of the parent protein have been deleted and/or wherein one or more amino acids have been inserted in protein and/or wherein one or more amino acids have been added to the parent protein. Such addition can take place either at the N-terminal end or at the C-terminal end of the parent protein or both. The "variant" or "variants" within this definition still have FVII activity in its activated form. In one embodiment a variant is 70 % identical with the sequence of wild-type Factor VII. In one embodiment a variant is 80 % identical with the sequence of wild-type factor VII. In another embodiment a variant is 90 % identical with the sequence of wild-type factor VII. In a further embodiment a variant is 95 % identical with the sequence of wild-type factor VII.

Non-limiting examples of Factor VII variants having substantially the same biological activity as wild-type Factor VII include S52A-FVIIa, S60A-FVIIa ( Lino et al., Arch. Biochem. Biophys. 352: 182-192, 1998); FVIIa variants exhibiting increased proteolytic stability as disclosed in U.S. Patent No. 5,580,560; Factor VIIa that has been proteolytically cleaved between residues 290 and 291 or between residues 315 and 316 (Mollerup et al., Biotechnol. Bioeng. 48:501-505, 1995); oxidized forms of Factor VIIa (Kornfelt et al., Arch. Biochem. Biophys. 363:43-54, 1999); FVII variants as disclosed in PCT/DK02/00189; and FVII variants exhibiting increased proteolytic stability as disclosed in WO 02/38162 (Scripps Research Institute); FVII variants having a modified Gla-domain and exhibiting an enhanced membrane binding as disclosed in WO 99/20767 (University of Minnesota); and FVII variants as disclosed in WO 01/58935 (Maxygen ApS).

Particular mentioning is made of FVII variants having increased biological activity compared to wild-type FVIIa include FVII variants as disclosed in WO 01/83725, WO 02/22776, WO 02/077218, PCT/DK02/00635, Danish patent application PA 2002 01423, Danish patent application PA 2001 01627; WO 02/38162 (Scripps Research Institute); and FVIIa variants with enhanced activity as disclosed in JP 2001061479 (Chemo-Sero-Therapeutic Res Inst.).

Examples of Factor VII variants having substantially reduced or modified biological activity relative to wild-type Factor VII include R152E-FVIIa (Wildgoose et al., Biochem 29:3413-3420, 1990), S344A-FVIIa (Kazama et al., J. Biol. Chem. 270:66-72, 1995), FFR-FVIIa (Holst et al., Eur. J. Vasc. Endovasc. Surg. 15:515-520, 1998), and Factor VIIa lacking the Gla domain, (Nicolaisen et al., FEBS Letts. 317:245-249, 1993).

Examples of variants of factor VII, factor VII or factor VII-related polypeptides include wild-type Factor VII, L305V-FVII, L305V/M306D/D309S-FVII, L305I-FVII, L305T-FVII, F374P-FVII, V158T/M298Q-FVII, V158D/E296V/M298Q-FVII, K337A-FVII, M298Q-FVII, V158D/M298Q-FVII, L305V/K337A-FVII, V158D/E296V/M298Q/L305V-FVII, V158D/E296V/M298Q/K337A-FVII, V158D/E296V/M298Q/L305V/K337A-FVII, K157A-FVII, E296V-FVII, E296V/M298Q-FVII, V158D/E296V-FVII, V158D/M298K-FVII, and S336G-FVII, L305V/K337A-FVII, L305V/V158D-FVII, L305V/E296V-FVII, L305V/M298Q-FVII, L305V/V158T-FVII, L305V/K337A/V158T-FVII, L305V/K337A/M298Q-FVII, L305V/K337A/E296V-FVII, L305V/K337A/V158D-FVII, L305V/V158D/M298Q-FVII, L305V/V158D/E296V-FVII, L305V/V158T/M298Q-FVII, L305V/V158T/E296V-FVII, L305V/E296V/M298Q-FVII, L305V/V158D/E296V/M298Q-FVII, L305V/V158T/E296V/M298Q-FVII, L305V/V158T/K337A/M298Q-FVII, L305V/V158T/E296V/K337A-FVII, L305V/V158D/K337A/M298Q-FVII, L305V/V158D/E296V/K337A-FVII, L305V/V158D/E296V/M298Q/K337A-FVII, L305V/V158T/E296V/M298Q/K337A-FVII, S314E/K316H-FVII, S314E/K316Q-FVII, S314E/L305V-FVII, S314E/K337A-FVII, S314E/V158D-FVII, S314E/E296V-FVII, S314E/M298Q-FVII, S314E/V158T-FVII, K316H/L305V-FVII, K316H/K337A-FVII, K316H/V158D-FVII, K316H/E296V-FVII, K316H/M298Q-FVII, K316H/V158T-FVII, K316Q/L305V-FVII, K316Q/K337A-FVII, K316Q/V158D-FVII, K316Q/E296V-FVII, K316Q/M298Q-FVII, K316Q/V158T-FVII, S314E/L305V/K337A-FVII, S314E/L305VN158D-FVII, S314E/L305V/E296V-FVII, S314E/L305V/M298Q-FVII, S314E/L305V/V158T-FVII, S314E/L305V/K337A/V158T-FVII, S314E/L305V/K337A/M298Q-FVII, S314E/L305V/K337A/E296V-FVII, S314E/L305V/K337AN158D-FVII, S314E/L305VN158D/M298Q-FVII, S314E/L305VN158D/E296V-FVII, S314E/L305VN158T/M298Q-FVII, S314E/L305VN158T/E296V-FVII, S314E/L305V/E296V/M298Q-FVII, S314E/L305VN158D/E296V/M298Q-FVII, S314E/L305VN158T/E296V/M298Q-FVII, S314E/L305VN158T/K337A/M298Q-FVII, S314E/L305VN158T/E296V/K337A-FVII, S314E/L305VN158D/K337A/M298Q-FVII, S314E/L305VN158D/E296V/K337A-FVII, S314E/L305VN158D/E296V/M298Q/K337A-FVII, S314E/L305V/V158T/E296V/M298Q/K337A-FVII, K316H/L305V/K337A-FVII, K316H/L305VN158D-FVII, K316H/L305V/E296V-FVII, K316H/L305V/M298Q-FVII, K316H/L305V/V158T-FVII, K316H/L305V/K337A/V158T-FVII, K316H/L305V/K337A/M298Q-FVII, K316H/L305V/K337A/E296V-FVII, K316H/L305V/K337A/V158D-FVII, K316H/L305V/V158D/M298Q-FVII, K316H/L305V/V158D/E296V-FVII, K316H/L305V/V158T/M298Q-FVII, K316H/L305V/V158T/E296V-FVII, K316H/L305V/E296V/M298Q-FVII, K316H/L305VN158D/E296V/M298Q-FVII, K316H/L305V/V158T/E296V/M298Q-FVII, K316H/L305V/V158T/K337A/M298Q-FVII, K316H/L305V/V158T/E296V/K337A-FVII, K316H/L305V/V158D/K337A/M298Q-FVII, K316H/L305V/V158D/E296V/K337A -FVII, K316H/L305V/V158D/E296V/M298Q/K337A-FVII, K316H/L305V/V158T/E296V/M298Q/K337A-FVII, K316Q/L305V/K337A-FVII, K316Q/L305VN158D-FVII, K316Q/L305V/E296V-FVII, K316Q/L305V/M298Q-FVII, K316Q/L305V/V158T-FVII, K316Q/L305V/K337A/V158T-FVII, K316Q/L305V/K337A/M298Q-FVII, K316Q/L305V/K337A/E296V-FVII, K316Q/L305V/K337A/V158D-FVII, K316Q/L305V/V158D/M298Q-FVII, K316Q/L305V/V158D/E296V-FVII, K316Q/L305V/V158T/M298Q-FVII, K316Q/L305VN158T/E296V-FVII, K316Q/L305V/E296V/M298Q-FVII, K316Q/L305V/V158D/E296V/M298Q-FVII, K316Q/L305V/V158T/E296V/M298Q-FVII, K316Q/L305V/V158T/K337A/M298Q-FVII, K316Q/L305V/V158T/E296V/K337A-FVII, K316Q/L305V/V158D/K337A/M298Q-FVII, K316Q/L305V/V158D/E296V/K337A-FVII, K316Q/L305V/V158D/E296V/M298Q/K337A-FVII, K316Q/L305V/V158T/E296V/M298Q/K337A-FVII, F374Y/K337A-FVII, F374Y/V158D-FVII, F374Y/E296V-FVII, F374Y/M298Q-FVII, F374Y/V158T-FVII, F374Y/S314E-FVII, F374Y/L305V-FVII, F374Y/L305V/K337A-FVII, F374Y/L305V/V158D-FVII, F374Y/L305V/E296V-FVII, F374Y/L305V/M298Q-FVII, F374Y/L305V/V158T-FVII, F374Y/L305V/S314E-FVII, F374Y/K337A/S314E-FVII, F374Y/K337A/V158T-FVII, F374Y/K337A/M298Q-FVII, F374Y/K337A/E296V-FVII, F374Y/K337A/V158D-FVII, F374YN158D/S314E-FVII, F374YN158D/M298Q-FVII, F374YN158D/E296V-FVII, F374Y/V158T/S314E-FVII, F374Y/V158T/M298Q-FVII, F374Y/V158T/E296V-FVII, F374Y/E296V/S314E-FVII, F374Y/S314E/M298Q-FVII, F374Y/E296V/M298Q-FVII, F374Y/L305V/K337AN158D-FVII, F374Y/L305V/K337A/E296V-FVII, F374Y/L305V/K337A/M298Q-FVII, F374Y/L305V/K337A/V158T-FVII, F374Y/L305V/K337A/S314E-FVII, F374Y/L305VN158D/E296V-FVII, F374Y/L305V/B158D/M298Q-FVII, F374Y/L305V/V158D/S314E-FVII, F374Y/L305V/E296V/M298Q-FVII, F374Y/L305V/E296VN158T-FVII, F374Y/L305V/E296V/S314E-FVII, F374Y/L305V/M298Q/V158T-FVII, F374Y/L305V/M298Q/S314E-FVII, F374Y/L305V/V158T/S314E-FVII, F374Y/K337A/S314E/V158T-FVII, F374Y/K337A/S314E/M298Q-FVII, F374Y/K337A/S314E/E296V-FVII, F374Y/K337A/S314EN158D-FVII, F374Y/K337A/V158T/M298Q-FVII, F374Y/K337A/V158T/E296V-FVII, F374Y/K337A/M298Q/E296V-FVII, F374Y/K337A/M298Q/V158D-FVII, F374Y/K337A/E296V/V158D-FVII, F374Y/V158D/S314E/M298Q-FVII, F374Y/V158D/S314E/E296V-FVII, F374Y/V158D/M298Q/E296V-FVII, F374YN158T/S314E/E296V-FVII, F374Y/V158T/S314E/M298Q-FVII, F374Y/V158T/M298Q/E296V-FVII, F374Y/E296V/S314E/M298Q-FVII, F374Y/L305V/M298Q/K337A/S314E-FVII, F374Y/L305V/E296V/K337A/S314E-FVII, F374Y/E296V/M298Q/K337A/S314E-FVII, F374Y/L305V/E296V/M298Q/K337A-FVII, F374Y/L305V/E296VIM298Q/S314E-FVII, F374Y/V158D/E296V/M298Q/K337A-FVII, F374Y/V158D/E296V/M298Q/S314E-FVII, F374Y/L305V/V158D/K337A/S314E-FVII, F374Y/V158D/M298Q/K337A/S314E-FVII, F374YN158D/E296V/K337A/S314E-FVII, F374Y/L305VN158D/E296V/M298Q-FVII, F374Y/L305VN158D/M298Q/K337A-FVII, F374Y/L305VN158D/E296V/K337A-FVII, F374Y/L305V/V158D/M298Q/S314E-FVII, F374Y/L305VN158D/E296V/S314E-FVII, F374Y/V158T/E296V/M298Q/K337A-FVII, F374Y/V158T/E296V/M298Q/S314E-FVII, F374Y/L305V/V158T/K337A/S314E-FVII, F374Y/V158T/M298Q/K337A/S314E-FVII, F374Y/V158T/E296V/K337A/S314E-FVII, F374Y/L305VN158T/E296V/M298Q-FVII, F374Y/L305VN158T/M298Q/K337A-FVII, F374Y/L305V/V158T/E296V/K337A-FVII, F374Y/L305V/V158T/M298Q/S314E-FVII, F374Y/L305VN158T/E296V/S314E-FVII, F374Y/E296V/M298Q/K337A/V158T/S314E-FVII, F374Y/V158D/E296V/M298Q/K337A/S314E-FVII, F374Y/L305VN158D/E296V/M298Q/S314E-FVII, F374Y/L305V/E296V/M298Q/V158T/S314E-FVII, F374Y/L305V/E296V/M298Q/K337A/V158T-FVII, F374Y/L305V/E296V/K337A/V158T/S314E-FVII, F374Y/L305V/M298Q/K337A/V158T/S314E-FVII, F374Y/L305V/V158D/E296V/M298Q/K337A-FVII, F374Y/L305V/V158D/E296V/K337A/S314E-FVII, F374Y/L305VN158D/M298Q/K337A/S314E-FVII, F374Y/L305V/E296V/M298Q/K337A/V158T/S314E-FVII, F374Y/L305V/V158D/E296V/M298Q/K337A/S314E-FVII, S52A-Factor VII, S60A-Factor VII; R152E-Factor VII, S344A-Factor VII, Factor VIIa lacking the Gla domain; and P11Q/K33E-FVII, T106N-FVII, K143N/N145T-FVII, V253N-FVII, R290N/A292T-FVII, G291N-FVII, R315NN317T-FVII, K143N/N145T/R315NN317T-FVII; and FVII having substitutions, additions or deletions in the amino acid sequence from 233Thr to 240Asn, FVII having substitutions, additions or deletions in the amino acid sequence from 304Arg to 329Cys.

Growth hormone (GH) applicable in the methods of the present invention includes human growth hormone (hGH), which sequence and characteristics are set froth in, e.g. *Hormone Drugs,* Gueriguian, U.S.P. Covention, Rockvill, 1982 and growth hormone compounds. The term "growth hormone compound" is intended to indicate human growth hormone (hGH) in which one or more amino acid residues have been deleted and/or replaced by other amino acid residues, natural or unnatural, and/or hGH comprising addition amino acid residues, natural or unnatural, and/or hGH in which at least one organic substituent is bound to one or more organic substituent. Particular mentioning is made of the 191 native amino acid sequence (somatropin) and the 192 amino acid N-terminal methionine species (somatrem).

Other examples of growth hormone compound applicable in the present invention include wherein amino acid No 172, 174, 176 and 178 as a group are replaced by one of the following groups of amino acids (R, S, F, R); (R, A, Y, R), (K, T, Y, K); (R, S, Y, R); (K, A, Y, R); (R, F, F, R); (K, Q, Y, R); (R, T, Y, H); (Q, R, Y, R); (K, K, Y, K); (R, S, F, S) or (K, S, N, R) as disclosed in WO 92/09690 (Genentech).

Other examples of growth hormone compound applicable in the present invention include hGH with the following substitutions G120R, G120K, G120Y, G120F and G120E, as disclosed in US 6,004931 (Genentech).

Other examples of growth hormone compound applicable in the present invention include hGH with the following set of substitutions R167N, D171S, E174S, F176Y and I179T; R176E, D171S, E174S and F176Y; F10A, M14W, H18D and H21N; F10A, M14W, H18D, H21N, R167N, D171S, E174S, F176Y, I179T; F10A, M14W, H18D, H21N, R167N, D171A, E174S, F176Y, I179T; F10H, M14G, H18N and H21N; F10A, M14W, H18D, H21N, R167N, D171A, T175T and I179T; and F101, M14Q, H18E, R167N, D171S and I179T, as disclosed in US 6,143,523 (Genentech).

Other examples of growth hormone compound applicable in the present invention include hGH with the following set of substitutions H18A, Q22A, F25A, D26A, Q29A, E65A, K168A, E174A and G120K as disclosed in US 6,136,536 (Genentech).

Other examples of growth hormone compound applicable in the present invention include hGH with the following set of substitutions H18D, H21 N, R167N, K168A, D171S, K172R, E174S, I179T and wherein G120 is further substituted with either R, K, W, Y, F or E, as disclosed in US 6,057,292 (Genentech).

Other examples of growth hormone compound applicable in the present invention include hGH with the following set of substitutions H18D, H21 N, R167N, K168A, D171S, K172R, E174S and I179T, as disclosed in US 5,849,535 (Genentech).

Other examples of growth hormone compound applicable in the present invention include hGH with the following set of substitutions H18D, H21 D, R167N, K168A, D171S, K172R, E174S and I179T; and H18A, Q22A, F25A, D26A, Q29A, E65A, K168A and E174A, as disclosed in WO 97/11178 (Genentech).

Other examples of growth hormone compound applicable in the present invention include hGH with the following set of substitutions K168A and E174A; R178N and I179M; K172A and F176A; and H54F, S56E, L58I, E62S, D63N and Q66E as disclosed in WO 90/04788 (Genentech).

Examples of cytokines which could be modified using the method of the present invention include erythropoietin (EPO), thrombopoietin, INF-α, IFN-β, IFN-γ, TNF-α, interleukin-1β (IL-1-β), IL-3, IL-4, IL-5, IL-10, IL-12, IL-15, IL-18, IL-19, IL-20, IL-21 IL-24, grannolyte colony-stimulating factor (G-CSF), GM-CSF, and chemokines such as machrophage inflammatory protein-1 (MIP-1) gamma interferon inducible protein and monokines induced by IFNγ (MIG).

Particular examples of IL-19 applicable in the methods of the present invention include those disclosed WO 98/08870 (Human Genome Science),. Particular mentioning is made of the peptide disclosed as SEQ ID NO:2 in WO 98/08870.

Particular examples of applicable IL-20 include those disclosed in WO 99/27103 (Zymogenetics). In the present context, IL-20 is intended to indicate IL-20 itself and fragments thereof as well as polypeptides being at least 90% identical to IL-20 or fragments thereof. Proteins particular applicable in the methods of the present invention includes those disclosed in WO 99/27103 as SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34 and SEQ ID NO:35.

Examples of IL-21 applicable in the methods of the present invention include those disclosed in WO 00/53761 (Zymogenetics). particular mentioning is made of the peptide disclosed as SEQ ID NO:2 in WO 00/53761.

TTF are applicable in the methods of the present invention. TTF peptides are a family of peptides found mainly in association with the gastrointestinal tract. Particular mentioning is made of breast cancer associated pS2 peptide (TFF-1), which is known from human, mouse, and rat, spasmolytical polypeptide (TFF-2), which is known from human, pig, rat, and mouse and intestinal trefoil factor (TFF-3), known from human, rat and mouse.

Other peptides from the TFF family applicable in the methods of the present invention include those disclosed in WO 02/46226 (Novo Nordisk), which is included herein by reference. Particular mentioning is made of a TFF-2 peptide wherein a TFF2 peptide with an amino acid as disclosed in SEQ ID NO:1 of WO 02/46226 comprising disulphide bonds between Cys6-Cys104, Cys8-Cys35, Cys19-Cys34, Cys29-Cys46, Cys58-Cys84, Cys68-Cys83, and Cys78-Cys95 and wherein a moiety X independently selected from sugar residues and oligosaccharides is covalently attached to Asn15.

Other peptides of the TFF family include TFF-1 and TFF-3 dimers as those disclosed in WO 96/06861 (Novo Nordisk).

Several melanorcortin receptors are known, and particular mentioning of peptides applicable for the methods of the present invention is made of peptidic melanocortin-4 receptor agonists, which are known to have an appetite suppressive effect. Particular mentioning is made of peptides or proteins disclosed in the following patent documents,: US 6,054,556 (Hruby), WO 00/05263 (William Harvey Research), WO 00/35952 (Melacure), WO 00/35952 (Melacure), WO 00/58361 (Procter & Gamble), WO 01/52880 (Merck), WO 02/26774 (Procter & Gamble), WO 03/06620 (Palatin), WO 98/27113 (Rudolf Magnus Institute) and WO 99/21571 (Trega).

Other classes of peptides or proteins which are applicable in the methods of the present invention include enzymes. Many enzymes are used for various industrial purposes, and particular mentioning is made of hydrolases (proteases, lipases, cellulases, esterases), oxidoreductases (laccases, peroxidaxes, catalases, superoxide dismutases, lipoxygenases), transferases and isomerases.

Other peptides or proteins applicable in the methods of the present invention include ACTH, corticotropin-releasing factor, angiotensin, calcitonin, insulin and fragments and analogues thereof, glucagon, IGF-1, IGF-2, enterogastrin, gastrin, tetragastrin, pentagastrin, urogastrin, epidermal growth factor, , secretin, nerve growth factor, thyrotropin releasing hormone, somatostatin, growth hormone releasing hormone, somatomedin, parathyroid hormone, thrombopoietin, erythropoietin, hypothalamic releasing factors, prolactin, thyroid stimulating hormones, endorphins, enkephalins, vasopressin, oxytocin, opiods and analogues thereof, asparaginase, arginase, arginine deaminase, adenosine deaminase and ribonuclease.

Peptides to be modified according to the methods of the present invention may either be isolated from natural sources (e.g. plants, animals or micro-organisms, such as yeast, bacteria, fungi or vira) or they may be synthesised. Peptides form natural sources also include peptides form transgenic sources, e.g. sources which have been genetically modified to express or to increase the expression of a peptide, wherein said peptide may be "natural" in the sense that it exists in nature or "unnatural" in the sense that it only exists due to human intervention. Peptides isolated form natural sources may also be subjected to synthetic modification prior to the conjugation of the present invention.

In one embodiment, the application discloses conjugated peptides obtained according to the methods of the present invention. If the conjugated peptide obtained by the methods of the present invention is a therapeutic peptide, the application also provides the use of such compounds in therapy, and pharmaceutical compositions comprising such compounds.

The application discloses conjugated peptides of the formula 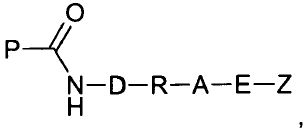 wherein P, R, A, D, E and Z are as defined above, and wherein the group 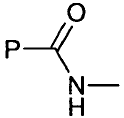 represents a peptide radical obtained by removing a hydrogen from -NH₂ in the side chain of a Gln residue, and pharmaceutically acceptable salts and solvates thereof.

As discussed above, a peptide may contain more than one Gln-residue where the peptide can be conjugated. In that case, the above formula is intended also to indicate a peptide which has been conjugated at more than one site.

To the extend that the unconjugated peptide (P-C(O)-NH₂) is a therapeutic peptide, the invention also relates to the use of the the conjugated peptides I therapy, and in particular to pharmaceutical compositions comprising said conjugated peptides.

Insulin is used to treat or prevent diabetes, and in one embodiment, the present invention thus provides a method of treating type 1 or type 2 diabetes, the method comprising administering to a subject in need thereof a therapeutically effective amount of an insulin or insulin compound conjugate according to the present invention.

In another embodiment, the application provides the use of an insulin or insulin compound conjugate according to the present invention in the manufacture of a medicament used in the treatment of type 1 or type 2 diabetes.

GLP-1 may be used in the treatment of hyperglycemia, type 2 diabetes, impaired glucose tolerance, type 1 diabetes, obesity, hypertension, syndrome X, dyslipidemia, β-cell apoptosis, β-cell deficiency, inflammatory bowel syndrome, dyspepsia, cognitive disorders, e.g. cognitive enhancing, neuroprotection, atheroschlerosis, coronary heart disease and other cardiovascular disorders. In one embodiment, the present application thus provides a method of treating said diseases, the method comprising administering to a subject in need thereof a therapeutically effective amount of a GLP-1 or GLP-1 compound conjugate according to the present invention.

In another embodiment, the application provides the use of a GLP-1 or GLP-1 compound conjugate according to the present invention in the manufacture of a medicament used in the treatment of the above mentioned diseases.

GLP-2 may be used in the treatment of intestinal failure leading to malabsorption of nutrients in the intestines, and in particular GLP-2 may be used in the treatment of small bowel syndrome, Inflammatory bowel syndrome, Crohns disease, colitis including collagen colitis, radiation colitis, post radiation atrophy, non-tropical (gluten intolerance) and tropical sprue, damaged tissue after vascular obstruction or trauma, tourist diarrhea, dehydration, bacteremia, sepsis, anorexia nervosa, damaged tissue after chemotherapy, premature infants, schleroderma, gastritis including atrophic gastritis, postantrectomy atrophic gastritis and helicobacter pylori gastritis, ulcers, enteritis, cul-de-sac, lymphatic obstruction, vascular disease and graft-versus-host, healing after surgical procedures, post radiation atrophy and chemotherapy, and osteoporosis. It is therefore an intension of the present application to provide methods of treating the above diseases, the method comprising administering to a subject in need thereof a therapeutically effective amount of a GLP-2 or GLP-2 compound conjugate according to this invention.

In another embodiment, the present application provides the use of a GLP-2 or GLP-2 compound conjugate according to this invention in the manufacture of a medicament used in the treatment of the above mentioned diseases.

Growth hormone may be used in the treatment of growth hormone deficiency (GHD); Turner Syndrome; Prader-Willi syndrome (PWS); Noonan syndrome; Down syndrome; chronic renal disease, juvenile rheumatoid arthritis; cystic fibrosis, HIV-infection in children receiving HAART treatment (HIV/HALS children); short children born short for gestational age (SGA); short stature in children born with very low birth weight (VLBW) but SGA; skeletal dysplasia; hypochondroplasia; achondroplasia; idiopathic short stature (ISS); GHD in adults; fractures in or of long bones, such as tibia, fibula, femur, humerus, radius, ulna, clavicula, matacarpea, matatarsea, and digit; fractures in or of spongious bones, such as the scull, base of hand, and base of food; patients after tendon or ligament surgery in e.g. hand, knee, or shoulder; patients having or going through distraction oteogenesis; patients after hip or discus replacement, meniscus repair, spinal fusions or prosthesis fixation, such as in the knee, hip, shoulder, elbow, wrist or jaw; patients into which osteosynthesis material, such as nails, screws and plates, have been fixed; patients with non-union or mal-union of fractures; patients after osteatomia, e.g. from tibia or 1^{st} toe; patients after graft implantation; articular cartilage degeneration in knee caused by trauma or arthritis; osteoporosis in patients with Turner syndrome; osteoporosis in men; adult patients in chronic dialysis (APCD); malnutritional associated cardiovascular disease in APCD; reversal of cachexia in APCD; cancer in APCD; chronic abstractive pulmonal disease in APCD; HIV in APCD; elderly with APCD; chronic liver disease in APCD, fatigue syndrome in APCD; Crohn's disease; impaired liver function; males with HIV infections; short bowel syndrome; central obesity; HIV-associated lipodystrophy syndrome (HALS); male infertility; patients after major elective surgery, alcohol/drug detoxification or neurological trauma; aging; frail elderly; osteo-arthritis; traumatically damaged cartilage; erectile dysfunction; fibromyalgia; memory disorders; depression; traumatic brain injury; subarachnoid haemorrhage; very low birth weight; metabolic syndrome; glucocorticoid myopathy; or short stature due to glucucorticoid treatment inchildren. Growth hormones have also been used for acceleration of the healing of muscle tissue, nervous tissue or wounds; the acceleration or improvement of blood flow to damaged tissue; or the decrease of infection rate in damaged tissue, the method comprising administration to a patient in need thereof an effective amount of a therapeutivcally effective amount of a compound of formula I. The present application thus provides a method for treating these diseases or states, the method comprising administering to a patient in need thereof a therapeutically effective amount of a growth hormone or growth hormone compound conjugate according to the present invention.

Typically, the amount of conjugated growth hormone administered is in the range from 10⁻⁷ - 10⁻³ g/kg body weight, such as 10⁻⁶ - 10⁻⁴ g/kg body weight, such as 10⁻⁵ -10⁻⁴ g/kg body weight.

In another embodiment, the application provides the use of a growth hormone or growth hormone compound conjugate in the manufacture of a medicament used in the treatment of the above mentioned diseases or states.

Cytokines are implicated in the etiology of a host of diseases involving the immune system. In particular it is mentioned that IL-20 could be involved in psoriasis and its treatment, and I-21 is involved in cancer and could constitute a treatment to this disease. In one embodiment, the invention provides a method for the treatment of psoriasis comprising the administration of a therapeutically effective amount of a IL-20 conjugate according to the present invention. In another embodiment, the invention relates to the use of an IL-20 conjugate of the present invention in the manufacture of a medicament used in the treatment of psoriasis.

In another embodiment, the present application relates to a method of treating cancer, the method comprising administration of a therapeutically effective amount of a IL-21 conjugate of the present invention to a subject in need thereof.

In another embodiment, the application relates to the use of an IL-21 conjugate according to the present invention in the manufacture of a medicament used in the treatment of cancer.

TTF peptides may be used to increase the viscosity of muscus layers in subject, to reduce secretion of salvia, e.g. where the increase salvia secretion is caused by irradiation therapy, treatment with anticholinergics or Sjögren's syndrome, to treat allergic rhinitis, stress induced gastric ulcers secondary to trauma, shock, large operations, renal or liver diseases, treatment with NSAID, e.g. aspirin, steroids or alcohol. TTF peptides may also be used to treat Chrohn's disease, ulcerative colitis, keratoconjunctivitis, chronic bladder infections, intestinal cystitis, papillomas and bladder cancer. In one embodiment, the invention thus relates the a method of treating the above mention diseases or states, the method comprising administering to a subject patient in need thereof a therapeutically effective amount of a TTF conjugate according to the present invention.

In another embodiment, the application relates the use of a TTF conjugate of the present invention in the manufacture of a medicament for the treatment of the above mentioned diseases or states.

Melanocortin receptor modifiers, and in particular melanorcortin 4 recpetor agonists have been implicated the treatment and prevention of obesity and related diseases. In one embodiment, the present application provides a method for preventing or delaying the progression of impaired glucose tolerance (IGT) to non-insulin requiring type 2 diabetes, for preventing or delaying the progression of non-insulin requiring type 2 diabetes to insulinj requiring diabetes, for treating obesity and for regulating the appetite. Melanocortin 4 receptor agonists have also been implicated in the treatment of diseases selected from atherosclerosis, hypertension, diabetes, type 2 diabetes, impaired glucose tolerance (IGT), dyslipidemia, coronary heart disease, gallbladder disease, gall stone, osteoarthritis, cancer, sexual dysfunction and the risk of premature death. In one embodiment, the application thus provides a method of treating the above diseases or states, the method comprising administering to a subject in need thereof a therapeutically effective amount of an melanocortin 4 recpetor agonist conjugate of the present invention.

In still another embodiment, the application relates to the use of a melanocortin 4 receptor agonist conjugate of the present invention in the manufacture of a medicament for the treatment of the above mentioned diseases or states.

Factor VII compounds have been implicated in the treatment of disease related to coagulation, and biological active Factor VII compounds in particular have been implicated in the treatment of hemophiliacs, hemophiliacs with inhibitors to Factor VIII and IX, patients with thrombocytopenia, patients with thrombocytopathies, such as Glanzmann's thrombastenia platelet release defect and strorage pool defects, patient with von Willebrand's disease, patients with liver disease and bleeding problems associated with traumas or surgery. Biologically inactive Factor VII compounds have been implicated in the treatment of patients being in hypercoagluable states, such as patients with sepsis, deep-vein thrombosis, patients in risk of myocardial infections or thrombotic stroke, pulmonary embolism, patients with acute coronary syndromes, patients undergoing coronary cardiac, prevention of cardiac events and restenosis for patient receiving angioplasty, patient with peripheral vascular diseases, and acute respiratory distress syndrome. In one embodiment, the invention thus provides a method for the treatment of the above mentioned diseases or states, the method comprising administering to a subject in need thereof a therapeutically effective amount of a Factor VII compound conjugate according to the present invention.

In another embodiment, the application provides the use of a Factor VII compound conjugate according to the present invention in the manufacture of a medicament used in the treatment of the above mentioned diseases or states.

Many diseases are treated using more than one medicament in the treatment, either concomitantly administered or sequentially administered. It is therefore within the scope of the present application to use the peptide conjugates of the present invention in therapeutic methods for the treatment of one of the above mentioned diseases in combination with one or more other therapeutically active compound normally used to in the treatment said disease. By analogy, it is also within the scope of the present application to use the peptide conjugates of the present invention in combination with other therapeutically active compounds normally used in the treatment of one of the above mentioned diseases in the manufacture of a medicament for said disease.

As discussed above, therapeutic peptides conjugated according to the methods of the present invention may be used in therapy.

In another embodiment, the present application provides the use of conjugated peptides of the present invention in diagnostics.

### PHARMACEUTICAL COMPOSITIONS

Another purpose is to provide a pharmaceutical composition comprising a conjugated peptide, such as conjugated growth hormone (GH) of the present invention which is present in a concentration from 10⁻¹⁵ mg/ml to 200 mg/ml, such as e.g. 10⁻¹⁰ mg/ml to 5 mg/ml and wherein said composition has a pH from 2.0 to 10.0. The composition may further comprise a buffer system, preservative(s), tonicity agent(s), chelating agent(s), stabilizers and surfactants. In one embodiment of the invention the pharmaceutical composition is an aqueous composition, i.e. composition comprising water. Such composition is typically a solution or a suspension. In a further embodiment of the invention the pharmaceutical composition is an aqueous solution. The term "aqueous composition" is defined as a composition comprising at least 50 % w/w water. Likewise, the term "aqueous solution" is defined as a solution comprising at least 50 %w/w water, and the term "aqueous suspension" is defined as a suspension comprising at least 50 %w/w water.

In another embodiment the pharmaceutical composition is a freeze-dried composition, whereto the physician or the patient adds solvents and/or diluents prior to use.

In another embodiment the pharmaceutical composition is a dried composition (e.g. freeze-dried or spray-dried) ready for use without any prior dissolution.

In a further aspect the application relates to a pharmaceutical composition comprising an aqueous solution of a peptide conjugate, such as e.g. a GH conjugate, and a buffer, wherein said peptide conjugate, such as e.g. GH conjugate is present in a concentration from 0.1-100 mg/ml or above, and wherein said composition has a pH from about 2.0 to about 10.0.

In a another embodiment of the application the pH of the composition is selected from the list consisting of 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, and 10.0.

In a further embodiment of the application the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative embodiment of the application.

In a further embodiment of the application the composition further comprises a pharmaceutically acceptable preservative. In a further embodiment of the application the preservative is selected from the group consisting of phenol, o-cresol, m-cresol, p-cresol, methyl p-hydroxybenzoate, propyl p-hydroxybenzoate, 2-phenoxyethanol, butyl p-hydroxybenzoate, 2-phenylethanol, benzyl alcohol, chlorobutanol, and thiomerosal, bronopol, benzoic acid, imidurea, chlorohexidine, sodium dehydroacetate, chlorocresol, ethyl p-hydroxybenzoate, benzethonium chloride, chlorphenesine (3p-chlorphenoxypropane-1,2-diol) or mixtures thereof. In a further embodiment of the application the preservative is present in a concentration from 0.1 mg/ml to 20 mg/ml. In a further embodiment of the application the preservative is present in a concentration from 0.1 mg/ml to 5 mg/ml. In a further embodiment of the application the preservative is present in a concentration from 5 mg/ml to 10 mg/ml. In a further embodiment of the application the preservative is present in a concentration from 10 mg/ml to 20 mg/ml. Each one of these specific preservatives constitutes an alternative embodiment of the application The use of a preservative in pharmaceutical compositions is well-known to the skilled person. For convenience reference is made to Remington: The Science and Practice of Pharmacy, 20th edition, 2000.

In a further embodiment of the application the composition further comprises an isotonic agent. In a further embodiment of the application the isotonic agent is selected from the group consisting of a salt (e.g. sodium chloride), a sugar or sugar alcohol, an amino acid (e.g. L-glycine, L-histidine, arginine, lysine, isoleucine, aspartic acid, tryptophan, threonine), an alditol (e.g. glycerol (glycerine), 1,2-propanediol (propyleneglycol), 1,3-propanediol, 1,3-butanediol) polyethyleneglycol (e.g. PEG400), or mixtures thereof. Any sugar such as mono-, di-, or polysaccharides, or water-soluble glucans, including for example fructose, glucose, mannose, sorbose, xylose, maltose, lactose, sucrose, trehalose, dextran, pullulan, dextrin, cyclodextrin, soluble starch, hydroxyethyl starch and carboxymethylcellulose-Na may be used. In one embodiment the sugar additive is sucrose. Sugar alcohol is defined as a C4-C8 hydrocarbon having at least one -OH group and includes, for example, mannitol, sorbitol, inositol, galactitol, dulcitol, xylitol, and arabitol. In one embodiment the sugar alcohol additive is mannitol. The sugars or sugar alcohols mentioned above may be used individually or in combination. There is no fixed limit to the amount used, as long as the sugar or sugar alcohol is soluble in the liquid preparation and does not adversely effect the stabilizing effects obtained using the methods of the application. In one embodiment, the sugar or sugar alcohol concentration is between about 1 mg/ml and about 150 mg/ml. In a further embodiment of the the application isotonic agent is present in a concentration from 1 mg/ml to 50 mg/ml. In a further embodiment of the application the isotonic agent is present in a concentration from 1 mg/ml to 7 mg/ml. In a further embodiment of the application the isotonic agent is present in a concentration from 8 mg/ml to 24 mg/ml. In a further embodiment of the application the isotonic agent is present in a concentration from 25 mg/ml to 50 mg/ml. Each one of these specific isotonic agents constitutes an alternative embodiment of the application. The use of an isotonic agent in pharmaceutical compositions is well-known to the skilled person. For convenience reference is made to Remington: The Science and Practice of Pharmacy, 20th edition, 2000.

In a further embodiment of the application the composition further comprises a chelating agent. In a further embodiment of the application the chelating agent is selected from salts of ethylenediaminetetraacetic acid (EDTA), citric acid, and aspartic acid, and mixtures thereof. In a further embodiment of the application the chelating agent is present in a concentration from 0.1mg/ml to 5mg/ml. In a further embodiment of the application the chelating agent is present in a concentration from 0.1 mg/ml to 2mg/ml. In a further embodiment of the application the chelating agent is present in a concentration from 2mg/ml to 5mg/ml. Each one of these specific chelating agents constitutes an alternative embodiment of the application. The use of a chelating agent in pharmaceutical compositions is well-known to the skilled person. For convenience reference is made to Remington: The Science and Practice of Pharmacy, 20th edition, 2000.

In a further embodiment of the application the composition further comprises a stabilizer. The use of a stabilizer in pharmaceutical compositions is well-known to the skilled person. For convenience reference is made to Remington: The Science and Practice of Phar macy, 20th edition, 2000.

More particularly, compositions of the application are stabilized liquid pharmaceutical compositions whose therapeutically active components include a protein that possibly exhibits aggregate formation during storage in liquid pharmaceutical compositions. By "aggregate formation" is intended a physical interaction between the protein molecules that results in formation of oligomers, which may remain soluble, or large visible aggregates that precipitate from the solution. By "during storage" is intended a liquid pharmaceutical composition or composition once prepared, is not immediately administered to a subject. Rather, following preparation, it is packaged for storage, either in a liquid form, in a frozen state, or in a dried form for later reconstitution into a liquid form or other form suitable for administration to a subject. By "dried form" is intended the liquid pharmaceutical composition or composition is dried either by freeze drying (i.e., lyophilization; see, for example, Williams and Polli (1984) J Parenteral Sci. Technol. 38:48-59), spray drying (see Masters (1991) in Spray-Drying Handbook (5th ed; Longman Scientific and Technical, Essez, U.K.), pp. 491-676; Broadhead et al. (1992) Drug Devel. Ind. Pharm. 18:1169-1206; and Mumenthaler et al. (1994) Pharm. Res. 11:12-20), or air drying (Carpenter and Crowe (1988) Cryobiology 25:459-470; and Roser (1991) Biopharm. 4:47-53). Aggregate formation by a protein during storage of a liquid pharmaceutical composition can adversely affect biological activity of that protein, resulting in loss of therapeutic efficacy of the pharmaceutical composition. Furthermore, aggregate formation may cause other problems such as blockage of tubing, membranes, or pumps when the protein-containing pharmaceutical composition is administered using an infusion system.

The pharmaceutical compositions of the application may further comprise an amount of an amino acid base sufficient to decrease aggregate formation by the protein during storage of the composition. By "amino acid base" is intended an amino acid or a combination of amino acids, where any given amino acid is present either in its free base form or in its salt form. Where a combination of amino acids is used, all of the amino acids may be present in their free base forms, all may be present in their salt forms, or some may be present in their free base forms while others are present in their salt forms. In one embodiment, amino acids to use in preparing the compositions of the invention are those carrying a charged side chain such as arginine, lysine, aspartic acid, and glutamic acid. Any stereoisomer (i.e., L or D isomer, or mixtures thereof) of a particular amino acid (methionine, histidine, arginine, lysine, isoleucine, aspartic acid, tryptophan, threonine and mixtures thereof) or combinations of these stereoisomers or glycine or an organic base such as but not limited to imidazole, may be present in the pharmaceutical compositions of the application so long as the particular amino acid or organic base is present either in its free base form or its salt form. In one embodiment the L-stereoisomer of an amino acid is used. In one embodiment the L-stereoisomer is used. Compositions of the application may also be formulated with analogues of these amino acids. By "amino acid analogue" is intended a derivative of the naturally occurring amino acid that brings about the desired effect of decreasing aggregate formation by the protein during storage of the liquid pharmaceutical compositions of the application. Suitable arginine analogues include, for example, aminoguanidine, ornithine and N-monoethyl L-arginine, suitable methionine analogues include ethionine and buthionine and suitable cysteine analogues include S-methyl-L cysteine. As with the other amino acids, the amino acid analogues are incorporated into the compositions in either their free base form or their salt form. In a further embodiment of the application the amino acids or amino acid analogues are used in a concentration, which is sufficient to prevent or delay aggregation of the protein.

In a further embodiment of the application methionine (or other sulphuric amino acids or amino acid analogous) may be added to inhibit oxidation of methionine residues to methionine sulfoxide when the protein acting as the therapeutic agent is a protein comprising at least one methionine residue susceptible to such oxidation. By "inhibit" is intended minimal accumulation of methionine oxidized species over time. Inhibiting methionine oxidation results in greater retention of the protein in its proper molecular form. Any stereoisomer of methionine (L or D isomer) or any combinations thereof can be used. The amount to be added should be an amount sufficient to inhibit oxidation of the methionine residues such that the amount of methionine sulfoxide is acceptable to regulatory agencies. Typically, this means that the composition contains no more than about 10% to about 30% methionine sulfoxide. Generally, this can be obtained by adding methionine such that the ratio of methionine added to methionine residues ranges from about 1:1 to about 1000:1, such as 10:1 to about 100:1.

In a further embodiment of the application the composition further comprises a stabilizer selected from the group of high molecular weight polymers or low molecular compounds. In a further embodiment of the application the stabilizer is selected from polyethylene glycol (e.g. PEG 3350), polyvinyl alcohol (PVA), polyvinylpyrrolidone, carboxy-/hydroxycellulose or derivates thereof (e.g. HPC, HPC-SL, HPC-L and HPMC), cyclodextrins, sulphur-containing substances as monothioglycerol, thioglycolic acid and 2-methylthioethanol, and different salts (e.g. sodium chloride). Each one of these specific stabilizers constitutes an alternative embodiment of the invention.

The pharmaceutical compositions may also comprise additional stabilizing agents, which further enhance stability of a therapeutically active protein therein. Stabilizing agents of particular interest to the present application include, but are not limited to, methionine and EDTA, which protect the protein against methionine oxidation, and a nonionic surfactant, which protects the protein against aggregation associated with freeze-thawing or mechanical shearing.

In a further embodiment of the application the composition further comprises a surfactant. In a further embodiment of the application the surfactant is selected from a detergent, ethoxylated castor oil, polyglycolyzed glycerides, acetylated monoglycerides, sorbitan fatty acid esters, polyoxypropylene-polyoxyethylene block polymers (eg. poloxamers such as Pluronic^{®} F68, poloxamer 188 and 407, Triton X-100), polyoxyethylene sorbitan fatty acid esters, polyoxyethylene and polyethylene derivatives such as alkylated and alkoxylated derivatives (tweens, e.g. Tween-20, Tween-40, Tween-80 and Brij-35), monoglycerides or ethoxylated derivatives thereof, diglycerides or polyoxyethylene derivatives thereof, alcohols, glycerol, lectins and phospholipids (eg. phosphatidyl serine, phosphatidyl choline, phosphatidyl ethanolamine, phosphatidyl inositol, diphosphatidyl glycerol and sphingomyelin), derivates of phospholipids (eg. dipalmitoyl phosphatidic acid) and lysophospholipids (eg. palmitoyl lysophosphatidyl-L-serine and 1-acyl-sn-glycero-3-phosphate esters of ethanolamine, choline, serine or threonine) and alkyl, alkoxyl (alkyl ester), alkoxy (alkyl ether)- derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, and the positively charged DODAC, DOTMA, DCP, BISHOP, lysophosphatidylserine and lysophosphatidylthreonine, and glycerophospholipids (eg. cephalins), glyceroglycolipids (eg. galactopyransoide), sphingoglycolipids (eg. ceramides, gangliosides), dodecylphosphocholine, hen egg lysolecithin, fusidic acid derivatives- (e.g. sodium tauro-dihydrofusidate etc.), long-chain fatty acids and salts thereof C₆-C₁₂ (eg. oleic acid and caprylic acid), acylcarnitines and derivatives, N^{α}-acylated derivatives of lysine, arginine or histidine, or side-chain acylated derivatives of lysine or arginine, N^{α}-acylated derivatives of dipeptides comprising any combination of lysine, arginine or histidine and a neutral or acidic amino acid, N^{α}-acylated derivative of a tripeptide comprising any combination of a neutral amino acid and two charged amino acids, DSS (docusate sodium, CAS registry no [577-11-7]), docusate calcium, CAS registry no [128-49-4]), docusate potassium, CAS registry no [7491-09-0]), SDS (sodium dodecyl sulphate or sodium lauryl sulphate), sodium caprylate, cholic acid or derivatives thereof, bile acids and salts thereof and glycine or taurine conjugates, ursodeoxycholic acid, sodium cholate, sodium deoxycholate, sodium taurocholate, sodium glycocholate, N-Hexadecyl-N,N-dimethyl-3-ammonio-1-propanesulfonate, anionic (alkyl-aryl-sulphonates) monovalent surfactants, zwitterionic surfactants (e.g. N-alkyl-N,N-dimethylammonio-1-propanesuffonates, 3-cholamido-1-propyldimethylammonio-1-propanesulfonate, cationic surfactants (quaternary ammonium bases) (e.g. cetyl-trimethylammonium bromide, cetylpyridinium chloride), nonionic surfactants (eg. Dodecyl β-D-glucopyranoside), poloxamines (eg. Tetronic's), which are tetrafunctional block copolymers derived from sequential addition of propylene oxide and ethylene oxide to ethylenediamine, or the surfactant may be selected from the group of imidazoline derivatives, or mixtures thereof. Each one of these specific surfactants constitutes an alternative embodiment of the application.

The use of a surfactant in pharmaceutical compositions is well-known to the skilled person. For convenience reference is made to Remington: The Science and Practice of Pharmacy, 20th edition, 2000.

It is possible that other ingredients may be present in the pharmaceutical composition of the present application. Such additional ingredients may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical composition of the present application.

Pharmaceutical compositions containing a peptide conjugate, such as e.g. a GH conjugate according to the present invention may be administered to a patient in need of such treatment at several sites, for example, at topical sites, for example, skin and mucosal sites, at sites which bypass absorption, for example, administration in an artery, in a vein, in the heart, and at sites which involve absorption, for example, administration in the skin, under the skin, in a muscle or in the abdomen.

Administration of pharmaceutical compositions according to the application may be through several routes of administration, for example, lingual, sublingual, buccal, in the mouth, oral, in the stomach and intestine, nasal, pulmonary, for example, through the bronchioles and alveoli or a combination thereof, epidermal, dermal, transdermal, vaginal, rectal, ocular, for examples through the conjunctiva, uretal, and parenteral to patients in need of such a treatment.

Compositions of the current application may be administered in several dosage forms, for example, as solutions, suspensions, emulsions, microemulsions, multiple emulsion, foams, salves, pastes, plasters, ointments, tablets, coated tablets, rinses, capsules, for example, hard gelatine capsules and soft gelatine capsules, suppositories, rectal capsules, drops, gels, sprays, powder, aerosols, inhalants, eye drops, ophthalmic ointments, ophthalmic rinses, vaginal pessaries, vaginal rings, vaginal ointments, injection solution, in situ transforming solutions, for example in situ gelling, in situ setting, in situ precipitating, in situ crystallization, infusion solution, and implants.

Compositions of the application may further be compounded in, or attached to, for example through covalent, hydrophobic and electrostatic interactions, a drug carrier, drug delivery system and advanced drug delivery system in order to further enhance stability of the GH conjugate, increase bioavailability, increase solubility, decrease adverse effects, achieve chronotherapy well known to those skilled in the art, and increase patient compliance or any combination thereof. Examples of carriers, drug delivery systems and advanced drug delivery systems include, but are not limited to, polymers, for example cellulose and derivatives, polysaccharides, for example dextran and derivatives, starch and derivatives, poly(vinyl alcohol), acrylate and methacrylate polymers, polylactic and polyglycolic acid and block copolymers thereof, polyethylene glycols, carrier proteins, for example albumin, gels, for example, thermogelling systems, for example block co-polymeric systems well known to those skilled in the art, micelles, liposomes, microspheres, nanoparticulates, liquid crystals and dispersions thereof, L2 phase and dispersions there of, well known to those skilled in the art of phase behaviour in lipid-water systems, polymeric micelles, multiple emulsions, self-emulsifying, self-microemulsifying, cyclodextrins and derivatives thereof, and dendrimers.

Compositions of the current application are useful in the composition of solids, semi-solids, powder and solutions for pulmonary administration of a peptide conjugate, such as e.g. a GH conjugate, using, for example a metered dose inhaler, dry powder inhaler and a nebulizer, all being devices well known to those skilled in the art.

Compositions of the current application are specifically useful in the composition of controlled, sustained, protracting, retarded, and slow release drug delivery systems. More specifically, but not limited to, compositions are useful in composition of parenteral controlled release and sustained release systems (both systems leading to a many-fold reduction in number of administrations), well known to those skilled in the art. Even more preferably, are controlled release and sustained release systems administered subcutaneous. Examples of useful controlled release system and compositions are hydrogels, oleaginous gels, liquid crystals, polymeric micelles, microspheres, nanoparticles,

Methods to produce controlled release systems useful for compositions of the current application include, but are not limited to, crystallization, condensation, co-crystallization, precipitation, co-precipitation, emulsification, dispersion, high pressure homogenisation, encapsulation, spray drying, microencapsulating, coacervation, phase separation, solvent evaporation to produce microspheres, extrusion and supercritical fluid processes. General reference is made to Handbook of Pharmaceutical Controlled Release (Wise, D.L., ed. Marcel Dekker, New York, 2000) and Drug and the Pharmaceutical Sciences vol. 99: Protein Composition and Delivery (MacNally, E.J., ed. Marcel Dekker, New York, 2000).

Parenteral administration may be performed by subcutaneous, intramuscular, intraperitoneal or intravenous injection by means of a syringe, optionally a pen-like syringe. Alternatively, parenteral administration can be performed by means of an infusion pump. A further option is a composition which may be a solution or suspension for the administration of the peptide conjugate, such as e.g. the GH conjugate in the form of a nasal or pulmonal spray. As a still further option, the pharmaceutical compositions containing the peptide conjuaget, such as e.g. the GH conjugate of the invention can also be adapted to transdermal administration, e.g. by needle-free injection or from a patch, optionally an iontophoretic patch, or transmucosal, e.g. buccal, administration.

The term "stabilized composition" refers to a composition with increased physical stability, increased chemical stability or increased physical and chemical stability.

The term "physical stability" of the protein composition as used herein refers to the tendency of the protein to form biologically inactive and/or insoluble aggregates of the protein as a result of exposure of the protein to thermo-mechanical stresses and/or interaction with interfaces and surfaces that are destabilizing, such as hydrophobic surfaces and interfaces. Physical stability of the aqueous protein compositions is evaluated by means of visual inspection and/or turbidity measurements after exposing the composition filled in suitable containers (e.g. cartridges or vials) to mechanical/physical stress (e.g. agitation) at different temperatures for various time periods. Visual inspection of the compositions is performed in a sharp focused light with a dark background. The turbidity of the composition is characterized by a visual score ranking the degree of turbidity for instance on a scale from 0 to 3 (a composition showing no turbidity corresponds to a visual score 0, and a composition showing visual turbidity in daylight corresponds to visual score 3). A composition is classified physical unstable with respect to protein aggregation, when it shows visual turbidity in daylight. Alternatively, the turbidity of the composition can be evaluated by simple turbidity measurements well-known to the skilled person. Physical stability of the aqueous protein compositions can also be evaluated by using a spectroscopic agent or probe of the conformational status of the protein. The probe is preferably a small molecule that preferentially binds to a non-native conformer of the protein. One example of a small molecular spectroscopic probe of protein structure is Thioflavin T. Thioflavin T is a fluorescent dye that has been widely used for the detection of amyloid fibrils. In the presence of fibrils, and perhaps other protein configurations as well, Thioflavin T gives rise to a new excitation maximum at about 450 nm and enhanced emission at about 482 nm when bound to a fibril protein form. Unbound Thioflavin T is essentially non-fluorescent at the wavelengths.

Other small molecules can be used as probes of the changes in protein structure from native to non-native states. For instance the "hydrophobic patch" probes that bind preferentially to exposed hydrophobic patches of a protein. The hydrophobic patches are generally buried within the tertiary structure of a protein in its native state, but become exposed as a protein begins to unfold or denature. Examples of these small molecular, spectroscopic probes are aromatic, hydrophobic dyes, such as antrhacene, acridine, phenanthroline or the like. Other spectroscopic probes are metal-amino acid complexes, such as cobalt metal complexes of hydrophobic amino acids, such as phenylalanine, leucine, isoleucine, methionine, and valine, or the like.

The term "chemical stability" of the protein composition as used herein refers to chemical covalent changes in the protein structure leading to formation of chemical degradation products with potential less biological potency and/or potential increased immunogenic properties compared to the native protein structure. Various chemical degradation products can be formed depending on the type and nature of the native protein and the environment to which the protein is exposed. Elimination of chemical degradation can most probably not be completely avoided and increasing amounts of chemical degradation products is often seen during storage and use of the protein composition as well-known by the person skilled in the art. Most proteins are prone to deamidation, a process in which the side chain amide group in glutaminyl or asparaginyl residues is hydrolysed to form a free carboxylic acid. Other degradations pathways involves formation of high molecular weight transformation products where two or more protein molecules are covalently bound to each other through transamidation and/or disulfide interactions leading to formation of covalently bound dimer, oligomer and polymer degradation products (Stability of Protein Pharmaceuticals, Ahern. T.J. & Manning M.C., Plenum Press, New York 1992). Oxidation (of for instance methionine residues) can be mentioned as another variant of chemical degradation. The chemical stability of the protein composition can be evaluated by measuring the amount of the chemical degradation products at various time-points after exposure to different environmental conditions (the formation of degradation products can often be accelerated by for instance increasing temperature). The amount of each individual degradation product is often determined by separation of the degradation products depending on molecule size and/or charge using various chromatography techniques (e.g. SEC-HPLC and/or RP-HPLC).

Hence, as outlined above, a "stabilized composition" refers to a composition with increased physical stability, increased chemical stability or increased physical and chemical stability. In general, a composition must be stable during use and storage (in compliance with recommended use and storage conditions) until the expiration date is reached.

In one embodiment of the application the pharmaceutical composition comprising the GH conjugate is stable for more than 6 weeks of usage and for more than 3 years of storage.

In another embodiment of the application the pharmaceutical composition comprising the GH conjugate is stable for more than 4 weeks of usage and for more than 3 years of storage.

In a further embodiment of the application the pharmaceutical composition comprising the GH conjugate is stable for more than 4 weeks of usage and for more than two years of storage.

In an even further embodiment of the application the pharmaceutical composition comprising the GH conjugate is stable for more than 2 weeks of usage and for more than two years of storage.

All headings and sub-headings are used herein for convenience only and should not be construed as limiting the invention in any way.

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The citation of patent documents herein is done for convenience only and does not reflect any view of the validity, patentability, and/or enforceability of such patent documents.

### EXAMPLES

The peptide to be conjugated, dissolved in a suitable solvent, such as e.g. water is mixed with the first compound (as discussed above) in 5 to 1000 fold excess and the transglutaminase is added. Suitable transglutaminases are e.g. those isolated from *Streptomyces mobaraenese, Streptomyces lyticus* or guinea-pig liver. The amount of transglutaminase to be added depend on the desired rate of reaction. The more enzyme added the faster the reaction goes. The temperature can be ambient or slightly elevated up to approximately 40°C. When the reaction has reached a desired point, i.e. a point where a desired fraction of the peptide to be conjugated has been functionalised, the second compound (as discussed above) is added to afford the conjugated peptide. The conjugated peptide may subsequently be purified, e.g. by column techniques. One or more additional purification steps may also be included earlier in the reaction sequence, e.g. to remove excess first compound or to remove the enzyme. In the second step, the temperature may be raised to increase the reaction rate as this step does not depend on enzymatic activity. Typical reaction conditions can be found in Biochem., 35, 13072-13080, 1996, Bioconjugate Chem., 11, 502-509, 2000, and Bioconjugate Chem., 12, 701-710, 1991.

### Abbreviations

TGase: Microbial transglutaminase from Streptoverticillium mobaraenae according to US 5156956 or from Streptomyces Lydicus according to WO 9606931-A1.

### Analytical methods:

### Maldi-Tof mass spectrometry.

Molecular weights were determined using the Autoflex Maldi-Tof instrument (Bruker). Samples were prepared according to the sandwich method. Matrix 1 was a solution of 10 mg alfa-cyano-4-hydroxy-cinnamic acid in 1 ml acetone. Matrix 2 was a solution of 10 mg alfa-cyano-4-hydroxy-cinnamic acid in 1 ml 50% acetonitrile in water. Samples were prepared on the garget by sequentially applying 1 µl matrix 1, air drying, applying 1 µl 3% trifluoracetic acid, applying 1µl sample, applying 1 µl matrix 2, air drying, washing by flushing the target plate with water and finally air drying. The spectra were acquired using 20% laser power and the standard method for the 3-20 kDa range which was supplied with the instrument.

### RP-HPLC.

RP-HPLC analysis was performed on a Waters 2690 Separation Module equipped with a Waters 996 diode array detector. A Vydac 218TP54 4.6mm x 250mm 5µm C-18 silica column (The Separations Group, Hesperia) was used and detection was by UV at 214 nm, 254 nm, 280 nm and 301 nm. The column was equilibrated with 0.1% trifluoracetic acid / H₂O and eluted by a gradient of 0 to 90% acetonitrile against 0.1% trifluoracetic acid /H₂O over 50 min at 42°C, with a flow of 0.5ml/min.

### LC-MS

LC-MS analysis was performed on a PE-Sciex API 100 mass spectrometer equipped with two Perkin Elmer Series 200 Micropumps, a Perkin Elmer Series 200 autosampler, a Applied Biosystems 785A UV detector and a Sedex 75 Evaporative Light scattering detector. A Waters Xterra 3.0 mm x 50 mm 5µ C-18 silica column was eluted at 1.5 ml/min at room temperature. It was equilibrated with 5 % acetonitrile / 0.1% trifluoracetic acid / H₂O and eluted for 1.0 min with 5% acetonitrile / 0.1% trifluoracetic acid / H₂O and then with a linear gradient to 90% acetonitrile / 0.1% trifluoracetic acid / H₂O over 7 min. Detection was by UV detection at 214nm and Evaporative light Scattering. A fraction of the column eluate was introduced into the ionspray interface of a PE-Sciex API 100 mass spectrometer. The mass range 300 - 2000 amu was scanned every 2 seconds during the run.

### Edman sequencing:

Amino acid sequences were determined by automated Edman degradations using an Applied Biosystem Model 494 Protein Sequencer essentially as described by the manufacturer. In general 50 pmol of peptide was used for an analysis. A PEGylated or fatty-acid derivatized amino acid residue displays a blank Edman cycle.

### Quantification of protein

Protein concentrations were estimated by measuring absorbance at 280 nm using a UV-spectrofotometer. A molar molar extinction coefficient of 16170 M⁺¹ cm⁺¹ was used. Amounts were calculated from volumes and concentrations.

### Enzymatic peptide mapping for determation of site(s) of derivatization.

Peptide mapping was performed using Asp-N digestion of the reduced and alkylated protein. First the protein was treated with DTT (Dithiothreitol) and iodoacetamide according to standard procedures. The alkylated product was purified using HPLC. Subsequently the alkylated purified product was digested overnight with endoprotease Asp-N (Boehringer) at an enzyme:substrate ratio of 1:100. The digest was HPLC separated using a C-18 column and standard trifluoracetic acid/acetonitrile buffer system. The resulting peptide map was compared to that of un-derivatized hGH and fractions with different retention times were collected and further analyzed using Maldi-tof mass spectrometry.

### SDS page

SDS poly-acrylamide gel electrophoresis was performed using NuPAGE 4% - 12 % Bis-Tris gels (Invitrogen NP0321 BOX). The gels were silver stained (Invitrogen LC6100) or Coomassie stained (Invitrogen LC6065) and where relevant also stained for PEG with barium iodide as described by M. M. Kurfurst in Anal.Biochem. 200(2):244-248, 1992.

### Illustrative scheme for the conjugation of hGH with mPEG or with an lipophilic moiety

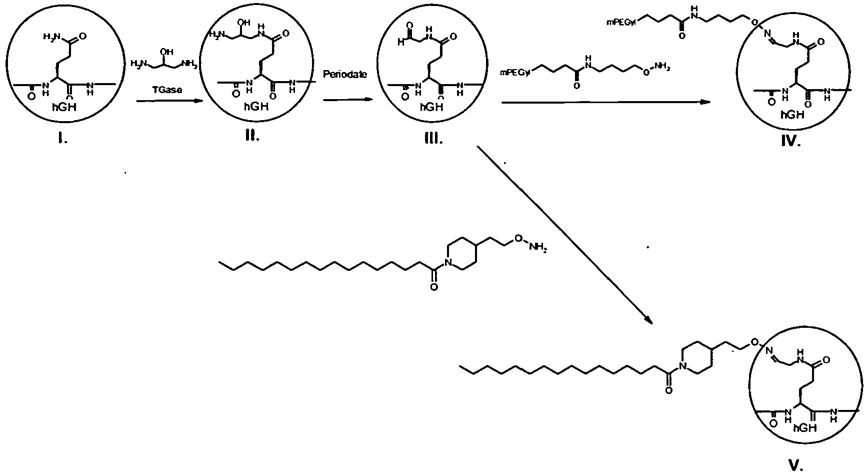

I. hGH, human growth hormone
II. N^{ε141}-(2-hydroxy-3-amino-propyl) hGH
III. N^{ε141}-(2-oxo-etyl) hGH
IV. N^{ε141}-[2-(4-(4-(mPEGyl)butanoyl)-amino-butyloxyimino)-ethyl] hGH
V. Nε¹⁴¹-[2-(1-(hexadecanoyl)piperidin-4-yl)ethyloxyimino)-ethyl] hGH

### Example 1. Trans-amination of hGH (I.) to give N^{ε141}-(2-hydroxy-3-amino-propyl) hGH (II.)

hGH (I.) (200 mg) was dissolved in phosphate buffer (50 mM, pH 8.0, 14 ml).

This solution was mixed with a solution of 1,3-Diamino-propan-2-ol (378 mg) dissolved in phosphate buffer (50 mM, 1 ml, pH 8.0, pH adjusted to 8.0 with dilute hydrochloric acid after dissolution of 1,3-Diamino-propan-2-ol).

Finally a solution of TGase (18 mg - 40 U) dissolved in phosphate buffer (50 mM, pH 8.0, 1 ml) was added and the volume was adjusted to 10 ml by addition of phosphate buffer (50 mM, pH 8) giving a concentration of 1,3-Diamino-propan-2-ol at 0.2 M. The combined mixture was incubated for 4 hours at 37 °O.

The temperature was lowered to room temperature and N-ethyl-maleimide was added to a final concentration of 1 mM.

After further 1 hour the mixture was diluted with 10 volumes of tris buffer (50 mM, pH 8.5)

### Example 2. Ion exchange chromatography of N^{ε141}-(2-hydroxy-3-amino-propyl) hGH (II.)

The solution resulting from example 1. was applied to a MonoQ 10/100 GL column (Amersham Biosciences cat. No. 17- 5167-01) prequilibrated with buffer A (50 mM tris, pH 8.5). It was then eluted at a flow of 2 ml/min with a gradient of 3% to 6% of buffer B (50 mM tris, 2 M NaCl, pH 8.5) in buffer A over 40 min. Fractions were collected based on UV absorbtion at 280 nm and Maldi-Tof analysis was performed on selected fractions. The fractions corresponding to the largest peak giving the expected mw according to Maldi-Tof mass spectrometry were pooled.

### Example 3 Characterization of N^{ε141}-(2-hydroxy-3-amino-propyl) hGH (II.)

Peptide mapping of the pool collected in example 2 showed that the Asp-N fragment AA 130-146 displayed a mass increase of 73 amu corresponding to the addition of the amino alcohol in the side chain of a Glutamine residue. This was the only peptide, that had changed retention time in the HPLC map when compared to that of native hGH. This fragment contains two Glutamine residues. The peptide was subjected to Edman sequencing and Gln-137 was found at the expected yield, whereas Gln-141 displayed a blank Edman cycle. It was concluded, that derivatization had taken place selectively at Gln-141.

### Example 4. Synthesis of N-(4-Aminooxy-butyl)-4-mPEGyl-butyramide wherein mPEGyl is polydisperse and has a molecular weight of approximately 20 kDa.

### Step 1: 2-(4-(tert-Butoxycarbonylaminoxy)butyl)isoindole-1,3-dione

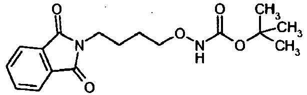

To a mixture of commercially available N-(4-bromobutyl)phthalimide (2.82g, 10 mmol) and N-Boc-hydroxylamine (2.08 g, 15.6 mmol) was added acetonitrile (2 ml) and successively 1,8-diazabicyclo[5.4.0]undec-7-ene (2.25 ml, 15 mmol). The reaction mixture was stirred at room temperature for 30 min and then at 50°C for 2 days. It was diluted with a mixture of water (30 ml) and 1 N hydrochloric acid (20 ml). It was extracted with ethyl acetate (2 x 100 ml). The organic phase was washed with brine (50 ml) and was dried over magnesium sulphate. The crude product was purified by chromatography on silica (60 g), using a gradient of heptane/ethyl acetate 1:0 to 0:1 as eluent to give 2.08 g of 2-(4-(tert-butoxycarbonylaminoxy)butyl)isoindole-1,3-dione.

### Step 2: N-(4-aminobutoxy)carbamic acid tert-butyl ester

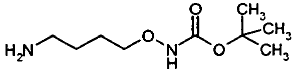

Hydrazine hydrate (1.0 ml, 20 mmol) was added to a solution of 2-(4-(tert-butoxycarbonylaminoxy)butyl)isoindole-1,3-dione (2.08 g, 6.22 mmol) in ethanol (8.0 ml). The reaction mixture was stirred at 80°C for 65 h. The solvent was removed in vacuo. The residue was dissolved in toluene (10 ml) and the solvent was removed in vacuo. The residue was suspended in 1 N hydrochloric acid (10 ml). The precipitation was removed by filtration and was washed with water (2 ml). The filtrate and the wash-liquids were combined and made basic with potassium carbonate. The solution was extracted with dichloromethane (4 x 20 ml). The organic layer was dried over magnesium sulphate. The solvent was removed in vacuo to give 0.39 g of *N*-(4-aminobutoxy)carbamic acid tert-butyl ester. Potassium carbonate (3 g) was added to the aqueous phase, which was extracted with dichloromethane (3 x 20 ml). These combined organic layers were dried over magnesium sulphate. The solvent was removed in vacuo to give another 0.39 g of *N*-(4-aminobutoxy)carbamic acid tert-butyl ester.

### Step 3: N-(4-(4-(mPEG20000yl)butanolyamino)butoxy)carbamic acid tert-butyl ester

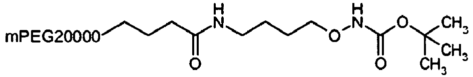

The commercially available *N*-hydroxysuccinimide ester of mPEG2000ylbutanoic acid (Nektar "mPEG-SBA", # 2M450P01, 3 g, 0.15 mmol) was dissolved in dichloromethane (25 ml). *N*-(4-Aminobutoxy)carbamic acid tert-butyl ester (0.12 g, 0.59 mmol) was added. The reaction mixture was shaken at room temperature. Diethyl ether was added until a precipitation was obtained. The precipitation was isolated by filtration. The material was dried in vacuo to yield 2.39 g of *N*-(4-(4-(mPEG20000yl)butanolyamino)butoxy)carbamic acid tert-butyl ester.

### Step 4: N-(4-Aminoxybutyl)-4-(mPEG20000yl)butanolyamide

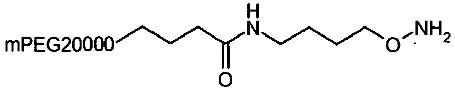

Trifluoroacetic acid (20 ml) was added to a solution of *N*-(4-(4-(mPEG20000yl)butanolyamino)butoxy)carbamic acid tert-butyl ester (2.39 g, 0.12 mmol) in dichloromethane (20 ml). The reaction mixture was shaken for 30 min. Diethyl ether (100 ml) was added. The formed precipitation was isolated by filtration. It was washed with diethyl ether (2 x 100 ml) and dried in vacuo to give 1.96 g of *N*-(4-aminoxybutyl)-4-(mPEG20000yl)butanolyamide

### Example 5. Oxidation of N^{ε141}-(2-hydroxy-3-amino-propyl) hGH (II.) to give N^{ε141}-(2-oxoetyl) hGH (III.)

The buffer of the pooled fractions from example 2 containing 48.7 mg of (II.) was exchanged four times to a 15 mM triethanolamine pH 8.5 (adjusted with 1 N hydrochloric acid) buffer using an Amicon Ultra-15 ultrafiltration device (Millipore). Finally the solution was concentrated to 2 ml .To this was added 2 mll of a 100 mM methionine solution in 15 mM triethanolamine buffer at pH 8.5. Finally 0.4 ml of a 25 mM sodiumperiodate in water was added, and the mixture was incubated for 30 min at room temperature. Then it was cooled on ice and 1.6 ml ice cold N,N-dimethylformamide was added.

### Example 6. Oximation of N^{ε141}-(2-oxo-etyl) hGH (III.) with N-(4-Aminooxy-butyl)-4-mPEGyl-butyramide to give N^{ε141}-[2-(4-(4-(mPEGyl)butanoyl)-amino-butyloxyimino)-ethyl] hGH (IV.) wherein mPEGyl is polydisperse and has a molecular weight of approximately 20 kDa

380 mg *N*-(4-Aminooxy-butyl)-4-mPEGyl-butyramide was dissolved in 4 ml water and pH adjusted to 6.0 with 1 N hydrochloric acid. The mixture resulting from example 5 was then added slowly under gentle mixing and the reaction was allowed to proceed at room temperature for 72 h.

### Example 7. Ion exchange chromatography of N^{ε141}-[2-(4-(4-(mPEGyl)butanoyl)-amino-butyloxyimino)-ethyl] hGH (IV.) wherein mPEGyl is polydisperse and has a molecular weight of approximately 20 kDa

The solution resulting from example 6 was applied to a MonoQ 10/100 GL column (Amersham Biosciences cat. No. 17- 5167-01) pre-equilibrated with buffer A (50 mM tris, pH 8.5). It was then eluted at a flow of 0.5 ml/min with a gradient of 0% to 7% of buffer B (50 mM tris, 2 M NaCl, pH 8.5) in buffer A over 1120 min. Fractions were collected based on UV absorption at 280 nm and Maldi-Tof analysis was performed on selected fractions. The fractions corresponding to the largest peak giving the expected mw according to Maldi-Tof mass spectrometry were pooled. Maldi-Tof analysis gave a broad peak centered around 43130 Da in agreement with the polydisperse nature of mPEG. SDS page showed a single band with an apparent molecular weight of 60 kDa. The band stained both with silver and with barium iodide, confirming that it was a PEG derivatized protein. These analytical results confirmed that the isolated product compound was a mono pegylated derivative of hGH.

### Example 8. Synthesis of 1-[4-(2-(Aminooxy)ethyl)piperidin-1-yl]hexadecan-1-one

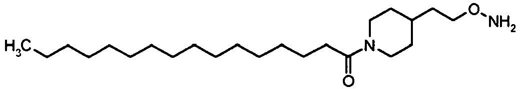

### Step 1:

### 4-[2-(Toluene-4-sulfonyloxy)ethyl]piperidine-1-carboxylic acid tert-butyl ester

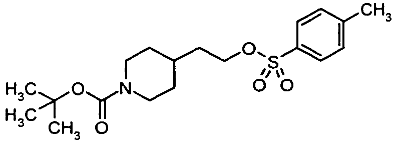

Tosyl chloride (4.16 g, 21.8 mmol) was added to a solution of commercially available 4-(2-hydroxyethyl)piperidine-1-carbocylic ester tert-butyl ester (e.g Aldrich 54,724-7, 5.0 g, 21.8 mmol) and triethylamine (4.25 ml, 30.5 mmol) in dichloromethane (100 ml). The reaction mixture was stirred at room temperature for 16 h. It was diluted with ethyl acetate (300 ml) and washed with a 10% aqueous solution of sodium hydrogen sulphate (200 ml). The aqueous phase was extracted with ethyl acetate (150 ml). The combined organic layers were washed with a saturated aqueous solution of sodium hydrogencarbonate (250 ml) and dried over magnesium sulphate. The solvent was removed in vacuo. The crude product was purified by flash chromatography on silica (80 g), using ethyl acetate/heptane first: 1:2 then 1:1 as eluent, to give 6.04 g of 4-[2-(toluene-4-sulfonyloxy)ethyl]piperidine-1-carboxylic acid tert-butyl ester.

¹H-NMR (CDCl₃): δ 1.05 (m, 2 H); 1.45 (s, 9 H); 1.55 (m, 5 H); 2.50 (s, 3 H); 2.65 (t, 2 H); 4.05 (m, 4 H); 7.35 (d, 2 H); 7.80 (d, 2 H).

Step 2:

### 4-[2-(1,3-Dioxo-1,3-dihydroisoindol-2-yloxy)ethyl]piperidine-1-carboxylic acid tert-butyl ester

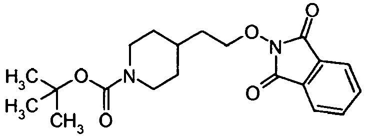

At 0°C, a 60% suspension of sodium hydride in mineral oil (0.69 g, 17.2 mmol) was added to a solution of *N*-hydroxyphthalimide (2.80 g, 17.2 mmol) in *N,N*-dimethylformamide (20 ml). The reaction mixture was stirred for 45 min at 0°C. A solution of 4-[2-(toluene-4-sulfonyloxy)ethyl]piperidine-1-carboxylic acid tert-butyl ester (5.99 g, 15.6 mmol) in N,N-dimethylformamide (15 ml) and tetrabutylammonium iodide (0.17 g, 0.47 mmol) were added successively. The reaction mixture was heated to 60°C for 2 days and cooled to room temperature. Water (5 ml) was added carefully. The reaction mixture was diluted with ethyl acetate (250 ml) and washed with a 10% aqueous solution of sodium hydrogensulphate (200 ml). The aqueous phase was extracted with ethyl acetate (200 ml). The combined organic layers were washed with a saturated aqueous solution of sodium hydrogencarbonate (150 ml) and dried over magnesium sulphate. The solvent was removed in vacuo. The crude product was purified by flash chromatography on silica (80 g), using ethyl acetate/heptane 1:1 as eluent to give 4.36 g of 4-[2-(1,3-dioxo-1,3-dihydroisoindol-2-yloxy)ethyl]piperidine-1-carboxylic acid tert-butyl ester.

¹H-NMR (CDCl₃): δ 1.15 (m, 2 H); 1.50 (s, 9 H); 1.75 (m, 5 H); 2.75 (m, 2 H); 4.10 (m, 2 H); 4.30 (t, 2 H); 7.80 (m, 4 H).

### Step 3:

### 2-(2-(Piperidin-4-yl)ethoxy)isoindole-1,3-dione

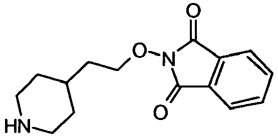

Trifluoroacetic acid (20 ml) was added to a solution of 4-[2-(1,3-dioxo-1,3-dihydroisoindol-2-yloxy)ethyl]piperidine-1-carboxylic acid tert-butyl ester (4.26 g, 11.4 mmol) in dichloromethane (20 ml). The reaction mixture was stirred at room temperature for 50 min. The solvent was removed in vacuo. The residue was dissolved in dichloromethane (50 ml) and the solvent was removed in vacuo. The latter procedure was repeated twice to give 6.46 g of the crude trifluoroacetate salt of 2-(2-(piperidin-4-yl)ethoxy)isoindole-1,3-dione.
MS: m/z = 275 [M+1⁺]
¹H-NMR (DMSO-d₆): δ 1.30 (m, 2 H); 1.65 (m, 2 H); 1.90 (m, 3 H); 2.90 (q, 2 H); 3.30 (d, 2 H); 4.20 (t, 2 H); 7.90 (s, 4 H); 8.30 (br, 1 H); 8.65 (br, 1 H).

### Step 4:

### 2-[2-(1-(Hexadecanoyl)piperidin-4-yl)ethoxy]isoindole-1,3-dione

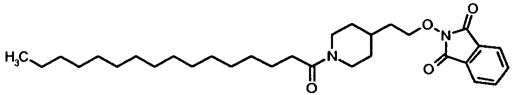

At 0°C, 1-(3-dimethylaminopropyl)-3-ethylcarbodiimide hydrochloride (1.04 g, 5.44 mmol) was added to a solution of palmic acid (1.40 g, 5.44 mmol) and 3,4-dihydro-3-hydroxy-4-oxo-1,2,3-benzotriazole (0.89 g, 5.44 mmol) in *N,N*-dimethylformamide (20 ml) and dichloromethane (20 ml). The reaction mixture was stirred at 0°C for 20 min. A solution of the trifluoroacetate salt of 2-(2-(piperidin-4-yl)ethoxy)isoindole-1,3-dione (2.11 g, 5.44 mmol) in *N,N*-dimethylformamide (5 ml) and ethyldiisopropylamine (6.19 ml, 38.1 mmol) were added successively. The reaction mixture was stirred for 16 h, while it was warming up to room temperature. It was diluted with ethyl acetate (150 ml) and was washed with a 10% aqueous solution of sodium hydrogensulphate (150 ml). The aqueous phase was extracted with ethyl acetate. The combined organic layers were washed with a mixture of water (50 ml) and a saturated aqueous solution of sodium hydrogencarbonate (50 ml) and dried over magnesium sulphate. The crude productwas purified by flash chromatography on silica (40 g), using ethyl acetate/heptane 1:1 as eluent to give 1.52 g of 2-[2-(1-(hexadecanoyl)piperidin-4-yl)ethoxy]isoindole-1,3-dione.
MS: m/z = 513 [M+1]⁺
¹H-NMR (DMSO-d₆): δ 0.90 (t, 3 H); 1.10 (m, 2 H); 1.25 (m, 26 H); 1.45 (m, 2 H); 1.65 (m, 1 H); 1.80 (m, 2 H); 2.30 (t, 2 H); 2.95 (t, 1 H); 3.85 (m, 3 H); 4.20 (t, 2 H); 4.40 (d, 1 H); 7.90 (s, 4 H).

### Step 5:

Hydrazine hydrate (0.14 ml, 2.96 mmol) was added to a solution of 2-[2-(1-(hexadecanoyl)piperidin-4-yl)ethoxy]isoindole-1,3-dione (1.52 g, 2.96 mmol) in ethanol (30 ml). The reaction mixture was heated to reflux for 75 min and cooled to room temperature. The formed precipitation was removed by filtration. The solvent of the filtrate was removed in vacuo. The crude product was purified by flash chromatography on silica (30 g), using a mixture of dichloromethane/methanol/25% aqueous ammonia (100:10:1) as eluent, to give 800 mg of 1-[4-(2-(aminooxy)ethyl)piperidin-1-yl]hexadecan-1-one.
MS: m/z = 383 [M+1]⁺
¹H-NMR (CDCl₃): δ 0.80 (t, 3 H); 1.25 (m, 2 H); 1.60 (m, 26 H); 1.70 (m, 4 H); 1.65 (m, 3 H); 2.70 8t, 2 H); 2.60 (t, 1 H); 3.05 (t, 1 H); 3.80 (m, 3 H); 4.60 (d, 1 H).

### Example 9. Transamination of Z-Gln-Gly to give [4-(3-Amino-2-hydroxy-propylcarbamoyl)-2-benzyloxycarbonylamino-butyrylamino]-acetic acid

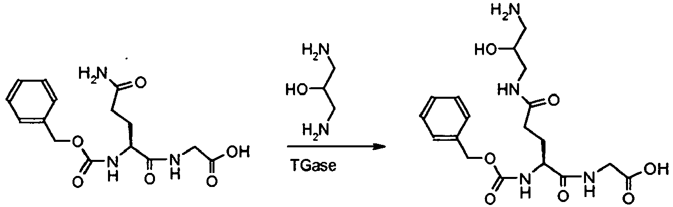

### 30 mg Z-Gln-Gly (Bachem C1635) was dissolved in phosphate buffer (50 mM, pH 8.0, 2 ml).

To this was added a solution of 1,3-Diamino-propan-2-ol (9 mg) in phosphate buffer (50 mM, pH 8.0, pH adjusted to 8.0 after dissolution of 1,3-Diamino-propan-2-ol, 0.9 ml). Finally a solution of TGase (0.9 mg ~ 2 U) dissolved in phosphate buffer (50 mM, pH 8.0, 0.1 ml) was added and the combined mixture was incubated for 4 hours at 37 °O. The temperature was lowered to room temperature and N-ethyl-maleimide was added to a final concentration of 1 mM. After further 1 hour the mixture was diluted with 10 volumes of water. The product was isolated from this solution by semipreparative HPLC in one run on a 25 mm x 250 mm column packed with 7µm C-18 silica. The column was eluted with a gradient of 10 to 30% acetonitrile in 0.1% trifluoracetic acid / H₂O at 10 ml/min at a temperature of 40°C for 50min. The peptide containing fractions corresponding to the major peak were collected, diluted to 30 ml with approximately 3 volumes of H₂O and lyophilized. The final product obtained was characterized by RP-HPLC where it had a retention time of 12.75 min and by LC-MS where a retention time of 1.9 min had a mass peak corresponding to M + H⁺ of 411.5 amu which was in agreement with the expected structure

### Example 10. Oxidation of [4-(3-Amino-2-hydroxy-propylcarbamoyl)-2-benzyloxycarbonylamino-butyrylamino]-acetic acid to give [2-Benzyloxycarbonylamino-4-(2-oxo-ethylcarbamoyl)-butyrylamino]-acetic acid

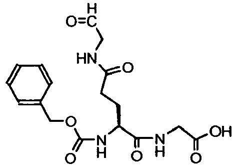

0.8 mg [4-(3-Amino-2-hydroxy-propylcarbamoyl)-2-benzyloxycarbonylamino-butyrylamino]-acetic acid was dissolved in 4 ml 15 mM triethanolamine buffer pH 8.5 (adjusted with 1 N hydrochloric acid). To this was added 1 ml of a 173 mM methionine solution in water. Finally 0.5 ml of a 24 mM sodiumperiodate in water was added, and the mixture was incubated for 10 min at 0 °C

### Example 11. Oximation of [2-Benzyloxycarbonylamino-4-(2-oxo-ethylcarbamoyl)-butyrylamino]-acetic acid to give (2-Benzyloxycarbonylamino-4-{2-[2-(1-hexadecanoyl-piperidin-4-yl)-ethoxyimino]-ethylcarbamoyl}-butyrylamino)-acetic acid.

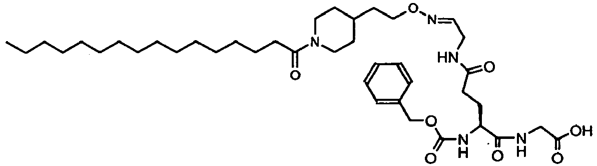

2 mg 1-[4-(2-(Aminooxy)ethyl)piperidin-1-yl]hexadecan-1-one was dissolved in 3 ml N,N-dimethylformamide and the solution was cooled on ice. To 0.53 ml of this solution was added 1.38 ml of the reaction mixture from example 10 and the mixture was allowed to react at 0 °C overnight. RP-HPLC confirmed the formation of a new product. It was isolated by RP-HPLC in analytical scale and submitted to Maldi-TOF mass spectrometry which gave a peak corresponding to M + H⁺ : 744.7 amu in agreement with the expected structure.

### Example 12. Oxidation of N^{ε141}-(2-hydroxy-3-amino-propyl) hGH (II.) to give N^{ε141}-(2-oxo-etyl) hGH (III.)

5 mg N^{ε141}-(2-hydroxy-3-amino-propyl) hGH (II.) was dissolved in 0.5 ml 15 mM triethanolamine buffer pH 8.5 (adjusted with 1 N hydrochloric acid). To this was added 0.13 ml of a 173 mM methionine solution in water. Finally 0.06 ml of a 24 mM sodiumperiodate in water was added, and the mixture was incubated for 10 min at 0 °C

### Example 13. Oximation of N^{ε141}-(2-oxo-etyl) hGH (III.) with 1-[4-(2-(Aminooxy)ethyl)piperidin-1-yl]hexadecan-1-one to give N^{ε141}-[2-(1-(hexadecanoyl)piperidin-4-yl)ethyloxyimino)-ethyl] hGH (V.).

2 mg 1-[4-(2-(Aminooxy)ethyl)piperidin-1-yl]hexadecan-1-one was dissolved in 3 ml N,N-dimethylformamide and the solution was cooled on ice. 0.14 ml of the reaction mixture from example 12 was added and the mixture was allowed to react at 0 °C overnight.

### Example 14 Characterization of N^{ε141}-[2-(1-(hexadecanoyl)piperidin-4-yl)ethyloxyimino)-ethyl] hGH (V.)

An aliquot of the crude reaction mixture from example 13 fractionated by RP-HPLC in analytical scale and Maldi-TOF mass spectrometry was performed on the fractions. The fraction giving the expected molecular weight molecular weight expected from the product structure was subjected to peptide mapping. The map showed that the Asp-N fragment AA 130-146 displayed a mass increase of 407 amu corresponding to the addition of the 2-(1-(hexadecanoyl)piperidin-4-yl)ethyloxyimino)-ethyl in the side chain of a Glutamine residue. This was the only peptide, that had changed retention time in the HPLC map when compared to that of native hGH. This fragment contains two Glutamine residues. The peptide was subjected to Edman sequencing and Gln-137 was found at the expected yield, whereas Gln-141 displayed a blank Edman cycle. It was concluded, that derivatization had taken place selectively at Gln-141.

## Claims

1. A method for conjugating peptides, said method comprising the steps of
i) reacting in one or more steps a Gln-residue containing peptide represented by the formula 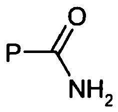 with a nitrogen containing nucleophile (first compound) represented by the formula
H₂N-D-R-X
comprising one or more functional groups or latent functional groups, which are not accessible in any of the amino acids residues constituting said peptide, in the presence of transglutaminase capable of catalysing the incorporation of said first compound into said peptide to form a transaminated peptide of the formula 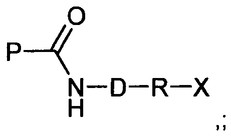 and
ii) optionally activate the latent functional group X; and
iii) reacting in one or more steps said transaminated peptide with a second compound of the formula
Y-E-Z
comprising one or more functional groups, wherein said functional group(s) do not react with functional groups accessible in the amino acid residues constituting said peptide, and wherein said functional group(s) in said second compound is capable of reacting with said functional group(s) in said first compound so that a covalent bond between said transaminated peptide and said second compound is formed resulting in a conjugated peptide of the formula 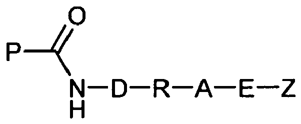 wherein D represents a bond or oxygen;
R represents a linker or a bond;
X represents a radical comprising a functional group or a latent functional group not accessible in the amino acid residues constituting the peptide P-C(O)-NH₂;
Y represents a radical comprising one or more functional groups which groups react with functional groups present in X, and which functional groups do not react with functional groups accessible in the peptide P-C(O)-NH₂;
E represents a linker or a bond;
A represents an oxime, hydrazone, phenylhydrazone, semicarbazone, triazole or isooxazolidine moiety, the moiety formed by the reaction between the functional groups comprised in X and Y; and
Z is the moiety to be conjugated to the peptide.

2. A method according to claim 1, wherein the functional group or latent functional group comprised in X is selected from or can be activated to keto-, aldehyde-, -NH-NH₂, -O-C(O)-NH-NH₂, -NH-C(O)-NH-NH₂, -NH-C(S)-NH-NH₂, -NHC(O)-NH-NH-C(O)-NH-NH₂, -NH-NH-C(O)-NH-NH₂, **-NH-NH-C(S)-NH-NH₂,** -NH-C(O)-C₆H₄-NH-NH₂, -C(O)-NH-NH₂, -O-NH₂, - C(O)-O-NH₂, -NH-C(O)-O-NH₂, -NH-C(S)-O-NH₂, alkyne, azide or nitril-oxide.

3. A method according to any of claims 1 and 2, wherein the functional group present in Y is selected from amongst keto-, aldehyde-, -NH-NH₂, -O-C(O)-NH-NH₂, -NH-C(O)-NH-NH₂, -NH-C(S)-NH-NH₂, -NHC(O)-NH-NH-C(O)-NH-NH₂, -NH-NH-C(O)-NH-NH₂, -NH-NH-C(S)-NH-NH₂, -NH-C(O)-C₆H₄-NH-NH₂, -C(O)-NH-NH₂, -O-NH₂, -C(O)-O-NH₂, -NH-C(O)-O-NH₂, - NH-C(S)-O-NH₂, alkyne, azide and nitril-oxide.

4. A method according to any of claims 1 to 3, wherein X is selected from or can be activated to keto- or aldehyde-derivatives, and Y is selected from -NH-NH₂, -O-C(O)-NH-NH₂, -NH-C(O)-NH-NH₂, -NH-C(S)-NH-NH₂, -NHC(O)-NH-NH-C(O)-NH-NH₂, -NH-NH-C(O)-NH-NH₂, -NH-NH-C(S)-NH-NH₂, -NH-C(O)-C₆H₄-NH-NH₂, -C(O)-NH-NH₂, -O-NH₂, -C(O)-O-NH₂, -NH-C(O)-O-NH₂, and -NH-C(S)-O-NH₂.

5. A method according to claim 4, wherein the latent group comprised in X is selected amongst 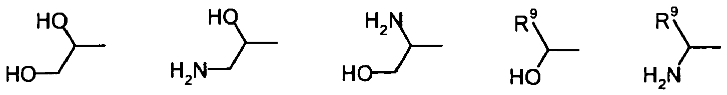 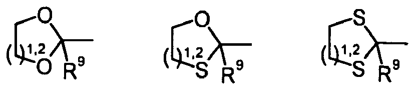 wherein R⁹ is selected amongst H, C₁₋₆alkyl, aryl and heteroaryl.

6. A method according to any of claims 1 to 3, wherein X and Y each represent a different member of the group consisting of alkyne and triazole, or of the group consisting of alkyne and nitril-oxide.

7. A method according to any of claims 1 to 4, wherein said nitrogen containing nucleophile is selected from 4-(aminomethyl)phenyl ethanone, 4-(2-aminoethyl)phenyl ethanone, N-(4-acetylphenyl) 2-aminoacetamide, 1-[4-(2-aminoethoxy)phenyl]ethanone, 1-[3-(2-aminoethoxy)phenyl]ethanone, 1,4-bis(aminoxy)butane, 3-oxapentane-1,5-dioxyamine, 1,8-diaminoxy-3,6-dioxaoctane, 1,3-bis(aminoxy)propan-2-ol, 1,11-bis(aminoxy)-3,6,9-trioxaundecane, 1,3-diamino-2-propanol, 1,2-bis(aminoxy)ethane, and 1,3-bis(aminoxy)propane.

8. A method according to any of claims 1 to 7, wherein Z comprises one or more PEG or mPEG radicals and amino derivatives thereof (including straight and branched PEG and mPEG radicals); straight, branched and/or cyclic C₁₋₂₂alkyl, C₂₋₂₂alkenyl, C₂₋₂₂alkynyl, C₁₋₂₂heteroalkyl, C₂₋₂₂heteroalkenyl, C₂₋₂₂heteroalkynyl, wherein one or more homocyclic aromatic compound biradical or heterocyclic compound biradical may be inserted, and wherein said C₁-C₂₂ or C₂-C₂₂ radicals may optionally be substituted with one or more substituents selected from hydroxyl, halogen, carboxyl, heteroaryl and aryl, wherein said aryl or heteroaryl may optionally be further substituted by one or more substituents selected from hydroxyl, halogen, and carboxyl; steroid radicals; lipid radicals; polysaccharide radicals; dextrans; polyamide radicals; polyamino acid radicals; PVP radicals; PVA radicals; poly(1-3-dioxalane); poly(1,3,6-trioxane); ethylene/maleic anhydride polymer; Cibacron dye stuffs; or Cibacron Blue 3GA.

9. A method according to claim 8, wherein Z comprises one or more PEG or mPEG radicals with a molecular weight between around 10 kDa and 40 kDa.

10. A method according to claim 9, wherein Z represents one or more PEG or mPEG radicals with a molecular weight around 10 kDa, 20kDa, 30 kDa or 40 kDa.

11. A method according to claim 8, wherein Z comprises one or more C₁₀₋₂₀alkyl.

12. A method according to claim 6, wherein Z comprises one or more C₁₅alkyl, C₁₇alkyl, Cibacron Blue 3GA or radical of the formula

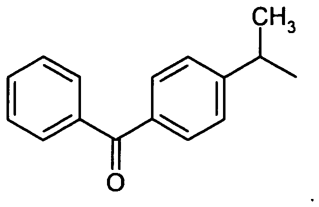

13. A method according to any of claims 1 to 12, wherein the enzyme is transglutaminase isolated from *Streptomyces mobaraenese, Streptomyces lydicus* or guinea-pig liver.

14. A method according to any of claims 1 to 13, wherein P represents a peptide selected from insulin, glucagon like-peptide 1 (GLP-1), glucagon like-peptide 2 (GLP-2), growth hormone, cytokines, TFF, melanocortin receptor modifiers and factor VII compounds.

15. A method according to claim 14, wherein P represents growth hormone.

## Patentansprüche

1. Verfahren zum Konjugieren von Peptiden, wobei das Verfahren die folgenden Schritte umfasst:
i) Umsetzen in einem oder mehreren Schritten eines Gln-Rests, der ein Peptid der Formel 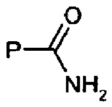 enthält, mit einem stickstoffhaltigen Nukleophil (erste Verbindung) der Formel
**H**₂**N-D-R-X**
die eine oder mehrere funktionelle Gruppen oder latente funktionelle Gruppen umfasst, die in einer beliebigen der das Peptid bildenden Aminosäurereste nicht zugänglich sind, in der Gegenwart von Transglutaminase, die in der Lage ist, das Einbringen der ersten Verbindung in das Peptid unter Bildung eines transaminierten Peptids der Formel 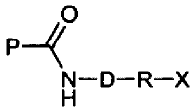 zu katalysieren, und
ii) wahlweise Aktivieren der latenten funktionellen Gruppe X; und
iii) Umsetzen in einem oder mehreren Schritten des transaminierten Peptids mit einer zweiten Verbindung der Formel
Y-E-Z
die eine oder mehrere funktionelle Gruppen umfasst, wobei die funktionelle(n) Gruppe(n) mit den funktionellen Gruppen, die in den das Peptidbildenden Aminosäureresten nicht zugänglich sind, reagiert (reagieren), und wobei die funktionelle(n) Gruppe(n) in der zweiten Verbindung in der Lage sind, mit den (der) funktionellen Gruppe(n) in der ersten Verbindung derart zu reagieren, dass eine kovalente Bindung zwischen dem transaminierten Peptid und der zweiten Verbindung gebildet wird, wodurch ein konjugiertes Peptid der Formel 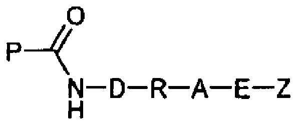 erhalten wird, wobei
D eine Bindung oder Sauerstoff darstellt;
R eine Verbindungsgruppe oder eine Bindung darstellt;
X einen Rest darstellt, der eine funktionelle Gruppe oder eine latente funktionelle Gruppe umfasst, die in den das Peptid P-C(O)-NH₂ bildenden Aminosäureresten nicht zugänglich ist;
Y einen Rest darstellt, der eine oder mehrere funktionelle Gruppen umfasst, wobei die Gruppen mit in X vorliegenden funktionellen Gruppen reagieren, und wobei die funktionellen Gruppen mit im Peptid P-C(O)-NH₂ vorliegenden funktionellen Gruppen nicht reagieren;
E eine Verbindungsgruppe oder eine Bindung darstellt;
A eine Oxim-, Hydrazon-, Phenylhydrazon-, Semicarbazon-, Triazol- oder Isooxazolidineinheit darstellt, wobei die Einheit durch die Reaktion zwischen den in X und Y enthaltenen funktionellen Gruppen gebildet wird; und
Z die zum Peptid zu konjugierende Einheit ist.

2. Verfahren nach Anspruch 1, wobei die in X enthaltene funktionelle Gruppe oder latente funktionelle Gruppe ausgewählt ist aus oder aktiviert werden kann zu Keto-, Aldehyd-, -NH-NH₂, -O-C(O)-NH-NH₂, -NH-C(O)-NH-NH₂, -NH-C(S)-NH-NH₂, -NHC(O)-NH-NH-C(O)-NH-NH₂, -NH-NH-C(O)-NH-NH₂, -NH-NH-C(S)-NH-NH₂, -NH-C(O)-C₆H₄-NH-NH₂, -C(O)-NH-NH₂, -O-NH₂, -C(O)-O-NH₂, -NH-C(O)-O-NH₂, -NH-C(S)-O-NH₂, Alkin, Azid oder Nitriloxid.

3. Verfahren nach einem der Ansprüche 1 und 2, wobei die in Y vorliegende funktionelle Gruppe ausgewählt ist aus Keto-, Aldehyd-, -NH-NH₂, -O-C(O)-NH-NH₂, -NH-C(O)-NH-NH₂, -NH-C(S)-NH-NH₂, -NHC(O)-NH-NH-C(O)-NH-NH₂, -NH-NH-C(O)-NH-NH₂, -NH-NH-C(S)-NH-NH₂, -NH-C(O)-C₆H₄-NH-NH₂, -C(O)-NH-NH₂, -O-NH₂, -C(O)-O-NH₂, -NH-C(O)-O-NH₂, -NH-C(S)-O-NH₂, Alkin, Azid oder Nitriloxid.

4. Verfahren nach einem der Ansprüche 1 bis 3, wobei X ausgewählt ist aus oder aktiviert werden kann zu Keto- oder Aldehydderivaten und Y ausgewählt ist aus -NH-NH₂, -O-C(O)-NH-NH₂, -NH-C(O)-NH-NH₂, -NH-C(S)-NH-NH₂, -NHC(O)-NH-NH-C(O)-NH-NH₂, -NH-NH-C(O)-NH-NH₂, -NH-NH-C(S)-NH-NH₂, -NH-C(O)-C₆H₄-NH-NH₂, -C(O)-NH-NH₂, -O-NH₂, -C(O)-O-NH₂, -NH-C(O)-O-NH₂ und -NH-C(S)-O-NH₂.

5. Verfahren nach Anspruch 4, wobei die in X enthaltene latente Gruppe
ausgewählt ist aus 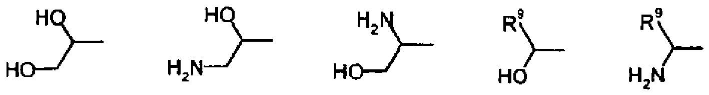 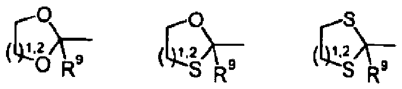 wobei R⁹ ausgewählt ist aus H, C₁₋₆-Alkyl, Aryl und Heteroaryl.

6. Verfahren nach einem der Ansprüche 1 bis 3, wobei X und Y jeweils einen anderen Vertreter der aus Alkin und Triazol bestehenden Gruppe oder der aus Alkin und Nitriloxid bestehenden Gruppe darstellen.

7. Verfahren nach einem der Ansprüche 1 bis 4, wobei das stickstoffhaltige Nukleophil ausgewählt ist aus 4-(Aminomethyl)phenylethanon, 4-(2-Aminoethyl)phenylethanon, N-(4-Acetylphenyl)-2-aminoacetamid, 1-[4-(2-Aminoethoxy)phenyl]ethanon, 1-[3-(2-Aminoethoxy)phenyl]ethanon, 1,4-Bis(aminoxy)butan, 3-Oxapentan-1,5-dioxyamin, 1,8-Diaminoxy-3,6-dioxaoctan, 1,3-Bis(aminoxy)propan-2-ol, 1,11-Bis(aminoxy)-3,6,9-trioxaundecan, 1,3-Diamino-2-propanol, 1,2-Bis(aminoxy)ethan und 1,3-Bis(aminoxy)propan.

8. Verfahren nach einem der Ansprüche 1 bis 7, wobei Z einen oder mehrere PEG- oder mPEG-Reste und Aminoderivate davon (einschließlich geradkettige und verzweigte PEG- und mPEG-Reste), geradkettiges, verzweigtes und/oder cyclisches C₁₋₂₂-Alkyl, C₂₋₂₂-Alkenyl, C₂₋₂₂Alkinyl, C₁₋₂₂-Heteroalkyl, C₂₋₂₂-Heteroalkenyl, C₂₋₂₂-Heteroalkinyl, wobei ein oder mehrere Bireste einer homocyclischen aromatischen Verbindung oder Bireste einer heterocyclischen Verbindung eingefügt werden können, und wobei die C₁₋₂₂- oder C₂₋₂₂-Reste wahlweise mit einem oder mehreren Substituenten substituiert sein können, die ausgewählt sind aus Hydroxy, Halogen, Carboxy, Heteroaryl und Aryl, wobei das Aryl oder Heteroaryl wahlweise weiter durch einen oder mehrere Substituenten substituiert sein kann, die ausgewählt sind aus Hydroxy, Halogen und Carboxy; Steroidreste;
Lipidreste; Polysaccharidreste; Dextrane; Polyamidreste; Polyaminosäurereste; PVP-Reste; PVA-Reste; Poly(1,3-dioxalan); Poly(1,3,6-trioxan); Ethylen/Maleinsäureanhydridpolymer; Cibacronfarbstoffe; oder Cibacron Blau 3GA umfasst.

9. Verfahren nach Anspruch 8, wobei Z einen oder mehrere PEG- oder mPEG-Reste mit einem Molekulargewicht zwischen etwa 10 kDa und 40 kDa umfasst.

10. Verfahren nach Anspruch 9, wobei Z einen oder mehrere PEG- oder mPEG-Reste mit einem Molekulargewicht von etwa 10 kDa, 20 kDa, 30 kDa oder 40 kDa umfasst.

11. Verfahren nach Anspruch 8, wobei Z ein oder mehrere C₁₀₋₂₀-Alkyle umfasst.

12. Verfahren nach Anspruch 6, wobei Z ein oder mehrere C₁₅-Alkyle, C₁₇-Alkyle, Cibacron Blau 3GA oder Reste der Formel 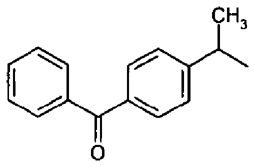 umfasst.

13. Verfahren nach einem der Ansprüche 1 bis 12, wobei das Enzym Transglutaminase ist, das von *Streptomyces mobaraense, Streptomyces lydicus* oder Meerschweinchenleber isoliert ist.

14. Verfahren nach einem der Ansprüche 1 bis 13, wobei P ein Peptid darstellt, das ausgewählt ist aus Insulin, glucagonartigem Peptid 1 (GLP-1), glucagonartigem Peptid-2 (GLP-2), Wachstumshormon, Cytokinen, TFF, Melanocortinrezeptormodifikatoren und Faktor-VII-Verbindungen.

15. Verfahren nach Anspruch 14, wobei P Wachstumshormon darstellt.

## Revendications

1. Procédé de conjugaison de peptides, ledit procédé comprenant les étapes de :
i) réaction, en une ou plusieurs étapes, d'un peptide contenant un résidu de Gln représenté par la formule 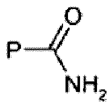 avec un nucléophile contenant de l'azote (premier composé) représenté par la formule
H₂N - D - R - X
comprenant un ou plusieurs groupes fonctionnels ou groupes fonctionnels latents, qui ne sont accessibles dans aucun des résidus d'acides aminés constituant ledit peptide, en présence d'une transglutaminase capable de catalyser l'incorporation dudit premier composé dans ledit peptide, pour former un peptide trans-aminé de formule 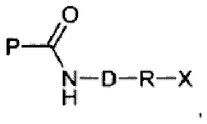 et
ii) en option, activation du groupe fonctionnel latent X ; et
iii) réaction, en une ou plusieurs étapes, dudit peptide trans-aminé avec un deuxième composé de formule
Y - E - Z
comprenant un ou plusieurs groupes fonctionnels, ledit ou lesdits groupes fonctionnels ne réagissant pas avec les groupes fonctionnels accessibles dans les résidus d'acides aminés constituant ledit peptide, et ledit ou lesdits groupes fonctionnels se trouvant dans ledit deuxième composé étant capables de réagir avec ledit ou lesdits groupes fonctionnels se trouvant dans ledit premier composé, de façon qu'une liaison covalente entre ledit peptide trans-aminé et ledit deuxième composé soit formée, conduisant à un peptide conjugué de formule 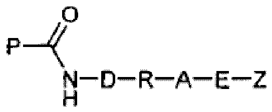 dans laquelle D représente une liaison ou un oxygène ;
R représente un lieur ou une liaison ;
X représente un radical comprenant un groupe fonctionnel ou un groupe fonctionnel latent non accessible dans les résidus d'acides aminés constituant le peptide P-C(O)-NH₂ ;
Y représente un radical comprenant un ou plusieurs groupes fonctionnels, ces groupes réagissant avec les groupes fonctionnels présents dans X, et ces groupes fonctionnels ne réagissant pas avec les groupes fonctionnels accessibles dans le peptide P-C(O)-NH₂ ;
E représente un lieur ou une liaison ;
A représente un fragment oxime, hydrazone, phénylhydrazone, semicarbazone, triazole ou isooxazolidine, le fragment formé par la réaction entre les groupes fonctionnels étant compris dans X et Y ; et
Z est le fragment devant être conjugué au peptide.

2. Procédé selon la revendication 1, dans lequel le groupe fonctionnel ou le groupe fonctionnel latent compris dans X est choisi parmi les groupes suivants, ou peut être activé en ceux-ci : céto, aldéhyde, -NH-NH₂, -OC(O)-NH-NH₂, -NH-C(O)-NH-NH₂, -NH-C (S)-NH-NH₂, -NHC(O)-NH-NH-C(O)-NH-NH₂, -NH-NH-C(O)-NH-NH₂, -NH-NH-C(S)-NH-NH₂, -NH-C(O)-C₆H₄-NH-NH₂, -C(O)-NH-NH₂, -O-NH₂, C(O)-O-NH₂, -NH-C(O)-O-NH₂, -NH-C(S)-O-NH₂, alcyne, azide ou nitrile-oxyde.

3. Procédé selon l'une quelconque des revendications 1 et 2, dans lequel le groupe fonctionnel présent dans Y est choisi parmi les groupes céto, aldéhyde, -NH-NH₂, -OC(O)-NH-NH₂, -NH-C(O)-NH-NH₂, -NH-C(S)-NH-NH₂, -NHC(O)-NH-NH-C(O)-NH-NH₂, -NH-NH-C(O)-NH-NH₂, -NH-NH-C(S)-NH-NH₂, -NH-C(O)-C₆H₄-NH-NH₂, -C(O)-NH-NH₂, -O-NH₂, C(O)-O-NH₂, -NH-C(O)-O-NH₂, -NH-C(S)-O-NH₂, alcyne, azide et nitrile-oxyde.

4. Procédé selon l'une quelconque des revendications 1 à 3, dans lequel X est choisi parmi les dérivés céto ou aldéhyde, ou peut être activé en ces derniers, et Y est choisi parmi -NH-NH₂, -O-C(O)-NH-NH₂, -NH-C(O)-NH-NH₂, -NH-C(S)-NH-NH₂, -NHC(O)-NH-NH-C(O)-NH-NH₂, -NH-NH-C(O)-NH-NH₂, -NH-NH-C (S)-NH-NH₂, -NH-C(O)-C₆H₄-NH-NH₂, -C(O)-NH-NH₂, -O-NH₂, C(O)-O-NH₂, -NH-C(O)-O-NH₂ et -NH-C(S)-O-NH₂.

5. Procédé selon la revendication 4, dans lequel le
groupe latent compris dans X est choisi parmi 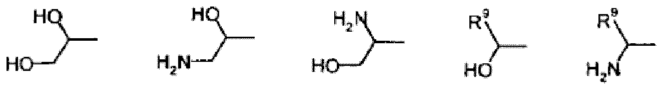 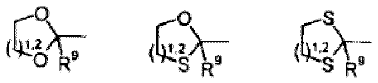 où R⁹ est choisi parmi H, les groupes alkyle en C₁₋₆, aryle et hétéroaryle.

6. Procédé selon l'une quelconque des revendications 1 à 3, dans lequel X et Y représentent chacun un membre différent du groupe consistant en un alcyne et un triazole, ou du groupe consistant en un alcyne et un nitrile-oxyde.

7. Procédé selon l'une quelconque des revendications 1 à 4, dans lequel ledit nucléophile contenant de l'azote est choisi parmi la 4-(aminométhyl)phényléthanone, la 4-(2-aminoéthyl)phényléthanone, le N-(4-acétylphényl)2-aminoacétamide, la 1-[4-(2-aminoéthoxy)phényl]éthanone, la 1-[3-(2-aminoéthoxy)phényl]éthanone, le 1,4-bis(aminoxy)butane, la 3-oxapentane-1,5-dioxyamine, le 1,8-diaminoxy-3,6-dioxaoctane, le 1,3-bis(aminoxy)propane-2-ol, le 1,11-bis(aminoxy)-3,6,9-trioxaundécane, le 1,3-diamino-2-propanol, le 1,2-bis(aminoxy)éthane et le 1,3-bis(aminoxy)propane.

8. Procédé selon l'une quelconque des revendications 1 à 7, dans lequel Z comprend un ou plusieurs radicaux PEG ou mPEG et dérivés aminés de ces derniers (y compris les radicaux PEG et mPEG à chaîne droite ou ramifiée), des groupes alkyle en C₁₋₂₂, alcényle en C₂₋₂₂, alcynyle en C₂₋₂₂, hétéroalkyle en C₁₋₂₂, hétéroalcényle en C₂₋₂₂, hétéroalcynyle en C₂₋₂₂ à chaîne droite ou ramifiée, et/ou cyclique, dans lesquels un ou plusieurs biradicaux de composés aromatiques homocycliques ou biradicaux de composés hétérocycliques peuvent être insérés, et dans lequel lesdits radicaux en C₁-C₂₂ ou en C₂-C₂₂ peuvent en option être substitués par un ou plusieurs substituants choisis parmi les groupes hydroxyle, halogéno, carboxyle, hétéroaryle et aryle, lesdits groupes aryle ou hétéroaryle pouvant en option être en outre substitués par un ou plusieurs substituants choisis parmi les groupes hydroxyle, halogéno et carboxyle ; des radicaux stéroïdes ; des radicaux lipidiques ; des radicaux polysaccharidiques ; des dextrans ; des radicaux polyamides ; des radicaux poly(acides aminés) ; des radicaux PVP ; des radicaux PVA ; du poly(1,3-dioxalanne) ; du poly(1,3,6-trioxanne) ; un polymère éthylène/anhydride maléique ; des colorants Cibacron ; ou du Cibacron Blue 3GA.

9. Procédé selon la revendication 8, dans lequel Z comprend un ou plusieurs radicaux PEG ou mPEG ayant une masse moléculaire comprise entre environ 10 et 40 kDa.

10. Procédé selon la revendication 9, dans lequel Z représente un ou plusieurs radicaux PEG ou mPEG ayant une masse moléculaire d'environ 10 kDa, 20 kDa, 30 kDa ou 40 kDa.

11. Procédé selon la revendication 8, dans lequel Z comprend un ou plusieurs groupes alkyle en C₁₀₋₂₀.

12. Procédé selon la revendication 6, dans lequel Z comprend un ou plusieurs groupes alkyle en C₁₅, alkyle en C₁₇, colorants Cibacron Blue 3GA ou radicaux de formule

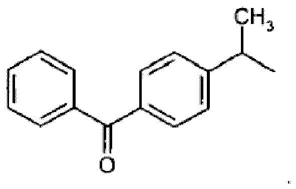

13. Procédé selon l'une quelconque des revendications 1 à 12, dans lequel l'enzyme est une transglutaminase isolée de *Streptomyces mobaraenese, Streptomyces lydicus,* ou du foie du cobaye.

14. Procédé selon l'une quelconque des revendications 1 à 3, dans lequel P représente un peptide choisi parmi l'insuline, le glucagon like-peptide 1 (GLP-1), le glucagon like-peptide 2 (GLP-2), l'hormone de croissance, les cytokines, le TFF, les agents modifiant les récepteurs de la mélanocortine et les composés du facteur VII.

15. Procédé selon la revendication 14, dans lequel P représente une hormone de croissance.
